(19) 

**Europäisches Patentamt**
**European Patent Office**
**Office européen des brevets**

(11) **EP 4 509 529 A1**

(12) **EUROPEAN PATENT APPLICATION**
published in accordance with Art. 153(4) EPC

(43) Date of publication:
**19.02.2025  Bulletin 2025/08**

(21) Application number: **23788361.6**

(22) Date of filing: **12.04.2023**

(51) International Patent Classification (IPC):
*C07K 16/40* (2006.01)     *A61K 39/395* (2006.01)
*A61P 31/14* (2006.01)     *C12N 15/13* (2006.01)
*C12N 15/57* (2006.01)     *C12P 21/08* (2006.01)

(52) Cooperative Patent Classification (CPC):
**A61K 39/395; A61P 31/14; C07K 16/00;**
**C07K 16/40; C12N 9/48**

(86) International application number:
**PCT/JP2023/014855**

(87) International publication number:
**WO 2023/199943 (19.10.2023 Gazette 2023/42)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority: **12.04.2022  JP 2022065547**

(71) Applicants:
• **RIKEN**
**Wako-shi, Saitama 351-0198 (JP)**
• **The University of Tokyo**
**Bunkyo-ku, Tokyo 113-8654 (JP)**
• **NATIONAL UNIVERSITY CORPORATION SHIGA**
**UNIVERSITY OF MEDICAL SCIENCE**
**Otsu-shi, Shiga 520-2192 (JP)**

(72) Inventors:
• **SAITO Takashi**
**Wako-shi, Saitama 351-0198 (JP)**
• **HARADA Michishige**
**Wako-shi, Saitama 351-0198 (JP)**
• **SHIROUZU Mikako**
**Wako-shi, Saitama 351-0198 (JP)**
• **MATSUMOTO Takehisa**
**Wako-shi, Saitama 351-0198 (JP)**

• **IDEI Akiko**
**Wako-shi, Saitama 351-0198 (JP)**
• **INOUE Jun-ichiro**
**Tokyo 113-8654 (JP)**
• **YAMAMOTO Mizuki**
**Tokyo 113-8654 (JP)**
• **GOHDA Jin**
**Tokyo 113-8654 (JP)**
• **KAWAGUCHI Yasushi**
**Tokyo 113-8654 (JP)**
• **ITO Yasushi**
**Otsu-shi, Shiga 520-2192 (JP)**
• **ISHIGAKI Hirohito**
**Otsu-shi, Shiga 520-2192 (JP)**
• **NAKAYAMA Misako**
**Otsu-shi, Shiga 520-2192 (JP)**
• **KITAGAWA Yoshinori**
**Otsu-shi, Shiga 520-2192 (JP)**

(74) Representative: **Klöckner, Christoph**
**df-mp Dörries Frank-Molnia & Pohlman**
**Patentanwälte Rechtsanwälte PartG mbB**
**Theatinerstraße 16**
**80333 München (DE)**

Remarks:
The complete document including Reference
Table(s) and the Sequence Listing(s) can be
downloaded from the EPO website

(54) **ANTIBODY USED TO TREAT CORONAVIRUS INFECTION**

(57)    The present invention provides an antibody that binds to an extracellular domain of TMPRSS2, capable of inhibiting beta coronavirus infection of cells, or an antigen-binding fragment thereof, and a composition including the antibody or an antigen-binding fragment thereof.

EP 4 509 529 A1

**(Cont. next page)**

# FIG. 1

**Description**

Technical Field

The present invention relates to an antibody for use in treating coronavirus infection.

Background Art

Nowadays, the coronavirus pandemic has been greatly affecting the global economy. Because of mRNA vaccine against coronavirus being developed and quickly spread out across the world, the threats of the coronavirus infection seem to have come to an end for a while. However, variants of the coronavirus have emerged and rapidly replaced the original strain and variants of the past. For example, the α variant was confirmed in the UK in September 2020. The β variant was confirmed in South America in May 2020. The γ variant was confirmed in Brazil in November 2020. The δ variant was confirmed in India in October 2020. Also, the omicron variant was confirmed in multiple countries in November 2021. Moreover, the λ variant was confirmed in Peru in December 2020, and the μ variant was confirmed in Colombia in January 2021. Besides that, the World Health Organization (WHO) has designated from time to time a large number of variants as variants of concern (VOC) and variants of interest (VOI), and the threats of coronavirus variants seem to continue to happen. As such, a large number of variants emerged within the past short period of 1 to 2 years, which causes threats across the world every time a new variant emerges.

Prophylactic and therapeutic drugs for coronavirus infection have been developed worldwide. In particular, mRNA vaccines that encode the spike (S) proteins of coronaviruses have been administered by intramuscular injection all over the world, and their extremely high effectiveness has been confirmed. The mRNA vaccines contain, as an active ingredient, lipid nanoparticles (LNPs) that encapsulate mRNA containing pseudo-uridine (Patent Literatures 1 and 2).

However, the emergence of a large number of variants raises concerns about the effectiveness of the vaccines, and therefore, every time a new variant emerges, the effectiveness of the vaccines needs to be confirmed, and if not effective, vaccines must be redesigned and developed again.

Citation List

Patent Literature

    Patent Literature 1: WO2007/024708A
    Patent Literature 2: WO2009/127060A

Summary of Invention

The present invention provides an antibody against coronaviruses, the antibody being expected to be effective regardless of virus mutations.

Specifically, the present inventors have found that antibodies that target human protein TMPRSS2 required to activate the S protein used by coronaviruses for infection, can widely prevent coronavirus infection regardless of variant.

According to the present invention, the following inventions are provided.

[1] An antibody or an antigen-binding fragment thereof that binds to an extracellular domain of TMPRSS2, capable of inhibiting coronavirus infection of cells.

[2] The antibody or an antigen-binding fragment thereof according to [1] above, capable of binding to (i) one or more extracellular domains of TMPRSS2 selected from the group consisting of mice, green monkeys, cats, hamsters, and macaque monkeys, and (ii) an extracellular domain of human TMPRSS2.

[3] The antibody or an antigen-binding fragment thereof according to [1] or [2] above, wherein a binding affinity (KD) to human TMPRSS2 is $10^{-8}$ M or less.

[4] The antibody or an antigen-binding fragment thereof according to any of [1] to [3] above, wherein $IC_{90}$ referring to inhibition of the coronavirus infection of human cells is 10 μg/mL or less.

[5] The antibody or an antigen-binding fragment thereof according to any of [1] to [4] above, wherein the antibody binds to a peptide having the amino acid sequence consisting of the amino acid sequence selected from the group consisting of the amino acid sequence set forth in SEQ ID NO: 75 (WNENYGRAACRDMGYKNNFY), and the amino acid sequence set forth in SEQ ID NO: 89 (VTAAHCVEKPLNNPWHWT) .

[6] The antibody or an antigen-binding fragment thereof according to any of [1] to [4] above, wherein the antibody binds to human TMPRSS2 having the amino acid sequence set forth in SEQ ID NO: 5, and wherein:

($\alpha$) the antibody does not significantly bind to chimeric TMPRSS2 having the amino acid sequence wherein the amino acid sequence set forth in SEQ ID NO: 75 (WNENYGRAACRDMGYKNNFY) of the human TMPRSS2 is substituted for a corresponding sequence of a cat (i.e., the amino acid sequence set forth in SEQ ID NO: 94: WSEGYGRAACQDMGYRNSFY):

($\beta$) the antibody does not significantly bind to chimeric TMPRSS2 having the amino acid sequence wherein the amino acid sequence set forth in SEQ ID NO: 89 (VTAAHCVEKPLNNPWHWT) of the human TMPRSS2 is substituted for a corresponding sequence of a cat (i.e., the amino acid sequence set forth in SEQ ID NO: 95: VTAAHCVEEPLNNPRHWT); or

($\gamma$) the antibody does not significantly bind to chimeric TMPRSS2 having the amino acid sequence wherein the amino acid sequence set forth in SEQ ID NO: 96 (RQSFMF) of the human TMPRSS2 is substituted for a corresponding sequence of a hamster (i.e., the amino acid sequence set forth in SEQ ID NO: 97: SQSLMF).

[7] The antibody or an antigen-binding fragment thereof according to any of [1] to [4] above, wherein the antibody binds to human TMPRSS2 having the amino acid sequence set forth in SEQ ID NO: 5, and wherein:

(A) the antibody does not significantly bind to a human TMPRSS2 variant having a point mutation at N179A; or
(B) the antibody does not significantly bind to a human TMPRSS2 variant having a point mutation at W306A.

[8] The antibody or an antigen-binding fragment thereof according to any of [1] to [4] above, wherein the antibody is:(1) an antibody or an antigen-binding fragment thereof, including a heavy chain variable region that has HCDR1 having the amino acid sequence set forth in SEQ ID NO: 11, HCDR2 having the amino acid sequence set forth in SEQ ID NO: 12, and

HCDR3 having the amino acid sequence set forth in SEQ ID NO: 13,
a light chain variable region that has LCDR1 having the amino acid sequence set forth in SEQ ID NO: 14,
LCDR2 having the amino acid sequence set forth in SEQ ID NO: 15, and
LCDR3 having the amino acid sequence set forth in SEQ ID NO: 16.
(2) an antibody or an antigen-binding fragment thereof, including a heavy chain variable region that has HCDR1 having the amino acid sequence set forth in SEQ ID NO: 19,
HCDR2 having the amino acid sequence set forth in SEQ ID NO: 20, and
HCDR3 having the amino acid sequence set forth in SEQ ID NO: 21,
a light chain variable region that has LCDR1 having the amino acid sequence set forth in SEQ ID NO: 22,
LCDR2 having the amino acid sequence set forth in SEQ ID NO: 23, and
LCDR3 having the amino acid sequence set forth in SEQ ID NO: 24.
(3) an antibody or an antigen-binding fragment thereof, including a heavy chain variable region that has HCDR1 having the amino acid sequence set forth in SEQ ID NO: 27,
HCDR2 having the amino acid sequence set forth in SEQ ID NO: 28, and
HCDR3 having the amino acid sequence set forth in SEQ ID NO: 29,
a light chain variable region that has LCDR1 having the amino acid sequence set forth in SEQ ID NO: 30,
LCDR2 having the amino acid sequence set forth in SEQ ID NO: 31, and
LCDR3 having the amino acid sequence set forth in SEQ ID NO: 32.
(4) an antibody or an antigen-binding fragment thereof, including a heavy chain variable region that has HCDR1 having the amino acid sequence set forth in SEQ ID NO: 35,
HCDR2 having the amino acid sequence set forth in SEQ ID NO: 36, and
HCDR3 having the amino acid sequence set forth in SEQ ID NO: 37,
a light chain variable region that has LCDR1 having the amino acid sequence set forth in SEQ ID NO: 38,
LCDR2 having the amino acid sequence set forth in SEQ ID NO: 39, and
LCDR3 having the amino acid sequence set forth in SEQ ID NO: 40.
(5) an antibody or an antigen-binding fragment thereof, including a heavy chain variable region that has HCDR1 having the amino acid sequence set forth in SEQ ID NO: 100,
HCDR2 having the amino acid sequence set forth in SEQ ID NO: 101, and
HCDR3 having the amino acid sequence set forth in SEQ ID NO: 102,
a light chain variable region that has LCDR1 having the amino acid sequence set forth in SEQ ID NO: 103,
LCDR2 having the amino acid sequence set forth in SEQ ID NO: 104, and
LCDR3 having the amino acid sequence set forth in SEQ ID NO: 105.
(6) an antibody or an antigen-binding fragment thereof, including a heavy chain variable region that has HCDR1 having the amino acid sequence set forth in SEQ ID NO: 108,
HCDR2 having the amino acid sequence set forth in SEQ ID NO: 109, and
HCDR3 having the amino acid sequence set forth in SEQ ID NO: 110,
a light chain variable region that has LCDR1 having the amino acid sequence set forth in SEQ ID NO: 111,
LCDR2 having the amino acid sequence set forth in SEQ ID NO: 112, and
LCDR3 having the amino acid sequence set forth in SEQ ID NO: 113.
(7) an antibody or an antigen-binding fragment thereof, competing with the antibody according to any of (1) to (6) above for binding with TMPRSS2; or
(8) an antibody or an antigen-binding fragment thereof, including the amino acid sequence having one or several amino acid mutations in the CDR sequence of the antibody according to any of (1) to (6) above; or
(9) a human chimeric antibody or a humanized antibody of the antibody according to any of (1) to (8) above.

[9] A composition including the antibody according to any of [1] to [8] above or an antigen-binding fragment thereof.
[10] The composition according to [9] above, for use in preventing a subject in need thereof from being infected with coronavirus.
[11] A pharmaceutical composition according to [9] above, for use in administering to a subject with coronavirus.
[12] The composition according to [9] above, for use in treating coronavirus infection in a subject infected with a beta coronavirus.

[21] The antibody according to any of [1] to [8] above, wherein the antibody is a humanized antibody, or an antigen-binding fragment thereof.

[22] The antibody according to any of [1] to [8] above, wherein the antibody is a human chimeric monoclonal antibody, or an antigen-binding fragment thereof.

[23] A composition, including the antibody according to [21] or [22] above or an antigen-binding fragment thereof.

[24] The composition according to [23] above, for use in preventing a subject in need thereof from being infected with coronavirus.

[25] A pharmaceutical composition according to [23] above, for use in administering to a subject with coronavirus.

[26] The composition according to [23] above, for use in treating coronavirus infection in a subject infected with a beta coronavirus.

[101] The antibody or composition according to any of the above, wherein the coronavirus is a beta coronavirus.

[102] The antibody or composition according to any of the above, wherein the coronavirus is SARS-CoV, MERS, or SARS-CoV-2.

[103] The antibody or composition according to any of the above, wherein the coronavirus is one or more selected from the alpha variant, the beta variant, the gamma variant, the delta variant, and the omicron variant of SARS-CoV-2.

[111] The antibody or composition according to any of the above, wherein TMPRSS2 is human TMPRSS2, and the cell is a human cell.

[112] The antibody or composition according to [101] above, wherein TMPRSS2 is human TMPRSS2, and the cell is a human cell.

[113] The antibody or composition according to [102] above, wherein TMPRSS2 is human TMPRSS2, and the cell is a human cell.

[114] The antibody or composition according to [103] above, wherein TMPRSS2 is human TMPRSS2, and the cell is a human cell.

[121] The antibody or an antigen-binding fragment thereof according to [1] above, wherein a binding affinity (KD) to the human TMPRSS2 of the antibody or the antigen-binding fragment is $10^{-8}$ M or less, and $IC_{90}$ referring to inhibition of the coronavirus infection of human cells is 10 $\mu$g/mL or less.

[122] The antibody or an antigen-binding fragment thereof according to [1] above, wherein the binding affinity (KD) to the human TMPRSS2 of the antibody or the antigen-binding fragment is $10^{-8}$ M or less, and the antibody or the antigen-binding fragment binds to the amino acid sequence set forth in SEQ ID NO: 85 (GVYGNVMVFTDWIY).

[123] The antibody or an antigen-binding fragment thereof according to [1] above, wherein $IC_{90}$ referring to inhibition of the coronavirus infection of human cells of the antibody or the antigen-binding fragment is 10 $\mu$g/mL or less, and the antibody or the antigen-binding fragment binds to the amino acid sequence set forth in SEQ ID NO: 85 (GVYGNVMVFTDWIY).

[124] The antibody or an antigen-binding fragment thereof according to [1] above, wherein the binding affinity (KD) to the human TMPRSS2 of the antibody or the antigen-binding fragment is $10^{-8}$ M or less, $IC_{90}$ referring to inhibition of the coronavirus infection of human cells is 10 $\mu$g/mL or less, and the antibody or the antigen-binding fragment binds to the amino acid sequence set forth in SEQ ID NO: 85 (GVYGNVMVFTDWIY).

[131] The antibody or an antigen-binding fragment thereof according to [121] above, competing with the antibody according to any of (2) to (4) above for binding to TMPRSS2.

[132] The antibody or an antigen-binding fragment thereof according to [122] above, competing with the antibody according to any of (2) to (4) above for binding to TMPRSS2.

[133] The antibody or an antigen-binding fragment thereof according to [123] above, competing with the antibody according to any of (2) to (4) above for binding to TMPRSS2.

[134] The antibody or an antigen-binding fragment thereof according to [124] above, competing with the antibody according to any of (2) to (4) above for binding to TMPRSS2.

[135] The antibody or an antigen-binding fragment thereof according to [121] above, competing with the antibody according to (1) above for binding to TMPRSS2.

[136] The antibody or an antigen-binding fragment thereof according to [122] above, competing with the antibody according to (1) above for binding to TMPRSS2.

[137] The antibody or an antigen-binding fragment thereof according to [123] above, competing with the antibody according to (1) above for binding to TMPRSS2.

[138] The antibody or an antigen-binding fragment thereof according to [124] above, competing with the antibody according to (1) above for binding to TMPRSS2.

[141] An antibody or an antigen-binding fragment thereof that binds to an extracellular domain of TMPRSS2, capable of inhibiting coronavirus infection of cells and inhibiting an action of TMPRSS2 on an S protein.

[142] An antibody or an antigen-binding fragment thereof that binds to an extracellular domain of TMPRSS2, capable of inhibiting coronavirus infection of cells and inhibiting an interaction of TMPRSS2 with an S protein.

[151] A composition including the antibody according to any of the above or an antigen-binding fragment thereof.

[152] The composition according to [151] above, for use in preventing cells from being infected with coronavirus.

[153] The composition according to [151] above, for use in preventing the coronavirus infection.

[154] The composition according to [151] above, for use in treating a subject infected with coronavirus.

[161] A method for treating a subject, including administering to the subject an effective amount of the antibody according to any of the above or an antigen-binding fragment thereof.

[162]The method according to [161] above, wherein a subject carries coronavirus.

[163] The method according to [161] or [162] above, wherein further coronavirus infection of cells in the body of a subject is suppressed.

[164] A method for treating a subject infected with coronavirus, including administering to the subject an effective amount of the antibody according to any of the above or an antigen-binding fragment thereof.

[171] The antibody according to the above or an antigen-binding fragment thereof, or a pharmaceutical composition including the antibody or an antigen-binding fragment thereof, for use in the method according to any of [161] to [164] above.

[172] Use of the antibody according to the above or an antigen-binding fragment thereof, in manufacture of medicament for use in the method according to any of [161] to [164] above.

Brief Description of Drawings

[Figure 1] Figure 1 shows that TMPRSS2 which is essential for beta coronavirus infection of cells is expressed on a membrane surface of an infected cell (host cell), and that the antibody of the present invention binds to TMPRSS2, which is expressed on the membrane surface of the infected cell. The antibody of the present invention binds to an antigen that is expressed in a host cell, and it is therefore theoretically unaffected by viral mutation.
[Figure 2] Figure 2 shows the binding properties to human TMPRSS2 of the monoclonal antibodies that bind to human TMPRSS2 newly obtained in Examples. The human TMPRSS2 was expressed in Daudi cell lines, and the binding between the antibodies and the cells was detected by flow cytometry. Data on the antibodies whose bindings were particularly good were enclosed in square frames.
[Figure 3A] Figure 3A shows results from a test system that tests whether or not the monoclonal antibodies that bind to human TMPRSS2 newly obtained in Examples compete with a 752_1 antibody for binding to the human TMPRSS2.
[Figure 3B] Figure 3B shows results from the test system which tests whether or not the monoclonal antibodies that bind to human TMPRSS2 newly obtained in Examples compete with a 2228_15 antibody for binding to the human TMPRSS2.
[Figure 3C] Figure 3C shows results from the test system that tests whether or not the monoclonal antibodies that bind to human TMPRSS2 newly obtained in Examples compete with an 1831_15 antibody for binding to the human TMPRSS2.
[Figure 3D] Figure 3D shows results from the test system that tests whether or not the monoclonal antibodies that bind to human TMPRSS2 newly obtained in Examples compete with an 1864_10 antibody for binding to the human TMPRSS2.
[Figure 4] Figure 4 is data showing the binding properties of various monoclonal antibodies to 20 amino acid-long peptides that were designed by shifting 2 amino acids each in a whole area of the human TMPRSS2.
[Figure 5A] Figure 5A shows the binding properties of each monoclonal antibody to each peptide.
[Figure 5B] Figure 5B shows the binding properties of each monoclonal antibody to each peptide.
[Figure 6] Figure 6 shows epitope regions of the antibody on human TMPRSS2 predicted from the results of Figures 5A and 6B.

[Figure 7A] Figure 7A is data showing the binding properties between each monoclonal antibody and the human TMPRSS2 variants.

[Figure 7B] Figure 7B is data showing the binding properties between each monoclonal antibody and the human TMPRSS2 variants.

[Figure 7C] Figure 7C is data showing the binding properties between each monoclonal antibody and the human TMPRSS2 variants.

[Figure 7D] Figure 7D is data showing the binding properties between each monoclonal antibody and the human TMPRSS2 variants.

[Figure 8] Figure 8 shows three-dimensional structure models of the epitope region obtained by observing complexes of a Fab fragment of the 752_1 antibody, a Fab fragment of the 2228 antibody. and the human TMPRSS2 with a cryo-electron microscope. Graphics in string-shape shows human TMPRSS2, and a surface model drawn in the back shows an antibody.

[Figure 9] Figure 9 shows the binding properties of the obtained various antibodies to other TMPRSS family members.

[Figure 10A] Figure 10A shows amino acid sequence alignment results of TMPRSS2 from each animal species.

[Figure 10B] Figure 10B shows amino acid sequence alignment results of TMPRSS2 from each animal species.

[Figure 10C] Figure 10C shows the binding properties of each monoclonal antibody to mouse, cynomolgus monkey, or human TMPRSS2.

[Figure 10D] Figure 10D shows the binding properties of each monoclonal antibody to mouse or human TMPRSS2.

[Figure 11A] Figure 11A shows the binding properties of human chimeric antibodies obtained by replacing the Fc regions of the obtained monoclonal antibodies with the Fc region of human IgG4, to human TMPRSS2.

[Figure 11B] Figure 11B shows the binding properties of human chimeric antibodies derived from the 752_1 antibody obtained by replacing the Fc region thereof with the Fc region of human IgG4, to human and cynomolgus monkey TMPRSS2.

[Figure 12] Figure 12 shows inhibitory effects on viral infection by the obtained various monoclonal antibodies.

[Figure 13A] Figure 13A shows infection inhibiting effects on various virus variants by the obtained various monoclonal antibodies.

[Figure 13B] Figure 13B shows infection inhibiting effects on various virus variants by the obtained various monoclonal antibodies.

[Figure 13C] Figure 13C shows infection inhibiting effects on various virus variants by the obtained various monoclonal antibodies.

[Figure 13D] Figure 13D shows infection inhibiting effects on various virus variants by the obtained various monoclonal antibodies.

[Figure 14] Figure 14 shows inhibitory effects on cell fusion (fusion between TMPRSS2/ACE-2 expressing 293T cells and spike protein expressing 293T cells) by the obtained various monoclonal antibodies. The fact that the cell fusion was inhibited suggests that virus entry to cells is inhibited.

[Figure 15] Figure 15 shows measurement results of antibody-dependent cell cytotoxicity (ADCC) of Antibody-Dependent Effect (ADE) substituted (L244A/E245A/P339A) recombinant antibodies in the Fc regions of the obtained monoclonal antibodies.

[Figure 16] Figure 16 shows measurement results of antibody-dependent cell cytotoxicity (ADCC) of the obtained human chimeric monoclonal antibodies.

[Figure 17] Figure 17 shows results of SARS-CoV-2 infection inhibition experiment on cells expressing human or macaque TMPRSS2.

[Figure 18A] Figure 18A shows an experimental scheme for inhibitory effects of antibody administration on SARS-CoV-2 infection of cynomolgus monkeys.

[Figure 18B] Figure 18B shows changes in body temperature and body weight of individuals after administering antibodies against SARS-CoV-2 infection of cynomolgus monkeys.

[Figure 19A] Figure 19A shows measurement results (by titration method) of the number of viruses in the airway and bronchus swabs of the cynomolgus monkeys.

[Figure 19B] Figure 19B shows measurement results (by RT-PCR method) of the number of viruses in the bronchus swab of the cynomolgus monkeys.

[Figure 20] Figure 20 shows results of lung histopathologic diagnosis score of the cynomolgus monkeys by hematoxylin-eosin (HE) staining and immunohistochemical staining of SARS-CoV-2 N protein.

[Figure 21] Figure 21 shows results of binding tests for the prepared humanized antibodies to TMPRSS2 expressing cells.

[Figure 22] Figure 22 shows results of an experiment of inhibiting infection of cells with pseudo-typed virus by the prepared humanized antibodies.

Description of Embodiments

<Definition>

As used herein, the "subject" is a vertebrate, and for example, may be a mammal including a human, such as a mammal to be infected with coronavirus (e.g., SARS-CoV-2) (cats, ferrets, bats, and pangolins). The subject may be a subject infected with coronavirus (e.g., SARS-CoV-2), may be an asymptomatic carrier infected with coronavirus (e.g., SARS-CoV-2), and may be a subject infected with coronavirus (e.g., SARS-CoV-2) and developed COVID-19. The subject may be a subject who has a possibility (is at a risk) of having been infected with coronavirus (e.g., SARS-CoV-2), or may be a subject who has a possibility (is at a risk) of being infected with coronavirus (e.g., SARS-CoV-2). The subject may be a young child (e.g., infant (1- to 6-year-old), a schoolchild (6- to 12-year-old), an adolescent (12-year-old or above), or an adult (20-year-old or above). The adult may be an adult of 30-year-old or above, 40-year-old or above, 50-year-old or above, 60-year-old or above, or 70-year-old or above.

As used herein, the "coronavirus" is a virus belonging to the subfamily Ortho coronavirinae in the family Coronaviridae and the order Nidovirales, and a single-stranded plus-strand RNA virus. Coronaviruses have spike protein projections (S protein) on the surface of the viruses and was named as coronavirus because the appearance resembles the solar corona. In humans, it causes respiratory infection including common cold. The viruses belonging to the subfamily Ortho coronavirinae are classified into Alphacoronavirus, Beta coronavirus, Gamma coronavirus and Delta coronavirus. SARS-related coronaviruses are classified in Beta coronavirus. The SARS-related coronaviruses include SARS-coronavirus (SARS-CoV) and SARS-coronavirus 2 (SARS-CoV-2). SARS-CoV-2 has been causing novel coronavirus infection (COVID-19) pandemic since the end of 2019. The SARS-related coronaviruses infect host cells by binding to ACE2 receptors of the host cells through S proteins. The SARS-related coronaviruses have a common infection mechanism in that they infect cells using the ACE2 receptors. The S proteins of SARS-CoV-2 have furin cleavage sites that increase infectivity and pathogenicity therein (Andersen et al., Nature Medicine, 26, 450-452, 2020). Coronaviruses are known to cause respiratory infection such as common cold in humans, and SARS coronavirus (SARS-CoV), MERS coronavirus (MERS-CoV), and 2019 novel coronavirus (SARS-CoV-2) have lethality. As other coronaviruses, lethal viruses are known such as mouse hepatitis virus (MHV) and feline infectious peritonitis virus (FIPV). Coronaviruses infect cells by binding to target cell surfaces through the binding of the spike proteins exposed on the envelope surfaces and angiotensin-converting enzymes 2 (ACE2) in cell surface molecules, and then by being taken into the cells by endocytosis. Examples of coronaviruses include coronaviruses belonging to the subfamily Coronavirinae, i.e., Alphacoronavirus (e.g., canine coronavirus, alphacoronavirus 1, human coronavirus 229E, human coronavirus NL63, and porcine epidemic diarrhea virus), Beta coronavirus (e.g., subgenus Embecovirus, subgenus Sarbecovirus, subgenus Merbecovirus, and subgenus Nobecovirus, such as human enteric coronavirus 4408, human coronavirus OC43, mouse coronavirus, human coronavirus HKU1, SARS-related coronavirus (e.g., SARS coronavirus (SARS-CoV), 2019 novel coronavirus (SARS-CoV-2), and MERS coronavirus, and equine coronavirus), Gamma coronavirus (e.g., avian coronavirus, and beluga whale coronavirus SW1), and Delta coronavirus (e.g., bulbul coronavirus HKU11, munia coronavirus HKU13, and thrush coronavirus HKU12). Although vaccines against coronavirus have been developed, these are vaccines against antigens that coronavirus has, such as S proteins, and therefore, theoretically it cannot be said that these vaccines maintain their effectiveness against mutation of coronavirus.

As used herein, "SARS-CoV-2" is the coronavirus that caused the pandemic that occurred in 2020. On January 7, 2020, The World Health Organization (WHO) provisionally named this virus 2019-nCov. Also, on February 11 of the same year, The International Committee on Taxonomy of Viruses (ICTV) officially named this virus SARS-CoV-2. Coronaviruses can cause illnesses ranging from common cold to serious respiratory diseases such as severe acute respiratory syndrome (SARS) or Middle East respiratory syndrome (MERS). The WHO named the disease caused by this novel coronavirus COVID-19. The International Committee on Taxonomy of Viruses determined that SARS-CoV-2 belongs to the genus Beta coronavirus and is the same species as SARS-CoV (or its sister lineage). The complete genomic sequence of SARS-CoV-2 is registered to the National Center for Biotechnology Information (NCBI) under GenBank Accession Number: MN908947.3. Virus particles (virions) have particle sizes of about 50 to 200 nm and, similar to common coronaviruses, contain spike proteins, nucleocapsid protein, membrane proteins and envelope proteins, and viral genome RNA. Nucleocapsid proteins form a complex with RNA, around which spike proteins, membrane proteins and envelope proteins that are bound with lipid surround to form a virion envelope. The spike protein located on the outermost surface of the envelope is thought to facilitate the infection of cells by binding to an ACE2 receptor on the cell surface. There are people who do not show symptoms of the disease despite infection with SARS-CoV-2, and they are called asymptomatic carriers. It is pointed out that asymptomatic carriers have a possibility to infect others with the virus they carry. It is pointed out that because of infection with SARS-CoV-2, senses of smell and/or taste are decreased or lost. SARS-CoV-2 may cause severe acute respiratory syndrome. As the main symptoms of severe acute respiratory syndrome, fever of around 40°C, cough and shortness of breath have been reported. Significant complication is pneumonia. The presence or absence of SARS-CoV-2 infection is mainly determined by PCR testing. The PCR test is to evaluate whether or not the SARS-CoV-2 gene is present in the body, depending on whether or not a band is amplified specific to SARS-CoV-2. Examples of the treatment for SARS-CoV-2 include antiviral drugs against SARS-CoV-2 (e.g., remdesivir), steroidal anti-inflammatory drugs (e.g., dexamethasone), and inhibitors of proinflammatory cytokines (e.g., IL-6 inhibitors such as anti-IL-6 antibody, TNF-$\alpha$ inhibitor such as etanercept).

As used herein, the "spike protein" can be a protein to be encoded at positions 21563 to 25384 of SARS-CoV-2 genome registered to the National Center for Biotechnology Information (NCBI) under GenBank Accession Number: MN908947.3, or a protein having the amino acid sequence set forth in SEQ ID NO: 1; and the above spike protein of SARS-CoV-2 has the amino acid sequence registered to NCBI under GenBank Accession Number: QHD43416.1. The spike proteins include S1 and S2; S1 is present at positions 13 to 541 of the above amino acid sequence, and S2 is present at positions 543 to 1208 of the above amino acid sequence. S1 further has an N-terminal domain (NTD) and a receptor binding domain (RBD); NTD is present at positions 13 to 304 of the above amino acid sequence, and RBD is present at positions 319 to 541. S1 and S2 are cleaved in the cell, produced as separate peptides, and form a complex during virus particle formation. The spike protein is also referred to as S protein. As the spike protein, any spike protein that natural virus (e.g., including virus variants) has (those having the amino acid sequence corresponding to the amino acid sequence registered to NCBI under GenBank Accession Number: QHD43416.1) can be used.

As used herein, the "TMPRSS2" is a cell membrane protein called transmembrane protease, serine 2 (type II transmembrane serine protease), and is expressed in the prostate and airway epithelium. TMPRSS2 is expressed as an inactive precursor, and is converted into an active type by self-cleavage at the QSR sequence. Further, the active TMPRSS2 has been confirmed to cleave and activate respiratory viruses such as influenza A virus, influenza B virus, Sendai virus, human parainfluenza 1 to 4, and human metapneumovirus. However, for these viruses, TMPRSS2 is involved in the formation of virus particles produced in the cell and the like but is not involved in the invasion of these viruses into cells. On the other hand, TMPRSS2 does not particularly act against coronaviruses in the cell, but it acts on the S protein of coronaviruses that are entering cells from outside of the cells and cleaves and activates the S protein to facilitate cell entry of coronaviruses. TMPRSS2 knockout mice exhibit a healthy phenotype similar to that of normal mice (Kim TS. et al., Mol. Cell Bio., 26: 965-975, 2006; and Sakai K. et al., J. Viol., 88: 5608-5616, 2014). Therefore, when inhibiting functions of TMPRSS2 though, there will be little or no adverse effect on the living body. Ferret TMPRSS2 has, for example, the amino acid sequence set forth in SEQ ID NO: 1 or the amino acid sequence corresponding thereto. Hamster TMPRSS2 has, for example, the amino acid sequence set forth in SEQ ID NO: 2 or the amino acid sequence corresponding thereto. Mouse TMPRSS2 has, for example, the amino acid sequence set forth in SEQ ID NO: 3 or the amino acid sequence corresponding thereto. Feline TMPRSS2 has, for example, the amino acid sequence set forth in SEQ ID NO: 4 or the amino acid sequence corresponding thereto. Human TMPRSS2 has, for example, the amino acid sequence set forth in SEQ ID NO: 5 or the amino acid sequence corresponding thereto. Green monkey TMPRSS2 has, for example, the amino acid sequence set forth in SEQ ID NO: 6 or the amino acid sequence corresponding thereto. Cynomolgus monkey TMPRSS2 has, for example, the amino acid sequence set forth in SEQ ID NO: 7 or the amino acid sequence corresponding thereto. Rhesus macaque TMPRSS2 has, for example, the amino acid sequence set forth in SEQ ID NO: 8 or the amino acid sequence corresponding thereto. Human TMPRSS2 includes a cytoplasmic region on the N-terminal side, a transmembrane region for 85th to 105th amino acids, an extracellular domain for amino acids thereafter, an LDLRA region for 112th to 149th amino acids, an SRCR region for 150th to 242nd amino acids, and a serine protease region for 255th to 492nd amino acids. The serine protease region contains catalytic triad of H296, D345, and S441.

As used herein, the phrase "amino acid sequence corresponding to the amino acid sequence of SEQ ID NO: n" refers to a sequence corresponding to the amino acid sequence of SEQ ID NO: n {where n is a natural number} when aligning two amino acid sequences.

As used herein, the term "peptide" refers to an amino acid polymer. Polymers usually have no branches. The term "partial peptide" refers to a part of a particular peptide. Peptides and partial peptides can be produced from nucleic acids that encode the peptides. Peptides and partial peptides can also be chemically synthesized. Peptides and partial peptides are also isolated, concentrated or purified. Isolation means to separate peptides and partial peptides from at least other components, and purification means to separate peptides and partial peptides at least selectively. Concentration means that the concentration of peptides and partial peptides is increased.

As used herein, the term "antibody" refers to an immunoglobulin and is a protein having a structure wherein two heavy chains (H chains) and two light chains (L chains) both of which are stabilized by a pair of disulfide bonds, are associated with each other. The heavy chain consists of a heavy chain variable region VH, a heavy chain constant regions CH1, CH2 and CH3, and a hinge region located between CH1 and CH2; and the light chain consists of a light chain variable region VL, and a light chain constant region CL. Among these, a variable region fragment (Fv) consisting of VH and VL is a region directly involved in the antigen binding and adding diversity to the antibody. Also, the antigen-binding region consisting of VL, CL, VH and CH1 is referred to as Fab region, and a region consisting of the hinge region, CH2 and CH3 is referred to as Fc region.

Within the variable region, a region that directly contacts the antigen is particularly variable and is referred to complementarity-determining region (CDR). Portions other than CDR with relatively less mutations are referred to as framework regions (FR). There are 3 CDRs in each of the light and heavy chain variable regions, which are respectively referred to, from the N-terminal side, heavy chain CDR1 to 3 and light chain CDR1 to 3.

The antibody may be a monoclonal antibody or polyclonal antibody. Also, the antibody may be any isotype among IgG, IgM, IgA, IgD, and IgE. The antibody may be produced by immunizing a non-human animal, such as mouse, rat, hamster, guinea pig, rabbit, or chicken. The antibody may be a recombinant antibody. The antibody may be a chimeric antibody, humanized antibody, fully humanized antibody, human antibody, or the like.

The chimeric antibody is an antibody wherein fragments of antibodies derived from different species are linked. The chimeric antibody may be a human chimeric antibody wherein at least the constant region is derived from a human antibody.

The term "humanized antibody" refers to an antibody wherein the corresponding position of a non-human antibody is substituted with the amino acid sequence (e.g., framework region) characteristic of a human-derived antibody, and examples thereof include those having the heavy chain CDR1 to 3 and the light chain CDR1 to 3 of the antibody produced by immunizing a mouse or rat, and wherein all other regions including 4 framework regions (FR) of each of the heavy chain and the light chain are derived from a human antibody. Such an antibody may be referred to CDR-grafted antibody.

The "human chimeric antibody" is, in a non-human-derived antibody, an antibody wherein the constant region of the non-human-derived antibody is substituted for the constant region of a human antibody. For the human chimeric antibody, from the viewpoint of enhancing ADCC activity, for example, the subtype of the human antibody used for the constant region may be IgG1.

As used herein, the "composition" is a mixture of one or more components. The composition can contain, for example, a partial peptide and an aqueous solvent (e.g., water). The composition can further contain a pharmaceutically acceptable additive (e.g., excipient and/or carrier). The composition used to treat a subject is referred to as pharmaceutical composition. The antibody is usually present in the form of a composition. The isolated antibody can be contained in the composition together with other pharmaceutically acceptable additives.

As used herein, the "treatment" includes prophylactic treatment and therapeutic treatment. The therapeutic treatment can be directed against infected viruses, whereas the prophylactic treatment can be conducted in order to prevent future infection, to delay the onset of coronavirus infection (e.g., COVID-19) due to infection, to reduce, delay deterioration of, or cease deterioration of the symptoms of the coronavirus infection (e.g., COVID-19) that has developed, or to prevent the exacerbation. The therapeutic treatment can be administered to symptomatic patients or asymptomatic carriers. The prophylactic treatment can be administered to non-infected individuals. The therapeutic treatment can be administered to infected individuals.

As used herein, the phrase "effective amount" refers to an amount of active ingredients that achieve expected pharmacological effects.

<Antibody of The Present Invention or Antigen-Binding Fragment Thereof>

According to the present invention, provided is an antibody that binds to an extracellular domain of TMPRSS2, the antibody being capable of inhibiting coronavirus infection of cells, or an antigen-binding fragment thereof. In an embodiment, the antibody of the present invention can inhibit an interaction between TMPRSS2 and an S protein, resulting in inhibiting coronavirus infection of cells. In a preferred embodiment, TMPRSS2 can be human TMPRSS2. The inhibition of TMPRSS2 by the antibody of the present invention is considered not to affect the healthy phenotype of an individual and does not cause unacceptable side effects to the living body.

In an embodiment of the present invention, the antibody of the present invention can bind to (i) one or more extracellular domains of TMPRSS2 selected from the group consisting of mice, green monkeys, cats, hamsters, and macaque monkeys, as well as to (ii) human TMPRSS2. In a preferred embodiment, the antibody of the present invention can bind to mouse TMPRSS2 and human TMPRSS2. In a preferred embodiment, the antibody of the present invention can bind to macaque monkey TMPRSS2 and human TMPRSS2. In a preferred embodiment, the antibody of the present invention can bind to green monkey TMPRSS2 and human TMPRSS2.

In an embodiment of the present invention, the antibody of the present invention may have a binding affinity (KD) to human TMPRSS2 of $10^{-6}$ M or less, $10^{-7}$ M or less, $10^{-8}$ M or less, $10^{-9}$ M or less, or $10^{-10}$ M or less.

In an embodiment of the present invention, the antibody of the present invention may have IC90 referring to inhibition of coronavirus infection of human cells of 100 μg/mL or less, 90 μg/mL or less, 80 μg/mL or less, 70 μg/mL or less, 60 μg/mL or less, 50 μg/mL or less, 40 μg/mL or less, 30 μg/mL or less, 20 μg/mL or less, 10 μg/mL or less, 9 μg/mL or less, 8 μg/mL or less, 7 μg/mL or less, 6 μg/mL or less, 5 μg/mL or less, 4 μg/mL or less, 3 μg/mL or less, 2 μg/mL or less, or 1 μg/mL or less.

In an embodiment of the present invention, the antibody of the present invention can bind to a peptide having the amino acid sequence of any of SEQ ID NOs: 41 to 93. In an embodiment of the present invention, the antibody of the present invention can bind to a peptide having the amino acid sequence of any of SEQ ID NOs: 85 to 93. In an embodiment of the present invention, the antibody of the present invention can bind to a peptide having the amino acid sequence of SEQ ID NO: 85.

In an embodiment, the antibody of the present invention binds to human TMPRSS2 having the amino acid sequence set forth in SEQ ID NO: 5, and can bind to a peptide having the amino acid sequence consisting of the amino acid sequence selected from the group consisting of the amino acid sequence set forth in SEQ ID NO: 75 (WNENYGRAACRDMGYKNNFY), the amino acid sequence set forth in SEQ ID NO: 89 (VTAAHCVEKPLNNPWHWT), and the amino acid sequence set forth in SEQ ID NO: 96 (RQSFMF).

In an embodiment, the antibody of the present invention binds to human TMPRSS2 having the amino acid sequence set forth in SEQ ID NO: 5, and:

($\alpha$) binds to chimeric TMPRSS2 having the amino acid sequence wherein the amino acid sequence set forth in SEQ ID NO: 75 (WNENYGRAACRDMGYKNNFY) of the human TMPRSS2 is substituted for a corresponding sequence of a cat (i.e., the amino acid sequence set forth in SEQ ID NO: 94: WSEGYGRAACQDMGYRNSFY) with binding affinity less than that for the human TMPRSS2 having the amino acid sequence set forth in SEQ ID NO: 5 (e.g., KD value of less than 1-fold, 1/2 or less, 1/3 or less, 1/5 or less, 1/10 or less, 1/100 or less, or 1/1,000 or less), or does not significantly bind thereto;

($\beta$) binds to chimeric TMPRSS2 having the amino acid sequence wherein the amino acid sequence set forth in SEQ ID NO: 89 (VTAAHCVEKPLNNPWHWT) of the human TMPRSS2 is substituted for a corresponding sequence of a cat (i.e., the amino acid sequence set forth in SEQ ID NO: 95: VTAAHCVEEPLNNPRHWT) with binding affinity less than that for the human TMPRSS2 having the amino acid sequence set forth in SEQ ID NO: 5 (e.g., KD value of less than 1-fold, 1/2 or less, 1/3 or less, 1/5 or less, 1/10 or less, 1/100 or less, or 1/1,000 or less), or does not significantly bind thereto; or

($\gamma$) binds to chimeric TMPRSS2 having the amino acid sequence wherein the amino acid sequence set forth in SEQ ID NO: 96 (RQSFMF) of the human TMPRSS2 is substituted for a corresponding sequence of a hamster (i.e., the amino acid sequence set forth in SEQ ID NO: 97: SQSLMF) with binding affinity less than that for the human TMPRSS2 having the amino acid sequence set forth in SEQ ID NO: 5 (e.g., KD value of less than 1-fold, 1/2 or less, 1/3 or less, 1/5 or less, 1/10 or less, 1/100 or less, or 1/1,000 or less), or does not significantly bind thereto.

In a preferred embodiment, the antibody of the present invention binds to human TMPRSS2 having the amino acid sequence set forth in SEQ ID NO: 5, and
($\alpha$) binds to chimeric TMPRSS2 having the amino acid sequence wherein the amino acid sequence set forth in SEQ ID NO: 75 (WNENYGRAACRDMGYKNNFY) of the human TMPRSS2 is substituted for a corresponding sequence of a cat (i.e., the amino acid sequence set forth in SEQ ID NO: 94: WSEGYGRAACQDMGYRNSFY) with binding affinity less than that for the human TMPRSS2 having the amino acid sequence set forth in SEQ ID NO: 5 (e.g., KD value of less than 1-fold, 1/2 or less, 1/3 or less, 1/5 or less, 1/10 or less, 1/100 or less, or 1/1,000 or less), or does not significantly bind thereto.

In a preferred embodiment, the antibody of the present invention binds to human TMPRSS2 having the amino acid sequence set forth in SEQ ID NO: 5, and
($\beta$) binds to chimeric TMPRSS2 having the amino acid sequence wherein the amino acid sequence set forth in SEQ ID NO: 89 (VTAAHCVEKPLNNPWHWT) of the human TMPRSS2 is substituted for a corresponding sequence of a cat (i.e., the amino acid sequence set forth in SEQ ID NO: 95: VTAAHCVEEPLNNPRHWT) with binding affinity less than that for the human TMPRSS2 having the amino acid sequence set forth in SEQ ID NO: 5 (e.g., KD value of less than 1-fold, 1/2 or less, 1/3 or less, 1/5 or less, 1/10 or less, 1/100 or less, or 1/1,000 or less), or does not significantly bind thereto.

In a preferred embodiment, the antibody of the present invention binds to human TMPRSS2 having the amino acid sequence set forth in SEQ ID NO: 5, and
($\gamma$) binds to chimeric TMPRSS2 having the amino acid sequence wherein the amino acid sequence set forth in SEQ ID NO: 96 (RQSFMF) of the human TMPRSS2 is substituted for a corresponding sequence of a hamster (i.e., the amino acid sequence set forth in SEQ ID NO: 97: SQSLMF) with binding affinity less than that for the human TMPRSS2 having the amino acid sequence set forth in SEQ ID NO: 5 (e.g., KD value of less than 1-fold, 1/2 or less, 1/3 or less, 1/5 or less, 1/10 or less, 1/100 or less, or 1/1,000 or less), or does not significantly bind thereto.

In an embodiment, the antibody of the present invention binds to human TMPRSS2 having the amino acid sequence set forth in SEQ ID NO: 5, and:

(A) binds to a human TMPRSS2 variant having a point mutation at N179A with binding affinity less than that for the human TMPRSS2 having the amino acid sequence set forth in SEQ ID NO: 5 (e.g., KD value of less than 1-fold, 1/2 or less, 1/3 or less, 1/5 or less, 1/10 or less, 1/100 or less, or 1/1,000 or less), or does not significantly bind thereto; or
(B) binds to a human TMPRSS2 variant having a point mutation at W306A with binding affinity less than that for the human TMPRSS2 having the amino acid sequence set forth in SEQ ID NO: 5 (e.g., KD value of less than 1-fold, 1/2 or less, 1/3 or less, 1/5 or less, 1/10 or less, 1/100 or less, or 1/1,000 or less), or does not significantly bind thereto.

In an embodiment, the antibody of the present invention may be an antagonist antibody against TMPRSS2.
In an embodiment of the present invention, the antibody of the present invention or an antigen-binding fragment thereof can be:

(1) an antibody or an antigen-binding fragment thereof, including a heavy chain variable region that has HCDR1 having the amino acid sequence set forth in SEQ ID NO: 11,

HCDR2 having the amino acid sequence set forth in SEQ ID NO: 12, and
HCDR3 having the amino acid sequence set forth in SEQ ID NO: 13,
a light chain variable region that has LCDR1 having the amino acid sequence set forth in SEQ ID NO: 14,
LCDR2 having the amino acid sequence set forth in SEQ ID NO: 15, and
LCDR3 having the amino acid sequence set forth in SEQ ID NO: 16.

(2) an antibody or an antigen-binding fragment thereof, including a heavy chain variable region that has HCDR1 having the amino acid sequence set forth in SEQ ID NO: 19,

HCDR2 having the amino acid sequence set forth in SEQ ID NO: 20, and
HCDR3 having the amino acid sequence set forth in SEQ ID NO: 21,
a light chain variable region that has LCDR1 having the amino acid sequence set forth in SEQ ID NO: 22,
LCDR2 having the amino acid sequence set forth in SEQ ID NO: 23, and
LCDR3 having the amino acid sequence set forth in SEQ ID NO: 24.

(3) an antibody or an antigen-binding fragment thereof, including a heavy chain variable region that has HCDR1 having the amino acid sequence set forth in SEQ ID NO: 27,

HCDR2 having the amino acid sequence set forth in SEQ ID NO: 28, and
HCDR3 having the amino acid sequence set forth in SEQ ID NO: 29,
a light chain variable region that has LCDR1 having the amino acid sequence set forth in SEQ ID NO: 30,
LCDR2 having the amino acid sequence set forth in SEQ ID NO: 31, and
LCDR3 having the amino acid sequence set forth in SEQ ID NO: 32.

(4) an antibody or an antigen-binding fragment thereof, including a heavy chain variable region that has HCDR1 having the amino acid sequence set forth in SEQ ID NO: 35,

HCDR2 having the amino acid sequence set forth in SEQ ID NO: 36, and
HCDR3 having the amino acid sequence set forth in SEQ ID NO: 37,
a light chain variable region that has LCDR1 having the amino acid sequence set forth in SEQ ID NO: 38,
LCDR2 having the amino acid sequence set forth in SEQ ID NO: 39, and
LCDR3 having the amino acid sequence set forth in SEQ ID NO: 40.

(5) an antibody or an antigen-binding fragment thereof, including a heavy chain variable region that has HCDR1 having the amino acid sequence set forth in SEQ ID NO: 100,

HCDR2 having the amino acid sequence set forth in SEQ ID NO: 101, and
HCDR3 having the amino acid sequence set forth in SEQ ID NO: 102,
a light chain variable region that has LCDR1 having the amino acid sequence set forth in SEQ ID NO: 103,
LCDR2 having the amino acid sequence set forth in SEQ ID NO: 104, and
LCDR3 having the amino acid sequence set forth in SEQ ID NO: 105.

(6) an antibody or an antigen-binding fragment thereof, including a heavy chain variable region that has HCDR1 having the amino acid sequence set forth in SEQ ID NO: 108,

HCDR2 having the amino acid sequence set forth in SEQ ID NO: 109, and
HCDR3 having the amino acid sequence set forth in SEQ ID NO: 110,
a light chain variable region that has LCDR1 having the amino acid sequence set forth in SEQ ID NO: 111,
LCDR2 having the amino acid sequence set forth in SEQ ID NO: 112, and
LCDR3 having the amino acid sequence set forth in SEQ ID NO: 113.

(7) an antibody or an antigen-binding fragment thereof, competing with the antibody according to any of (1) to (6) above for binding with TMPRSS2; or
(8) an antibody or an antigen-binding fragment thereof, including the amino acid sequence having one or several amino acid mutations in the CDR sequence of the antibody according to any of (1) to (6) above; or
(9) a human chimeric antibody or a humanized antibody of the antibody according to any of (1) to (8) above.

In an embodiment of the present invention, the antibody of the present invention or an antigen-binding fragment thereof may be an antibody or an antigen-binding fragment thereof:

(7) having a heavy chain variable region having the amino acid sequence set forth in SEQ ID NO: 9, and a light chain variable region having the amino acid sequence set forth in SEQ ID NO: 10 {e.g., having a heavy chain variable region and a light chain variable region of the 752_1 antibody},
(8) having a heavy chain variable region having the amino acid sequence set forth in SEQ ID NO: 17, and a light chain variable region having the amino acid sequence set forth in SEQ ID NO: 18 {e.g., having a heavy chain variable region and a light chain variable region of the 1864_10 antibody},
(9) having a heavy chain variable region having the amino acid sequence set forth in SEQ ID NO: 25, and a light chain variable region having the amino acid sequence set forth in SEQ ID NO: 26 {e.g., having a heavy chain variable region and a light chain variable region of the 2020_12 antibody},
(10) having a heavy chain variable region having the amino acid sequence set forth in SEQ ID NO: 33, and a light chain variable region having the amino acid sequence set forth in SEQ ID NO: 34 {e.g., having a heavy chain variable region and a light chain variable region of the 2228_15 antibody},
(11) having a heavy chain variable region having the amino acid sequence set forth in SEQ ID NO: 98, and a light chain variable region having the amino acid sequence set forth in SEQ ID NO: 99 {e.g., having a heavy chain variable region and a light chain variable region of the 1831_15 antibody},
(12) competing with the antibody according to (7) above for binding with TMPRSS2.
(12) competing with the antibody according to (10) above for binding with TMPRSS2.
(13) competing with the antibody according to (11) above for binding with TMPRSS2; or (14) competing with the antibody according to any of (7) to (10) above for binding with TMPRSS2.

In a preferred embodiment of the present invention, the antibody of the present invention binds to human TMPRSS2 and competes with the antibody according to (7) above for binding with TMPRSS2. Accordingly, it is possible to suppress infection of human cells with beta coronavirus including SARS-CoV-2.
In a preferred embodiment of the present invention, the antibody of the present invention binds to human TMPRSS2 and competes with the antibody according to (10) above for binding with TMPRSS2. Accordingly, it is possible to suppress infection of human cells with beta coronavirus including SARS-CoV-2.
In a preferred embodiment of the present invention, the antibody of the present invention binds to human TMPRSS2 and competes with the antibody according to (11) above for binding with TMPRSS2. Accordingly, it is possible to suppress infection of human cells with beta coronavirus including SARS-CoV-2.
In an embodiment of the present invention, the antibody of the present invention may be an antagonist antibody against TMPRSS2. In an embodiment of the present invention, the antibody of the present invention inhibits the activating effect of TMPRSS2 on the S protein of coronavirus. In an embodiment of the present invention, the antibody of the present invention inhibits the interaction between TMPRSS2 and the S protein of coronavirus. In an embodiment of the present invention, the antibody of the present invention does not inhibit the interaction between TMPRSS2 and the S protein of coronavirus.
In an embodiment, the antibody of the present invention may be at least one of IgG1, IgG2, IgG3, IgG4, IgE, IgA, IgD, and IgM. In an embodiment, the antibody of the present invention contains a human x light chain constant region and/or a human heavy chain. In an embodiment, the antibody of the present invention contains a human λ light chain constant region and/or a human heavy chain. In an embodiment, the antibody of the present invention contains a human x light chain constant region and/or a human IgG2 heavy chain constant region. In an embodiment, the antibody of the present invention contains a human λ light chain and/or a human IgG4 heavy chain constant region. In an embodiment, the antibody of the present invention contains a human λ light chain constant region and/or a human IgG2 heavy chain constant region. In an embodiment, the antibody of the present invention contains a human x light chain and/or a human IgG4 heavy chain constant region.
In a preferred embodiment of the present invention, the antibody of the present invention does not have significant ADCC activity. In an embodiment, the antibody of the present invention may be a human chimeric antibody, humanized antibody, or human antibody. In an embodiment, the subtypes of the human chimeric antibody, humanized antibody, or human antibody may be IgG4. In an embodiment, IgG4 may have serine at position 108 from the N-terminal of the CH1 region being substituted for proline in the CH1 region. In an embodiment, IgG4 may have serine at position 120 from the N-terminal of the CH1 region being substituted for proline in the CH1 region.

In an embodiment, the humanized antibody has a light chain variable region containing a framework region 1 having the amino acid sequence of SEQ ID NO: 115 or 120, a framework region 2 having the amino acid sequence of SEQ ID NO: 116 or 121, a framework region 3 having the amino acid sequence of SEQ ID NO: 117 or 122, and a framework region 4 having the amino acid sequence of SEQ ID NO: 118 or 123.

In an embodiment, the humanized antibody has a light chain variable region containing a framework region 1 having the amino acid sequence of SEQ ID NO: 115, a framework region 2 having the amino acid sequence of SEQ ID NO: 116, a framework region 3 having the amino acid sequence of SEQ ID NO: 117, and a framework region 4 having the amino acid sequence of SEQ ID NO: 118.

In an embodiment, the humanized antibody has a light chain variable region containing a framework region 1 having the amino acid sequence of SEQ ID NO: 120, a framework region 2 having the amino acid sequence of SEQ ID NO: 121, a framework region 3 having the amino acid sequence of SEQ ID NO: 122, and a framework region 4 having the amino acid sequence of SEQ ID NO: 123.

In an embodiment, the humanized antibody has a heavy chain variable region containing a framework region 1 having the amino acid sequence of SEQ ID NO: 125 or 130, a framework region 2 having the amino acid sequence of SEQ ID NO: 126 or 131, a framework region 3 having the amino acid sequence of SEQ ID NO: 127 or 132, and a framework region 4 having the amino acid sequence of SEQ ID NO: 128 or 133.

In an embodiment, the humanized antibody has a heavy chain variable region containing a framework region 1 having the amino acid sequence of SEQ ID NO: 125, a framework region 2 having the amino acid sequence of SEQ ID NO: 126, a framework region 3 having the amino acid sequence of SEQ ID NO: 127, and a framework region 4 having the amino acid sequence of SEQ ID NO: 128.

In an embodiment, the humanized antibody has a heavy chain variable region containing a framework region 1 having the amino acid sequence of SEQ ID NO: 130, a framework region 2 having the amino acid sequence of SEQ ID NO: 131, a framework region 3 having the amino acid sequence of SEQ ID NO: 132, and a framework region 4 having the amino acid sequence of SEQ ID NO: 133.

In an embodiment, the humanized antibody has:

a heavy chain variable region containing a framework region 1 having the amino acid sequence of SEQ ID NO: 125 or 130, a framework region 2 having the amino acid sequence of SEQ ID NO: 126 or 131, a framework region 3 having the amino acid sequence of SEQ ID NO: 127 or 132, and a framework region 4 having the amino acid sequence of SEQ ID NO: 128 or 133; and

a light chain variable region containing a framework region 1 having the amino acid sequence of SEQ ID NO: 115 or 120, a framework region 2 having the amino acid sequence of SEQ ID NO: 116 or 121, a framework region 3 having the amino acid sequence of SEQ ID NO: 117 or 122, and a framework region 4 having the amino acid sequence of SEQ ID NO: 118 or 123.

Any one or more of the framework regions 1 to 4 above may have one or several amino acid mutations as long as the binding affinity to the antigen is not significantly lowered. In an embodiment, the amino acid mutation is amino acid substitution.

In an embodiment, the humanized antibody has:

(1) a heavy chain variable region having the amino acid sequence set forth in SEQ ID NO: 124, and a light chain variable region having the amino acid sequence set forth in SEQ ID NO: 114.

(2) a heavy chain variable region having the amino acid sequence set forth in SEQ ID NO: 129, and a light chain variable region having the amino acid sequence set forth in SEQ ID NO: 114.

(3) a heavy chain variable region having the amino acid sequence set forth in SEQ ID NO: 124, and a light chain variable region having the amino acid sequence set forth in SEQ ID NO: 119.

(4) a heavy chain variable region having the amino acid sequence set forth in SEQ ID NO: 129, and a light chain variable region having the amino acid sequence set forth in SEQ ID NO: 119.

(5) a heavy chain variable region having the amino acid sequence set forth in SEQ ID NO: 144, and a light chain variable region having the amino acid sequence set forth in SEQ ID NO: 134.

(6) a heavy chain variable region having the amino acid sequence set forth in SEQ ID NO: 149, and a light chain variable region having the amino acid sequence set forth in SEQ ID NO: 134.

(7) a heavy chain variable region having the amino acid sequence set forth in SEQ ID NO: 144, and a light chain variable region having the amino acid sequence set forth in SEQ ID NO: 139; and

(8) a heavy chain variable region having the amino acid sequence set forth in SEQ ID NO: 149, and a light chain variable region having the amino acid sequence set forth in SEQ ID NO: 139.

The antibody is not the specific antibody disclosed in WO2019/147831A (which is incorporated herein by reference in its entirety). More specifically, the antibody does not include any one or more or all of the light chain CDRs contained in the amino acid sequence of SEQ ID NO: 4 or 18 in WO2019/147831A, or any one or more or all of the heavy chain CDRs contained in the amino acid sequence of SEQ ID NO: 2, 17, or 19 in WO2019/147831A. Further, the antibody does not include any or all of the CDRs set forth in SEQ ID NOs: 25 to 27, or any or all of the CDRs set forth in SEQ ID NOs: 29 to 31 in WO2019/147831A. Moreover, the antibody does not include any or all of the CDRs set forth in SEQ ID NOs: 33 to 35, or any or all of the CDRs set forth in SEQ ID NOs: 37 to 39 in WO2019/147831A. The antibody does not include any or all of the CDRs set forth in SEQ ID NOs: 41 to 43, or any or all of the CDRs set forth in SEQ ID NOs: 45 to 47 in WO2019/147831A.

The antibody is not the specific antibody disclosed in WO2021/163076A (which is incorporated herein by reference in its entirety). Specifically, the antibody does not include any one or more or all of the CDRs contained in the heavy chain variable region set forth in SEQ ID NO: 2, or any one or more or all of the CDRs contained in the light chain variable region set forth in SEQ ID NO: 10 in WO2021/163076A. The antibody does not include any one or more or all of the CDRs contained in the heavy chain variable region set forth in SEQ ID NO: 22, or any one or more or all of the CDRs contained in the light chain variable region set forth in SEQ ID NO: 30 in WO2021/163076A. The antibody does not include any one or more or all of the CDRs contained in the heavy chain variable region set forth in SEQ ID NO: 42, or any one or more or all of the CDRs contained in the light chain variable region set forth in SEQ ID NO: 50 in WO2021/163076A.

The antibody is not the specific antibody disclosed in WO2021/211406 (which is incorporated herein by reference in its entirety). The antibody is not the specific antibody disclosed in WO2021/211416 (which is incorporated herein by reference in its entirety). The specific antibody refers to, for example, an antibody whose whole or a part (e.g., all or a part of CDRs) is defined by the amino acid sequence.

In an embodiment, the antibody is monospecific. In an embodiment, the antibody is Mult specific. In an embodiment, the antibody is bispecific.

The antibody can be obtained by a method well known to those skilled in the art. For example, recombinant proteins or cells that express recombinant proteins are used to immunize a TMPRSS2 KO animals, and then, antibodies that bind to TMPRSS2 can be obtained. Monoclonal antibodies can be obtained as culture supernatants of hybridomas obtained by fusing splenocytes of the immunized animals with myeloma. Monoclonal antibodies can also be obtained from the ascites or the like of animals into which hybridomas was transplanted. Also, monoclonal antibodies may be obtained from B cells by gene cloning. Alternatively, the antibodies can be obtained by a well-known method such as phage display. The antibodies can be modified to prepare chimeric antibodies (e.g., human chimeric antibodies) or humanized antibodies as necessary. The antibodies can be produced from animals (e.g., mice) having human antibody loci (e.g., variable regions). The obtained antibodies are tested in terms of whether or not they have properties to bind to TMPRSS2, and whether or not they have properties to inhibit coronavirus infection of cells, and antibodies that have such properties can be selected from the obtained antibodies. The obtained antibodies can also be tested depending on whether or not they further have one or more properties selected from the group consisting of the binding affinity (KD) to TMPRSS2, IC90 referring to infection inhibition, properties to bind to specific amino acids, and properties to compete with reference antibodies for binding to TMPRSS2, and antibodies having further useful properties can be selected from antibody pools.

When useful antibodies are obtained, the antibodies can be industrially produced from antibody-producing cells such as Chinese hamster ovary (CHO) cells, which have genes for encoding antibodies.

The competition can be determined, for example, by binding labeled antibodies to TMPRSS2 expressing cells and incubating them in the presence of unlabeled antibodies at various concentrations and based on the decreased amount of label bound to cells. For example, when an antibody competes with another antibody, the amount of label bound to cells can be decreased to 50% or less, 40% or less, 30% or less, 20% or less, or 10% or less.

KD of the antibodies can be appropriately determined by those skilled in the art. KD can be determined from Kon and Koff by, for example, the surface plasmon resonance (SPR) method. For example, by the following formula:

$$KD = Koff/Kon$$

KD can be determined. In other words, antibodies having small KD are considered to have strong binding affinity, and for example, the antibodies tend to easily bind to their antigen and not easily dissociate therefrom. The surface plasmon resonance (SPR) method can be performed by, for example, commercially available apparatus (e.g., Biacore (trademark)).

IC90 referring to infection inhibition is the concentration of test antibodies that can inhibit infection by 90% in the absence of the test antibodies. It is indicated that the smaller IC90 is, the greater the infection inhibiting effects of the test antibodies. IC90 can be determined by examining the infection inhibiting effects in the presence of the test antibodies having various concentrations. Usually, IC90 can be more precisely determined by conducting experiments using solutions of test antibodies at a lower concentration and a higher concentration than IC90.

The properties to bind to a specific amino acid can be confirmed, for example, depending on whether or not the test antibodies bind to a peptide having a specific amino acid sequence (e.g., peptide having the amino acid sequence of any of SEQ ID NOs: 41 to 93 and 96). This confirmation method can be appropriately conducted using a well-known technique such as ELISA by those skilled in the art.

In an embodiment of the present invention, the antibody of the present invention is an antibody that binds to an extracellular domain of TMPRSS2 and is capable of inhibiting coronavirus infection of cells. In an embodiment of the present invention, the antibody of the present invention is an antibody that binds to an extracellular domain of human TMPRSS2 and is capable of inhibiting coronavirus infection of human cells. The coronavirus may be a beta coronavirus, and in a preferred embodiment, may be a beta coronavirus such as SARS-CoV, MERS, or SARS-CoV-2. In a particularly preferred embodiment, the coronavirus may be the alpha variant, the beta variant, the gamma variant, the delta variant, and the omicron variant of SARS-CoV-2. According to the present invention, since the coronavirus infection is inhibited by inhibiting human proteins, it is theoretically assured to provide an antibody that are effective in inhibiting coronavirus infection, regardless of mutations in S proteins of the coronavirus.

In an embodiment of the present invention, the antibody of the present invention is an antibody that binds to an extracellular domain of human TMPRSS2, whose binding affinity (KD) to the human TMPRSS2 is 10-8 M or less, and that is capable of inhibiting coronavirus infection of human cells. In an embodiment of the present invention, the antibody of the present invention may be an antibody that binds to an extracellular domain of human TMPRSS2, whose IC90 referring to inhibition of the coronavirus infection of human cells is 10 $\mu$g/mL or less. In an embodiment of the present invention, the antibody of the present invention may be an antibody that binds to an extracellular domain of human TMPRSS2, whose binding affinity (KD) to the human TMPRSS2 is 10-8 M or less, and whose IC90 referring to inhibition of the coronavirus infection of human cells is 10 $\mu$g/mL or less. The coronavirus may be a beta coronavirus, and in a preferred embodiment, may be a beta coronavirus such as SARS-CoV, MERS, or SARS-CoV-2. In a particularly preferred embodiment, the coronavirus may be the alpha variant, the beta variant, the gamma variant, the delta variant, and the omicron variant of SARS-CoV-2.

The Centers for Disease Control and Prevention (CDC) designated as variants of concern (VOC) and variants of interest (VOI) of SARS-CoV-2, Alpha (B.1.1.7, Q.1-Q.8), Beta (B.1.351, B.1.351.2, B.1.351.3), Gamma (P.1, P.1.1, P.1.2), Epsilon (B.1.427 and B.1.429), Eta (B.1.525), Iota (B.1.526), Kappa (B.1.617.1), B.1.617.3, Mu (B.1.621, B.1.621.1), Zeta (P.2). In addition to that, the omicron variant (B.1.1.259, BA lineage) was added as a VOC, in November 2021. The antibody of the present invention can be effective against all of these variants.

Preferably, the antibody of the present invention also exhibits the binding properties with one or more extracellular domains of TMPRSS2 in laboratory animals, for example, selected from the group consisting of mice, green monkeys, cats, hamsters and macaque monkeys, from the viewpoint of conducting animal testing before human clinical trials.

<Composition Containing Antibody of The Present Invention>

According to the present invention, a composition containing the antibody of the present invention is provided. According to the present invention, a pharmaceutical composition containing the antibody of the present invention is also provided. The pharmaceutical composition of the present invention can further contain a pharmaceutically acceptable additive in addition to the antibody.

The composition or pharmaceutical composition of the present invention can be administered in a subject for use in inhibiting cell entry of coronavirus in the subject. Therefore, the composition or pharmaceutical composition of the present invention can be used in subjects who are not infected with coronavirus to prevent them from being infected with coronavirus. Also, the composition or pharmaceutical composition of the present invention can be used in subjects infected with coronavirus to prevent coronavirus from being spread from the infected cells. Therefore, the composition or pharmaceutical composition of the present invention can also be used to treat subjects infected with coronavirus. Administering to subjects infected with coronavirus the composition or pharmaceutical composition of the present invention make it possible to prevent the symptoms of the coronavirus infection from deteriorating or becoming severe, or to improve the symptoms in the subjects. Preventing deterioration includes delaying deterioration and inhibiting deterioration. The coronavirus may be a beta coronavirus, may be a coronavirus such as SARS-CoV, MERS, or SARS-CoV-2, and in particular, may be a coronavirus variant designated as VOC or VOI by The World Health Organization (WHO).

A subject to whom the composition or pharmaceutical composition of the present invention is administered is preferably a human. However, the coronavirus infection is known to be zoonotic infection. Also, coronaviruses having high pathogenicity in animals are known such as mouse hepatitis virus, feline infectious peritonitis virus, and porcine epidemic diarrhea virus. Accordingly, the composition or pharmaceutical composition of the present invention can be used to prevent infection of these coronaviruses and treat their symptoms. In this case, the composition or pharmaceutical composition of the present invention needs to contain an antibody capable of binding to TMPRSS2 in the target animal species and inhibiting the infection of cells of the animal species with the target coronavirus.

<Other Applications>

According to the present invention, provided is use of the antibody of the present invention or an antigen-binding fragment thereof, in production of a medicine for inhibiting cell entry of coronavirus in a subject.

According to the present invention, provided is use of the antibody of the present invention or an antigen-binding fragment thereof, for inhibiting cell entry of coronavirus in a subject.

According to the present invention, provided is the composition or pharmaceutical composition of the present invention for use in a method for inhibiting cell entry of coronavirus in a subject.

According to the present invention, provided is use of the antibody of the present invention or an antigen-binding fragment thereof for use in a method for inhibiting cell entry of coronavirus in a subject.

According to the present invention, provided is a method for preventing coronavirus infection in a subject, including administering to the subject an effective amount of the antibody of the present invention or an antigen-binding fragment thereof.

According to the present invention, provided is a method for treating a subject infected with coronavirus, including administering to the subject an effective amount of the antibody of the present invention or an antigen-binding fragment thereof.

According to the present invention, provided is a method for preventing coronavirus infection from deteriorating and/or becoming severe in a subject infected with coronavirus, including administering to the subject an effective amount of the antibody of the present invention or an antigen-binding fragment thereof.

Examples

[Materials and Methods]

Gene Synthesis

All amino acid sequences were obtained from UniProtKB. From the amino acid sequences, DNA that encodes the optimum human codon usage sequence was designed and chemically synthesized to produce a recombinant protein and a transfection cell line (Eurofins Genomics).

Construction of Plasmid Vector

The synthesized DNA was amplified by PCR using a forward primer and a reverse primer that were added with tag sequences at the C-terminal and/or the N-terminal and the 3' and 5' regions of homology required for In-Fusion (trademark) cloning. The sequences of these extensions must match precisely the 15 bp of the 5' end and 3' end of the recipient vector exposed by linearization of the vector at the position into which the PCR product is inserted.

For the retrovirus expression vector pMys-IRES-GFP (CELL BIOLABS, INC.), the synthesized DNA was amplified by PCR using a primer set with a restriction digestion site added in place of the 15 bp addition sequence for In-Fusion (trademark) cloning. Using a DNA fragment after being digested with each restriction enzyme, the retrovirus expression vector was inserted. The correct sequence of the cDNA in the expression vector was confirmed by DNA sequencing.

Serine Protease Domain of Human TMPRSS2 against E. Coli-Derived Immunizing Antigen

DNA fragments that encode the serine protease domain of human TMPRSS2 (containing a His sequence at the C-terminal) were inserted into pE-SUMOstar Amp (LifeSensors, Inc.) and pMAL-p5X Vector (BioLabs) using In-Fusion (trademark) cloning system (Takara Bio Inc.). In order to produce recombinant proteins, plasmid vectors were introduced into E. coli BL21 strain by heat shock, and colonies grown by culturing in a nutrient agar medium containing ampicillin at 37° for 24 hours, were cultured in a nutrient broth medium containing ampicillin for 24 hours, and the cultured bacteria were transferred to an inducing medium to induce the production of the recombinant proteins, and placed in a shaker incubator at 24 rpm. After the number of bacteria reached an appropriate level (absorbance of 0.6), 1 mM IPTG solution was added into bacterial cell suspension. At 2 hours and 4 hours after the addition of the IPTG solution, centrifugation was performed at 4000 rpm for 8 minutes to precipitate bacteria. The bacterial cells were pelleted by centrifugation (6000 g × 15 minutes) and washed with PBS. The pellets were suspended in 10 mL lysis buffer (50 mM potassium phosphate buffer, pH 7.8, 0.5 M NaCl, 5 mM MgCl2, 1 mg/mL lysozyme, 10 μg/mL DNase) and subjected to sonication in a water bath (Branson 200) for 5 minutes, and then the lysate was incubated on a shaker at room temperature for 30 minutes. Ultracentrifugation (150000 × g for 35 minutes) was performed to collect supernatant. Fractions from sucrose gradient were collected using a bent Pasteur pipette.

Production of Recombinant Protein of Human Full-Length TMPRSS2 against Immunizing Antigen from Insect Cells

Full-length cDNA of human TMPRSS2 having a His sequence at the C-terminal was inserted into a pFastBac1 vector using In-Fusion (trademark) cloning system (Takara Bio Inc.).

Using Bac-to-Bac Baculovirus Expression System (Thermo Fisher Scientific, Inc.), recombinant proteins were expressed in HighFive insect cells (Thermo Fisher Scientific, Inc.), and the cultured cells were homogenized and disrupted. Membrane fractions were separated and solubilized with 1% dodecyl maltoside (DDM, Anatrace, Maumee, OH).

Harvest of Recombinant Proteins for ELISA and BIACore Assays from Mammalian Cells.

A pcDNA3.4 expression vector was purchased from Thermo Fisher, Inc. PCR fragments that encode Strep-tag II (WSHPQFEK) at the C-terminal and His tag at the N-terminal of the extracellular region of the human TMPRSS family were inserted into an expression vector.

Expi293F cells were cultured in a humidified 8% $CO_2$ incubator at 37°C, 120 rpm, using Expi293 Expression Medium (Thermo Fisher, Inc., Cat. A1435101). On the day of transfection, the density of Expi293 cells was approximately $4.0 \times 10^6$ cells/mL. The cells were centrifuged at $200 \times g$ for 15 minutes at room temperature and the medium was decanted, and then the cells were resuspended in 100% fresh medium to a density of $2.5 \times 10^6$ cells/mL. Expi293 transfection was performed according to the manufacturer's protocol except for 100% replacement of the medium before the transfection described above. For all Expi293 transfections, Expi293 Expression System Kit (Thermo Fisher, Inc., Cat. A14635) containing a transfection enhancer and ExpiFectamine 293 transfection reagent was used. The ExpiFectamine 293 transfection reagent and plasmid DNA that were separately diluted with a complex medium, OptiMem (Thermo Fisher, Inc., Cat. 31985062) were used. After 5-minute incubation, the ExpiFectamine 293 and the DNA mixture were combined and incubated for further 20 minutes. The mixture of ExpiFectamine 293-DNA-OptiMEM was then added into the cells. An enhancer 1 and an enhancer 2 were added to the transfected culture 16 to 18 hours after transfection. The recombinant proteins were purified with Ni-NTA affinity chromatography.

Purification of Recombinant Proteins

The recombinant proteins were purified with Ni-NTA affinity chromatography. Resin was added thereto to bind to the supernatant at 4°C. The next day, the mixture was moved to a column, and the pass-through fractions were removed, and then the resultant was washed with Tris buffer (pH 7.0) containing 0.05% DDM, 0.002% Cholesteryl Hemisuccinate (CHS Anatrace Cat no CH210) and 5 mM DTT. After that, using wash buffer added with 2.5 mM desthiobiotin, recombinant proteins were eluted from the resin.

Retroviral Vector and Transfection Cell Line

Into the retrovirus expression vector pMys-IRES-GFP, the full-length TMPRSS family gene having an HA-tagged sequence (amino acid sequence: YPYDVPDYA) at the C-terminal was inserted.

Daudi lymphoma cell line was obtained from JCRB Cell Bank (JCRB9071). A20 and EL4 lymphoma cell lines were obtained from RIKEN-BRC Cell Bank. These cell lines were introduced using a retroviral vector as follows. A retrovirus expression vector and an envelope expression vector, p10A1 or pAmpho, were co-introduced into GP2-293 cells using a Retro-X Universal Packaging system (Takara Bio Inc., #631530). 2 to 3 days after transfection, the culture supernatant was filtered with a 0.45 μm filter, and then infected transfected with the above cell lines using 10 μg/ml polybrene (Sigma Aldrich Co. LLC, #P8155). After 1 week from the introduction, the cells expressed the introduced molecules on the surface. Finally, the cells were sorted with a purity of 98% or more using FACS Aria III (BD Biosciences).

Immunization

TMPRSS2 KO mice were provided by Dr. Takeda (The National Institute of Infectious Diseases), and subcutaneously primed with TMPRSS2-serine protease domain recombinant protein (50 μg/mouse) using adjuvant TiterMax Gold (TiterMax USA, Inc.), mice were alternately immunized in the abdominal cavity with TMPRSS2 transfectants ($2 \times 10^7$ cells/mouse) and recombinant proteins (50 μg/mouse) at 2-week intervals. The TMPRSS2 transfectant was treated with gamma radiation (10 Gy) before immunization. ODN 1826 Vaccine Grade (Nacalai tesque, Inc., Japan) was used for immunization of the TMPRSS2 transfectant cells. These mice were boost-immunized with full-length TMPRSS2

recombinant protein (10 μg/mouse) by tail vein injection 3 days before the fusion. Spleen B-cells were purified from the immunized mice and used for hybridoma preparation.

Enrichment of IgG+ B Cells from Immunized Mice

Mature B cells were separated by negative selection using the MASC system (Miltenyi Biotec, Bergisch Gladbach, Germany). Unwanted mononuclear cells (T cells, NK cells, monocytes, dendritic cells, granulocytes, and immature B cells prior to B cell receptor class switching) were targeted via a biotin-labeled antibody cocktail specific to the cell surface receptors of these cell types. Also, mature B cells were specifically enriched from the sample without retention in the LS column in the presence of a magnetic field.

Cell Fusion and Hybridoma Selection

Mature B cells enriched from the spleens of immunized mice were mixed with P3U1 myeloma cells at a ratio of 1:1, and the mixture was subjected to electrofusion using a Nepagene ECFG21 electroporator (Nepa Gene Co., Ltd., Ichikawa, Chiba, Japan) according to the manufacturer's instruction. The cells were then seeded at $2 \times 10^3$ cells /well in 96-well flat-bottom plates and selected in a culture solution containing a HAT supplement (Sigma Aldrich Co. LLC) and a BM Condimed H1 supplement (F. Hoffmann-La Roche Ltd.). When the cells covered half of the bottom of the wells, the culture supernatant was examined by ELISA for reactivity with TMPRSS2 recombinant protein. A portion of the hybridoma cells obtained from positive culture supernatants was frozen in CELLBANKER 1 plus (Takara Bio Inc.). The remaining cells were passage cultured to confluent, and then each supernatant was used for flow cytometry screening and inhibition screening for cell fusion analysis.

ELISA Screening

To a Nunc MaxiSorp flat-bottom 96-well microplate, 50 mM carbonate coating buffer containing 0.2 μg/mL recombinant protein of human TMPRSS2, pH 9.6 (100 μL) was applied and incubate at 4°C overnight. The plate was then washed with PBST wash buffer (0.05% Tween 20/PBS) three times, 1% BSA blocking buffer was added thereto at 200 μL/well to incubate for 2 hours at room temperature. After washing, 100 μL hybridoma supernatant was added thereto, and the mixture was incubated for 2 hours followed by washing. 100 μL HRP-labeled anti-mouse IgG antibody (MBL#330) was added to each well, and then the microplate was incubated for 1 hour. After washing the plate, 100 μL/well of TMB substrate solution (SeraCare Life Sciences, Inc. 5120-0077) was added thereto, and reacted in the dark for 15 minutes. The reaction was stopped by adding 2 M sulfuric acid (50 μL/well), and then the absorbance was measured at OD450 using a microplate reader (ARVO ×3, PerkinElmer, Inc.).

Screening by Flow Cytometry

For screening of the ELISA positive supernatant by flow cytometry, the Daudi cell line expressing TMPRSS2 and GFP and the parent cells of Daudi were mixed at a ratio of 1:1. The cells were then stained with 100 μL hybridoma supernatant in a 96-well plate, stored on ice for 20 minutes, and then incubated with allophycocyanin (APC)-labeled anti-mouse IgG (BioLegend, Inc., Poly4053). The cells were washed, resuspended in propidium iodide (Nacalai tesque, Inc., Japan), and the staining of TMPRSS2 was analyzed using LSR Fortessa X-20 (BD Biosciences, Franklin Lages, NJ). Hybridomas whose supernatant reacted with TMPRSS2+Daudi transfectant and did not react with the parent cell line were selected.

Screening of Dual Split Protein (DSP) Reporter Assay to Monitor Membrane Fusion (Cell Fusion Assay)

In order to establish a stable 293FT cell line that expresses spike proteins (S proteins) with DSP8-11, and expresses ACE2 and TMPRSS2 with DSP1-7, 293T cells introduced with VSV-G expressing plasmid were used to produce recombinant pseudo-typed retroviruses that express any of these proteins. Reporter cells derived from 293FT infected with pseudo-typed virus were selected with 1 μg/mL puromycin, 10 μg/mL blastidine, and 300 μg/mL hygromycin for at least 1 week. Fusion assay was performed using these batch-selected cells.

In DSP assays using 293FT cells, effector cells that express S proteins using DSP8-11, and target cells that express ACE2 and TMPRSS2 using DSP1-7 were seeded on a 12-well cell culture plate 1 day before the assay ($2 \times 10^5$ cells/500 μL). 2 Hours before the DSP assay, the cells were treated with a Renilla luciferase substrate, 6 μM EnduRen (Promega, Madison, WI, USA) to activate EnduRen. 50 μL Target cells that express ACE2 and TMPRSS2 were seeded on a 384-well plate using a multidrop dispenser (Thermo Scientific, Waltham, MA, USA), and the hybridoma supernatant was added to the wells. After incubation at 37°C for 1 hour, suspension of the effector cells expressing S proteins was added to the wells using a multidrop dispenser. After incubation at 37°C for 4 hours, the RL activity was measured using a Centro xS960 luminometer (Berthold Technologies GmbH, Germany). The inhibition rate calculated as relative light units (RLU) of the luciferase activity compared to control was reported as neutralizing antibody sensitivity of the hybridoma supernatant in the DSP reporter assay.

First Cloning of Hybridomas

Hybridomas of interest among 3,723 candidates were collected from a frozen cell stock, cultured in a HT medium overnight, and then cloned by limiting dilution (0.3 cells/well). Clones generally appeared in 8 to 15 days, and 120 clones obtained by limiting dilution were screened again for antibody reactivity, using flow cytometry analysis and inhibitory functions for DSP assays with 293FT effector cells that express S proteins and 293FT target cells that express ACE2 and TMPRSS2 or Calu-3 target cells introduced with DSP1-7.

Further, mouse IgG concentrations of the 120 hybridoma supernatants were analyzed by ELISA, and mAb concentrations (IC50) that reduces RLU by 50% compared to control were reported as neutralizing antibody titers for the DSP reporter assay with the 293FT target cells and 293 effector cells. Titers were calculated using a non-linear regression curve fit (GraphPad Prism Software Inc., La Jolla, CA).

Second Cloning of Hybridomas

20 Clones of interest from the independent hybridomas selected by the above functional screening targeting 120 clones were collected from the frozen cell stock, cultured in HT and BM-Condimed H1 media overnight, and then cloned again by limiting dilution (0.3 cells/well) to confirm the reactivity to TMPRSS2 by FACS analysis.

In order to adapt to a passage medium, concentrations of FCS, HT, BM-Condimed H1 were stepwisely reduced in the HT medium, cell growth was repeatedly confirmed, and eventually the cells were adapted to culture in 10% FCS RPMI-1640 medium without adding HT and BM-Condimed H1.

Identification of mAb Isotype

After subcloning twice, the isotype of mouse mAb was identified with Rapid Mouse Monoclonal Antibody Isotyping Kit according to the manufacturer's instruction (Antigen, ISO-M8ac).

Adaptation of Cells to Serum-Free Medium and Production of mAb

Hybridomas were passaged in 10% FCS RPMI-1640 medium and subjected to 3 passages in each of hybridoma-SFM media (manufactured by Thermo Fisher Scientific, Inc.) containing 6%, 4%, 2%, or 1% fetal bovine serum (FBS) followed by serial culture in a serum-free medium. Samples of $5 \times 10^7$ cells were gathered and transferred to CELLine flask culture (BD Biosciences) using the serum-free medium according to the manufacturer's instruction. After culturing at 37°C with 5% CO2 for 14 days, the cells and media were collected from a cell culture chamber.

Purification of mAbs from Hybridoma Supernatant Fluid

Monoclonal antibodies in culture supernatant collected in the serum-free medium or 10% Ultra Low IgG FBS medium (Thermo Fisher Science, Inc.) were purified with Protein-A Sepharose 4 Fast Flow according to the manufacturer's instruction. The monoclonal antibodies were purified with (GE healthcare), aseptically filtered, and then stored at 4°C.

Production of SARS-CoV-2 Pseudo virus and Neutralization Assay of Anti-TMPRSS2 mAs

In order to establish stable cell lines that express SARS-CoV-2 pseudo-typed S proteins, lentiviruses were prepared using HEK293T cells containing lentivirus transfer plasmids, psPAX2 packaging plasmids, and vesicular stomatitis virus (VSV)-G expression plasmids.

Neutralization assay was performed with Calu-3 cell lines. Pseudo viruses having a luciferase activity titer of about 106 RLU/ml were incubated with an antibody at 37°C for 1 hour. The mixture of pseudo viruses and the antibody (100 μl) was then seeded on a 96-well plate in which $3.0 \times 10^4$ cells/well have been seeded 1 day before infection. The infectivity of the pseudo viruses was scored in terms of the luciferase activity after 48 hours.

Isolation of SARS-CoV-2

VeroE6 (ATCC CRL-1586) cells were maintained in Eagle's minimal essential media (MEM) containing 10% FBS. Respiratory swabs were taken from a patient who tested positive for COVID-19. SARS-CoV-2 virus was proliferated in VeroE6 cells at 37°C using Opti-MEM (Invitrogen) containing 0.3% bovine serum albumin (BSA) and 1 μg L-1-tosylamide-2-phenylethyl chloromethyl ketone (TPCK)-trypsin/ml. All experiments using SARS-CoV-2 virus were conducted in an enhanced biosafety level 3 (BSL3).

SARS-CoV-2 Infection Assay

Calu-3 cells were cultured in an environment of 37°C with 5% $CO_2$ in MEM containing 10% FBS. In vitro infection experiments using Calu-3 cells as target cells were conducted in 2 groups of "pretreatments" and "without pretreatment". In the "pretreatment" group, cells were pretreated with nafamostat mesylate (10-fold serial dilutions from 100 μM to 1 nM, 4 wells per dilution) or anti-TMPRSS2 mAb 1 hour before infection. SARS-CoV-2 was then added thereto at a multiplicity of infection (MOI) of 0.1 in the absence of TPCK-trypsin, and the cells were further incubated for 24 hours to facilitate virus entry.

All cell samples were thoroughly washed with PBS, and then used for RNA extraction and RT-PCR according to SuprePrep II Cell Lysis & RT Kit for qPCR (TOYOBO Co., Ltd.). In a real-time RT-PCR multiplex used for SARS-CoV-2 detection, the expression levels of SARS-CoV-2 N and rpl13a genes were quantified, and the expression level of SARS-CoV-2 N was corrected by the expression level or rpl13a.

Plots of SARS-CoV-2 N and rpl13a were each evaluated in the green channel (excitation wavelength of 470 nm, detection wavelength of 510 nm, fluorescent FAM-labeled probe) and the orange channel (excitation wavelength of 585 nm, detection wavelength of 610 nm, fluorescent ROX-labeled probe). The results were analyzed taking into consideration of cycle threshold (Ct) values. The mAb concentration that was reduced by 90% (IC90) compared to control was reported as the neutralizing antibody titer. Titers were calculated using a non-linear regression curve fit (GraphPad Prism Software Inc., La Jolla, CA).

Competitive Inhibition between Anti-TMPRSS2 mAbs

For these studies, analysis using a flow cytometer was employed. Briefly, the staining concentrations of APC-labeled mAbs were determined by the concentrations showing a value of 80% of the maximum fluorescence intensity against Daudi-TMPRSS2 transfectant cell line. Competitors of unlabeled mAb were diluted 3-fold at concentrations ranging from 0.1- to 10-fold of APC-labeled mAb and stained on ice for 30 minutes. Thereafter, a labeled mAb for detection was added to the cell suspension and further stained for 30 minutes. The cells were washed and resuspended in propidium iodide for analysis.

ELISA Binding Assay for Cross-Reactivity of Anti-TMPRSS2 mAb to Extracellular Regions of Human TMPRSS Family Proteins

The reactivity of anti-TMPRSS2 mAbs to extracellular regions of human TMPRSS family proteins was analyzed by ELISA method. To a streptavidin-coated 96-well plate (Thermo Scientific, 436014), 100 μl each of 78 nM Strep-tagged recombinant proteins in PBS was captured at room temperature for 2 hours and washed 5 times with PBST (0.1% Tween 20/PBS). Anti-TMPRSS2 mAbs (10 μg/mL), mouse IgG (Equitech-Bio, SLM66) or Ni-NTA-HRP (KPL, 24-01-01) as controls, diluted to 0.2% BSA in PBST was added thereto to incubate for 2 hours. After washing with PBST, the bound IgG was detected with 100 μl horseradish peroxidase Goat anti-mouse IgG antibody (BioLegend, Inc.) diluted to 1:15,000 in PBST at room temperature for 1 hour and washed with PBST. 100 μl TMB substrate solution (SeraCare Life Sciences, Inc. 5120-0077) was added to the well and allowed to color at room temperature for a few minutes. The reaction was stopped with 100 μl 2M $H_2SO_4$. The optical density (OD) was read at 450 nm while referring to OD with a microplate reader (ARVO ×3, PerkinElmer, Inc.).

BIACore Binding Assay

BIAcore surface plasmon resonance system (BIAcore., Inc., Piscataway, NJ) was used at 25°C for kinetic binding analysis of extracellular regions of anti-TMPRSS2 mAbs and human TMPRSS2. Sensor chips to which rabbit anti (mouse IgG) was covalently attached by amino coupling were prepared according to the manufacturer's instruction (BIAcore AB, Stevenage, Hertfordshire, UK).

Human TMPRSS2 recombinant proteins at various concentrations were injected in PBS containing 0.05% Tween 20 at a flow rate of 35 μl/min for binding assay. The change in refractive index at the time of binding was used for kinetic measurement. The affinity of TMPTSS2 binding to mAb was calculated from on and off rates. Samples whose concentrations were raised to obtain stable measurements were injected therein, and the net increase in response units (RU) with background correction was recorded. Data analysis was performed by a method such as the Scatchard method, where RU is plotted relative to injected VEGF165 concentration, or values of RU divided by concentration (bound/total) are plotted relative to RU (bound) (the affinity as Kd is obtained from - (1/slope)).

FACS Analysis for Cross-Reactivity of Anti-TMPRSS2 mAbs to Human TMPRSS Family Proteins and TMPRSS2 Derived from Various Animal Species

Daudi-TMPRSS family transfected cell line was stained on ice with anti-TMPRSS2 mAbs (1 μg/mL) for 20 minutes, and then incubated with allophycocyanin (APC)-labeled anti-mouse IgG (BioLegend, Inc., Poly4053). The cells were washed, resuspended in propidium iodide (Nacalai tesque, Inc., Japan), and then the staining of TMPRSS2 was analyzed using LSR Fortessa X-20 (BD Biosciences, Franklin Lages, NJ).

Epitope Mapping by Peptide Array

Epitopes of mAbs were defined with synthesized peptides purified with a microarray (JPT Peptide Technologies GmbH, Berlin, Germany). A 20-meric peptide library that covers the human TMPRSS2 extracellular region was synthesized, immobilized on a glass slide of a microarray, and then detected with anti-TMPRSS2 mAbs for the sequential antibody profiling experiments (Table 2). The profiling experiments were conducted at JPT Peptide Technologies GmbH (Berlin, Germany).

Mutation Introduction of Human TMPRSS2 and Transfectant Thereof

For mutagenesis, the inserted human TMPRSS2 cDNA was ligated into the expression vector pIRES2-ZsGreen using its unique XhoI and EcoRI sites (Takara 632478). Mutant human TMPRSS2 cDNA was obtained by KOD-Plus-Mutagenesis Kit (TOYOBO Co., Ltd., #SMK-101) using primers in Table 3. All constructs were confirmed by DNA sequencing. Transfection was performed using COS7 cells. Expression of wild-type and mutant TMPRSS2 was detected by staining with indicated monoclonal antibodies.

Analysis of mAb Sequences

The cDNAs that encodes variable heavy (VH) and light (VL) genes of mouse mAbs were obtained by PCR using single-stranded cDNA libraries from each of the hybridoma cell lines. Total RNA was extracted from the hybridoma cells using TRIzol Reagent (Invitrogen, 15596026) according to the manufacturer's instruction. SuperScript (trademark) III First-Strand Synthesis System (Thermo Fisher Science, Inc., 18080-051) was used. The amplification of VH-CH1 and VL-CL genes was performed using a mixture of 12 or 10 forward primers designed for complementing each N-terminal sequence of FVH- or FVL-encoding region, and a reverse primer designed for complementing each C-terminal sequence of CH1 or CL region. The PCR products were cloned into pCR2.1-TOPO vector and the sequencing analysis was conducted.

Preparation of Recombinant Human-Mouse Chimeric IgG4

Gene sequences that encode VH and VL were fused to gene that each encode human IgG4 heavy chain and human κ light chain constant regions. The above genes that encode chimeric antibody chains were cloned into a pcDNA3.4 vector downstream of a leader sequence of the immunoglobulin kappa light chain. Before transfection into expi293F cells, all the expression vectors were verified by DNA sequencing. Strategies similar to those used to prepare recombinant antigens were used for obtaining chimeric antibodies as well.

To avoid Fab arm replacement, mouse/human chimeric antibodies were prepared in which the heavy chain constant region was replaced with the human IgG4 S108P variant (Silva et al., J Biol Chem, 2015, vol. 290: 5462-5469).

Antibody-Dependent Cell Cytotoxicity Assay (ADCC)

For these studies, calcein release analysis was used. Daudi that expresses TMPRSS2 was used for target cells, and KHYG-1 that expresses Fc receptor was used for effector cells. Target cells were labeled using Calcein-AM (1 μg/ml, Nacalai tesque, Inc., Japan) at 37°C for 1 hour while occasionally agitating, washed, and then plated on a 96-well U bottom plate at a density of $1 \times 10^4$ cells/well. The indicated mAbs were added at various concentrations, and the effector cells were added at an effector:target (E:T) ratio of 10:1. After incubation at 37°C with 5% CO2 for 3 hours, each supernatant was collected and analyzed for the fluorescence with an excitation filter using ARVO $\times 3$ (PerkinElmer, Inc.). 485 $\pm$ 9 nm; band pass filter. Analysis was performed using 530 $\pm$ 9 nm). Spontaneous release was determined by incubating the target cells in the medium alone, and the maximum release was obtained by suspending the cells in 1% Triton X-100. The ADCC rate of each sample (triplicate) was calculated using the following formula.

% Lysis = (experimental release - spontaneous release) / (maximum release - spontaneous release) $\times$ 100.

[Results]

TMPRSS2 is a human protein expressed in human airway cells. It is required for TMPRSS2 that spike (S) proteins of viruses are cleaved by TMPRSS2 such that coronaviruses such as betacoronaviruses (e.g., SARS-CoV-2) infect human airway cells (Tomita et al., Journal of Virology, 95(12), 2021, doi/10.1128/JVI.00434-21).

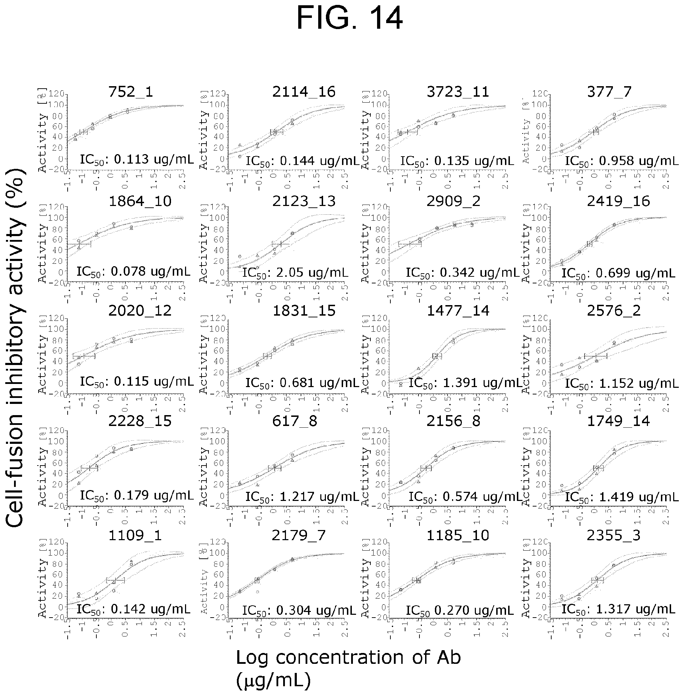

In a dual split protein (dsp) reporter assay to monitor membrane fusion (cell fusion assay), ACE2 expressing cells and S protein expressing cells are fused by the interaction between ACE2 and S protein. DSP1-7 is expressed in ACE2 expressing cells and DSP9-11 is expressed in S protein expressing cells; when the two are fused, DSP1-7 and DSP8-11 are associated within cells to become GFP to emit fluorescence (Yamamoto et al, Viruses, 2020). The fact that the above cell fusion is inhibited in the presence of the obtained monoclonal antibodies indicates the ability of the antibody to be capable of inhibiting virus entry to ACE2 expressing cells of the virus. In this example, it was tested whether or not the above cell fusion was inhibited in the presence of a monoclonal antibody. The concentrations of the monoclonal antibodies were set to 0.08 $\mu$g/mL, 0.31 $\mu$g/mL, 1.25 $\mu$g/mL, or 5 $\mu$g/mL. The infection inhibiting effects of the antibodies were evaluated by showing the cell fusion inhibitory activity (%) as the vertical axis, and the antibody concentration as the transverse axis. The results were as shown in Figure 14. As shown in Figure 14, all 20 antibodies were able to inhibit the cell fusion between the ACE2 expressing cells and the S protein expressing cells in a concentration-dependent manner.

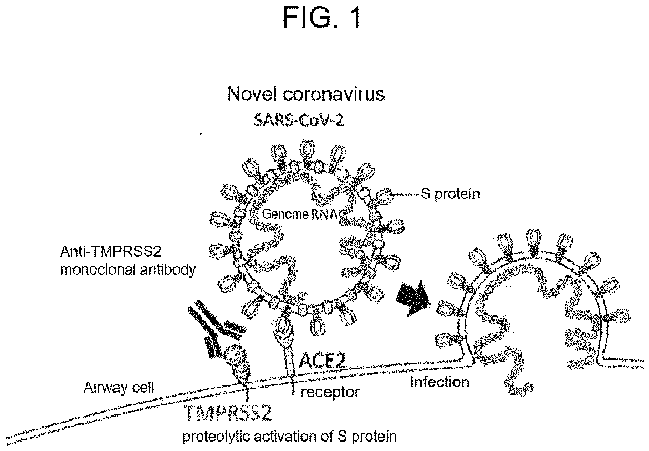

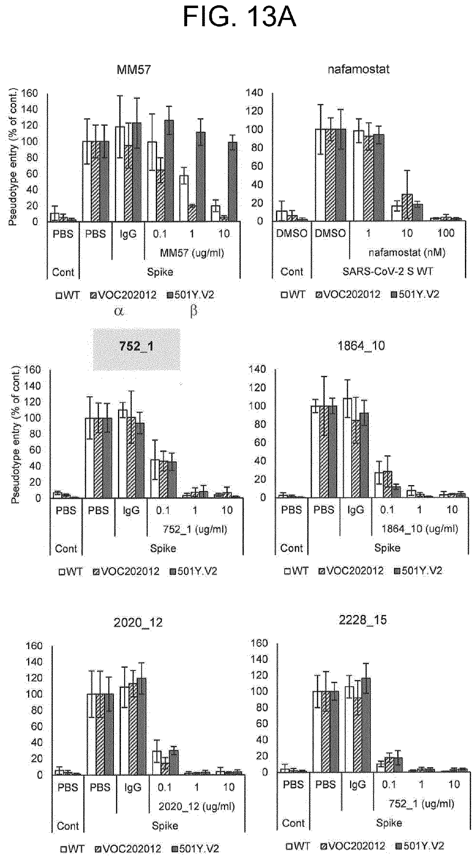

Next, in this example, a group of antibodies that bind to human TMPRSS2 were produced, and those having the protection effect against coronavirus infection were obtained (see Figure 1). The advantage of this strategy is that, as demonstrated in this example, effective antibodies can be obtained regardless of virus mutation, by targeting proteins on the side of hosts such as humans, rather than proteins on the side of viruses where mutation occurs (see Figures 13A to D).

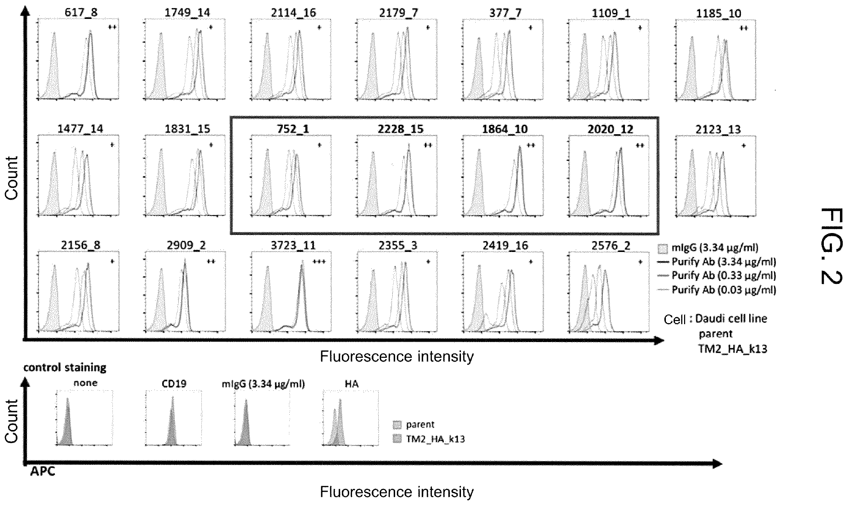

Monoclonal antibodies that bind to human TMPRSS2 were obtained, and the binding properties with TMPRSS2 expressing cells were confirmed under various concentration conditions. Some of the results were as shown in Figure 2 1. As shown in Figure 2 1, it bound to TMPRSS2 expressing cells. Also, the results that the binding affinity of various antibodies to TMPRSS2 was measured by BIACore were as shown in Table 1.

**[Table 1]**

Table 1: Binding affinity of anti-human TMPRSS2 antibody to TMPRSS2

| Antibody ID | ka (1/Ms) | kd (1/s) | KD (M) |
|---|---|---|---|
| 752-1 | $3.98 \times 10^5$ | 0.003836 | $9.63 \times 10^{-9}$ |
| 1109-1 | $1.54 \times 10^5$ | 0.00178 | $1.16 \times 10^{-8}$ |
| 1831-15 | $5.63 \times 10^5$ | $2.36 \times 10^{-4}$ | $4.18 \times 10^{-10}$ |
| 1864-10 | $5.63 \times 10^5$ | 0.002894 | $5.24 \times 10^{-9}$ |
| 2020-12 | $6.54 \times 10^5$ | 0.001759 | $2.69 \times 10^{-9}$ |
| 2114-16 | $5.70 \times 10^5$ | $5.91 \times 10^{-4}$ | $1.04 \times 10^{-9}$ |
| 2123-13 | $1.22 \times 10^5$ | 0.001143 | $9.40 \times 10^{-9}$ |
| 2228-15 | $2.61 \times 10^5$ | 0.00219 | $8.41 \times 10^{-9}$ |

As shown in Figure 2, all of the obtained monoclonal antibodies were antibodies having excellent binding affinity to TMPRSS2. Therefore, 752_1 antibody, 2228_15 antibody, 1864_10 antibody, and 2020_12 antibody were selected from the obtained monoclonal antibodies and subjected to the following analysis (see Figure 2).

Competition Experiment

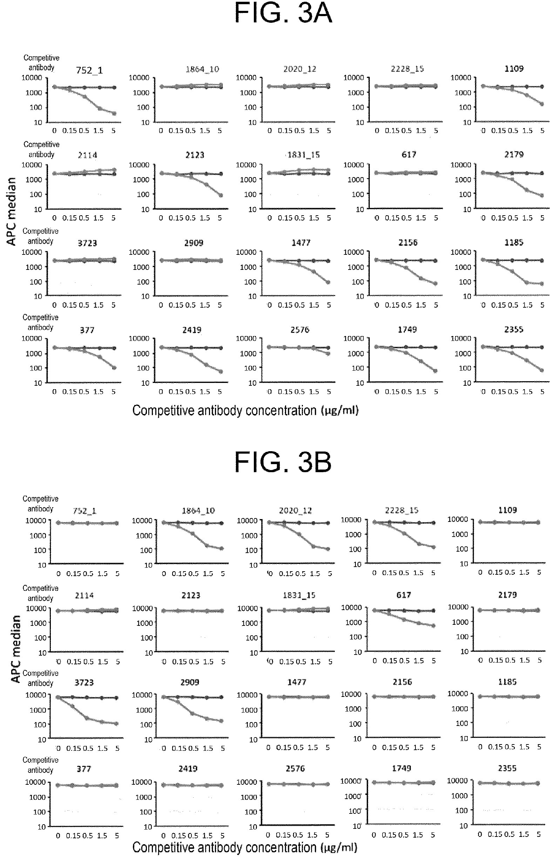

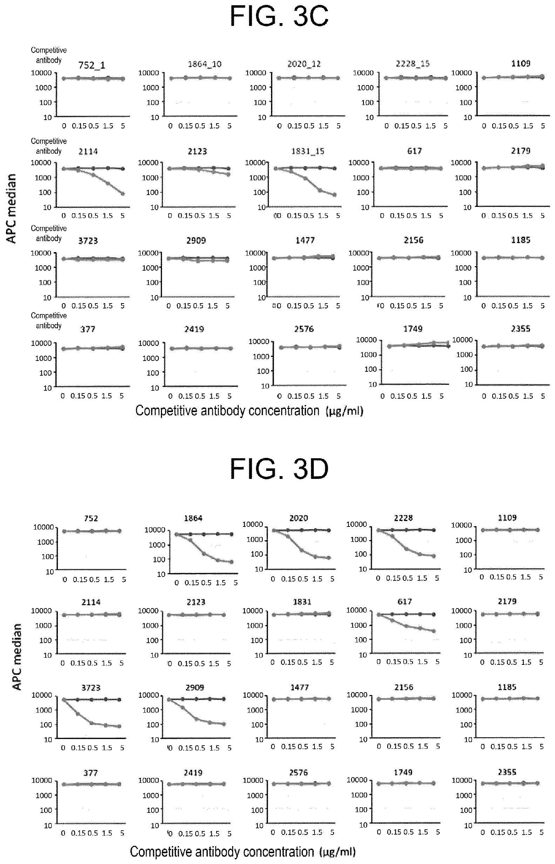

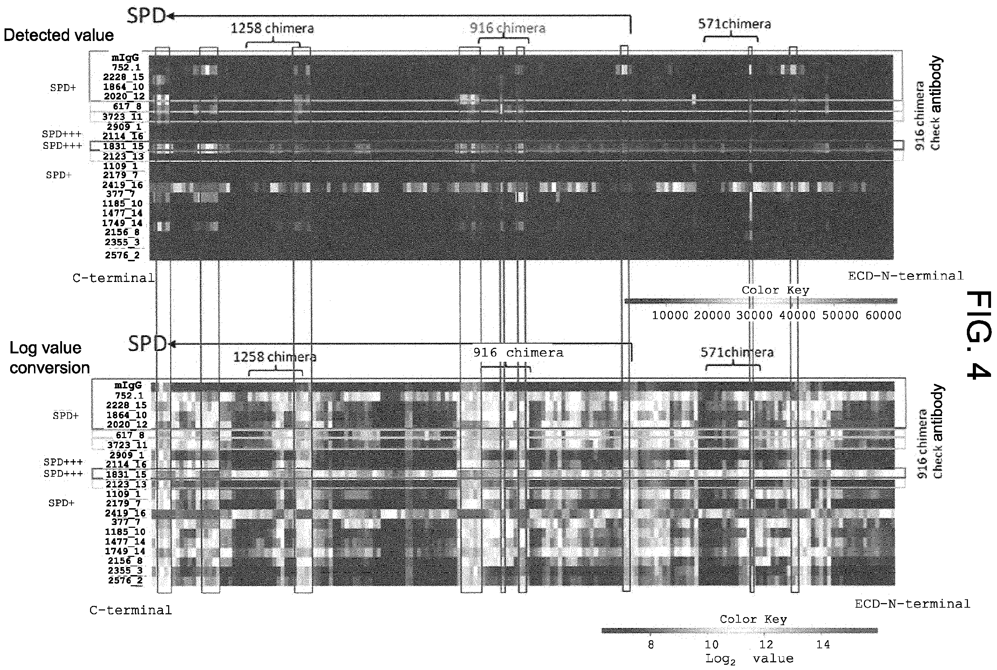

A test was performed to determine whether or not the 20 monoclonal antibodies having infection inhibiting effects obtained for the binding to human TMPRSS2 competed with each other. The results were as shown in Figures 3A to 3C. Figures 3A, 4B, 4C, and 4D each show the results of whether or not the antibodies compete with 752_1 antibody (Figure 3A), 2228_15 antibody (Figure 3B), 1831_15 antibody (Figure 3C), 1864_10 antibody(Figure 3D) for binding to human TMPRSS2. As shown in Figure 3A, 752_1 antibody competed with 1109 antibody, 2123 antibody, 2179 antibody, 1477 antibody, 2156 antibody, 1185 antibody, 377 antibody, 2419 antibody, 2576 antibody, 1749 antibody, and 2355 antibody. Also, as shown in Figure 3B, 2228_15 antibody competed with 2020_12 antibody, 1864_10 antibody, 617 antibody, 3723 antibody, and 2909 antibody. Further, as shown in Figure 3C, 1831_15 antibody competed with 2123 antibody, and 2114 antibody. 1831_15 antibody weakly competed with 2114 antibody. 2576 antibody weakly competes with only 752_1 antibody. From these results, the obtained antibodies having infection inhibiting effects were classified into 3 competitive groups. In Table 2 below, the antibodies included in the same competitive groups were classified into bin 1 to bin 3. Note that the results shown in Figure 3D closely resembled the results shown in Figure 3B. 2228_15 antibody and 1864_10 antibody are thought to probably bind to nearly the same location.

**[Table 2]**

| Table 2: Results of epitope binning | | |
|---|---|---|
| Bin 1 | Bin 2 | Bin 3 |
| 7 5 2_1 | 1 8 6 4_1 0 | 2 1 1 4 |
| 1 1 0 9 | 2 0 2 0_1 2 | 1 8 3 1_1 5 |
| 2 1 2 3 | 2 2 2 8_1 5 | 2 1 2 3 |
| 2 1 7 9 | 6 1 7 | |
| 1 4 7 7 | 3 7 2 3 | |
| 2 1 5 6 | 2 9 0 9 | |
| 1 1 8 5 | | |
| 3 7 7 | | |
| 2 4 1 9 | | |
| 2 5 7 6 | | |
| 1 7 4 9 | | |
| 2 3 5 5 | | |

Competitive antibodies are likely to bind to the same or overlapping epitopes. The groups of antibodies that can strongly inhibit SARS-CoV-2 infection can be classified into the 3 competitive groups, suggesting that the epitopes to which these antibodies bind have significant technical meanings in relation to the infection inhibition. 2576 antibody is an antibody that can strongly inhibit SARS-CoV-2 infection but does not strongly compete with either 1864_10 or 2114, and it weakly competed with 752_1. This result indicates that although 2576 antibody has a small epitope overlap with 752_1 antibody, it is still enough to inhibit SARS-CoV-2 infection. Also, 2123 antibody strongly competed with 752_1.

Epitope Mapping

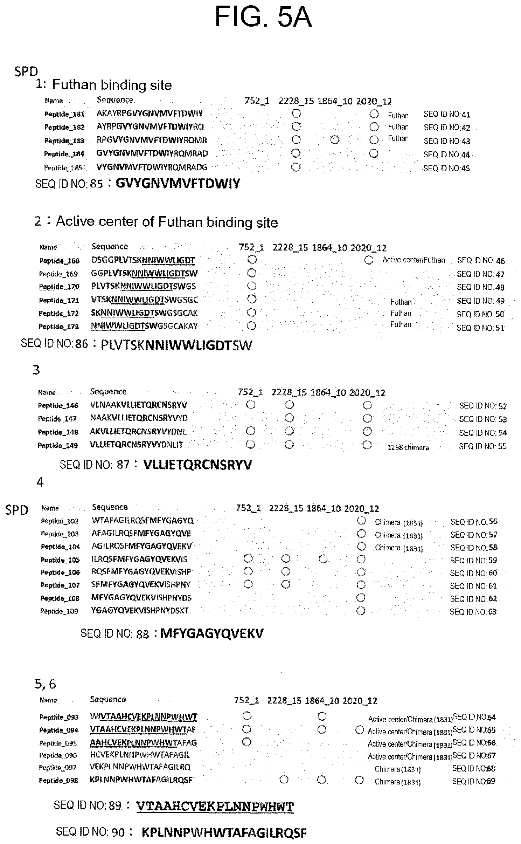

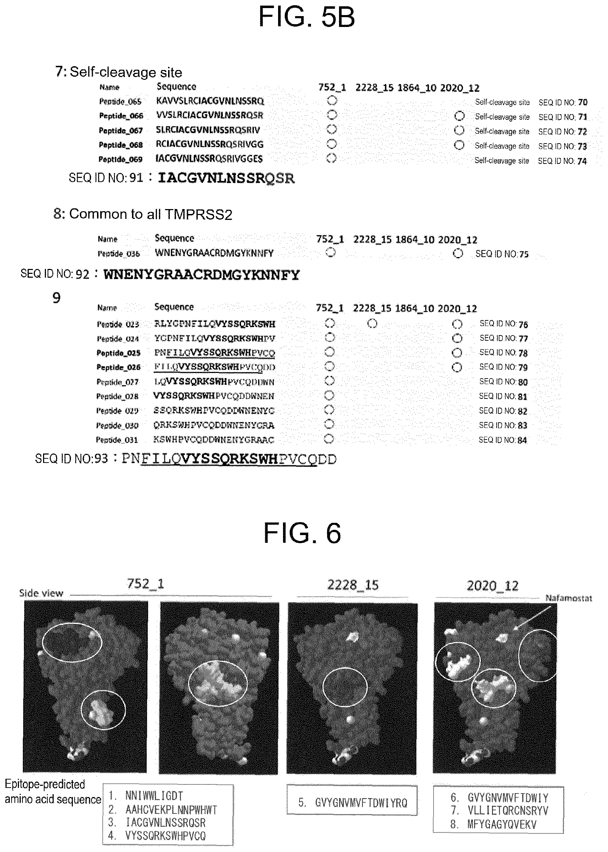

Peptides with a length of 20 amino acids were prepared by shifting 2 amino acids each to the extracellular region (387 amino acids) of TMPRSS2 as an antigen. Total number of obtained peptides were 185. The 20 monoclonal antibodies were each allowed to react with them, information was obtained to which peptides they showed binding properties, and based on the information, epitope mapping was performed. The results were as shown in Figure 4.
Epitope candidates for 4 monoclonal antibodies (752_1 antibody, 2228_15 antibody, 1864_10 antibody, and 2020_12 antibody) were as shown in Figures 5A to 5B. 2228_15 antibody, 1864_10 antibody, and 2020_12 antibody particularly bound to the amino acid sequence set forth in SEQ ID NO: 85 (GVYGNVMVFTDWIY). The circled regions are epitopes in Figure 6. In the leftmost panel in Figure 6, peptide 3 is shown in the lower circle, and peptide 4 is shown in the upper circle. In the second panel from the left in Figure 6, peptides 1 and 2 are shown in the circle. In the third panel from the left in Figure 6, peptide 5 is shown in the circle. In the fourth panel from the left in Figure 6, peptide 6 is shown in the center circle, peptide 7 is shown in the right circle, and peptide 8 is shown in the left circle.

Binding Properties of TMPRSS2 to Amino Acid Substitution Variants

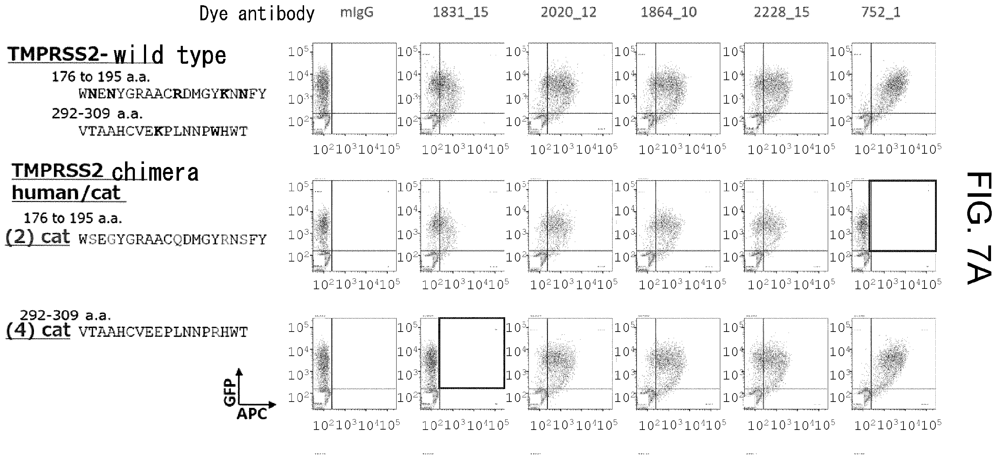

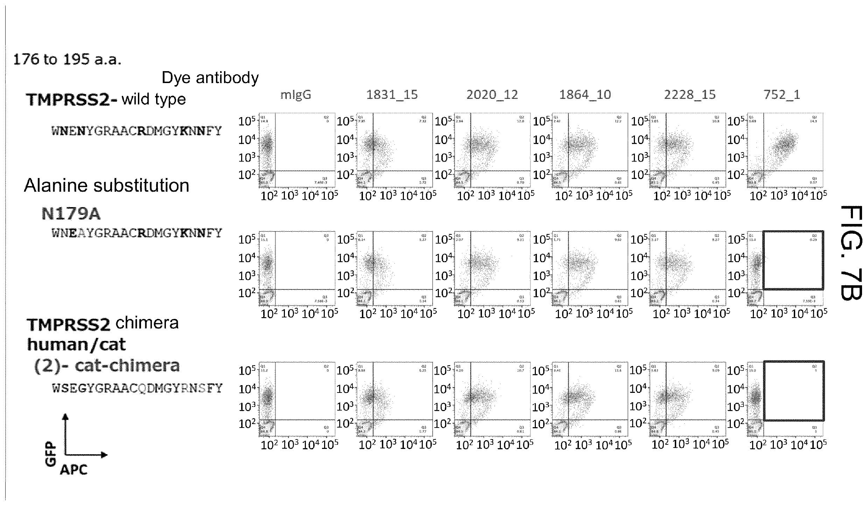

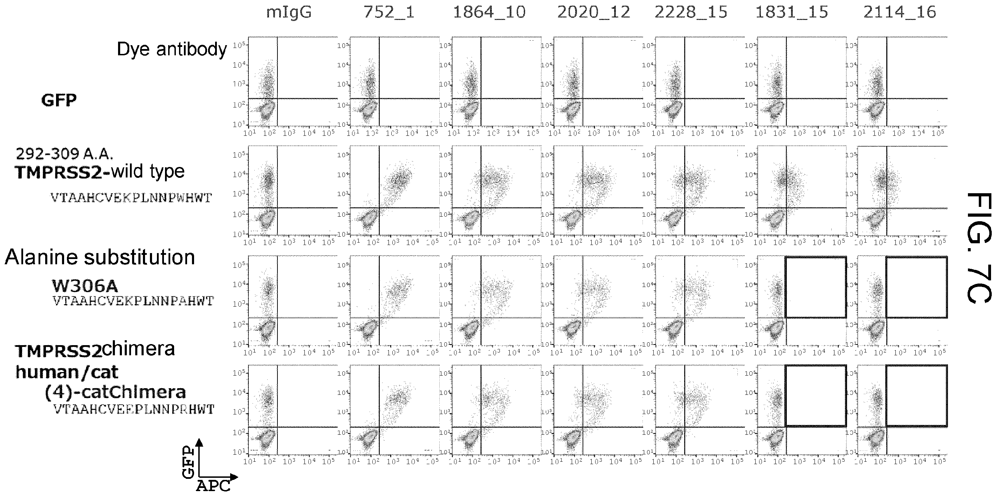

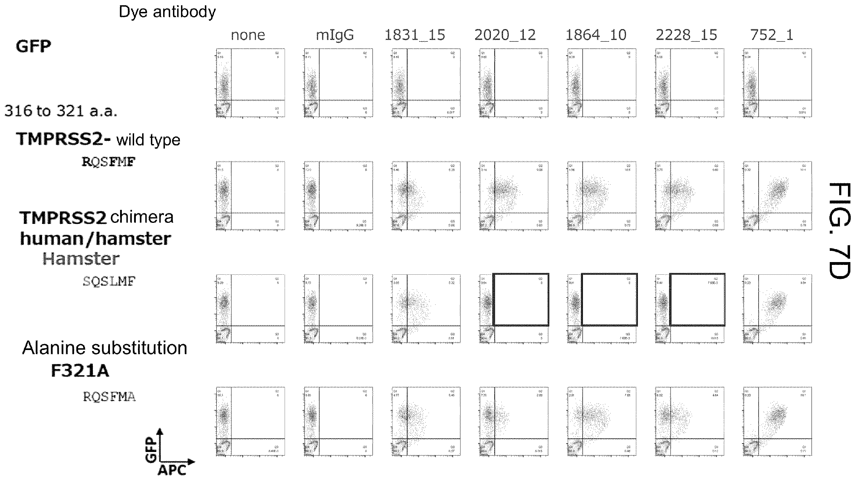

For the purpose of identification of amino acids critical for binding between TMPRSS2 and antibodies, TMPRSS2 variants having amino acid point mutation were produced to confirm the binding with the antibodies. The TMPRSS2 variants thus produced were linked to GFP via IRES and forcibly expressed in COS7 cells. GFP-positive cells are thought to express TMPRSS2 variants, and the binding between the cells and each antibody was analyzed by flow cytometry. The results were as shown in Figures 7A to 7D. As shown in Figure 7A, 752_1 antibody lost binding properties to TMPRSS2 chimera (human/176 to 195 cat) in which the 176th to 195th amino acid sequences of the human TMPRSS2 were replaced with a corresponding region of cat TMPRSS2. Therefore, N179 of the human TMPRSS2 was shown to be important for the binding of 752_1 antibody to human TMPRSS2. Also, as shown in Figure 7A, 1831_15 antibody lost binding properties to TMPRSS2 chimera (human/292 to 309 cat) in which the 292nd to 309th amino acid sequences of the human TMPRSS2 were replaced with a corresponding region of cat TMPRSS2. From this, the 292nd to 309th amino acid sequences of the human TMPRSS2 were shown to be important for the binding of 1831_15 antibody to human TMPRSS2. Next, as shown in Figure 7B, 752_1 antibody lost binding properties to N179A variant of TMPRSS2. Therefore, N179 of the human TMPRSS2 was shown to be important for the binding of 752_1 antibody to human TMPRSS2. Next, as shown in Figure 7C, 1831_15 antibody lost binding properties to W306A variant of human TMPRSS2. Therefore, W306 of the human TMPRSS2 was shown to be important for the binding of 1831_15 antibody to human TMPRSS2. Further, as shown in Figure 7D, 2020_12 antibody, 1864_10 antibody, and 2228_15 antibody lost binding properties to TMPRSS2 chimera (human/316 to 321 hamster cat) in which the 316th to 321st amino acid sequences of the human TMPRSS2 were replaced with a corresponding region of hamster cat TMPRSS2. From this, the 316th to 321st amino acid sequences of the human TMPRSS2 were shown to be important for the binding of 2020_12 antibody, 1864_10 antibody, and 2228_15 antibody to human TMPRSS2.

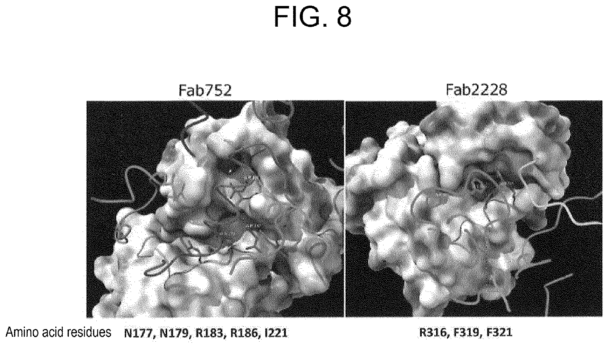

A Fab fragment of the 752_1 antibody and a Fab fragment of the 2228_15 antibody described above were prepared and formed into a complex with the human TMPRSS2 to observe with a cryo-electron microscope. Electron microscope images were analyzed on a computer, and the structures of the binding sites between TMPRSS2 and respective Fab fragments as shown in Figure 8 were obtained. As shown in Figure 8, the 752 Fab fragment was close to N177, N179, R182, R186, and I221 of TMPRSS2 in the complex and predicted to bind to these amino acids. Also, this result coincides with the binding data of TMPRSS2 variants shown in Figure 7A. In addition to that, as shown in Figure 8, the 2228 Fab fragment was close to R316, F319, and F321 of TMPRSS2 in the complex and predicted to bind to these amino acids. Further, this result coincides with the binding data of TMPRSS2 variants shown in Figure 7D. According to the above epitope binning, many antibodies were found to bind to these sites, and therefore, these epitopes are likely to be useful binding sites of the antibodies for inhibiting SARS-CoV-2 infection of cells.

The human TMPRSS2 is a cytoplasmic region on the N-terminal, the 84th to 106th amino acids are a transmembrane region, the 133rd to 148th amino acids are a LDLRA region, the 149th and 242nd amino acids are a SRCR region, and the 255th to 492nd are a serine protease region. The serine protease region contains catalytic triad of H296, D345, and S441.

Binding Properties to TMPRSS Family

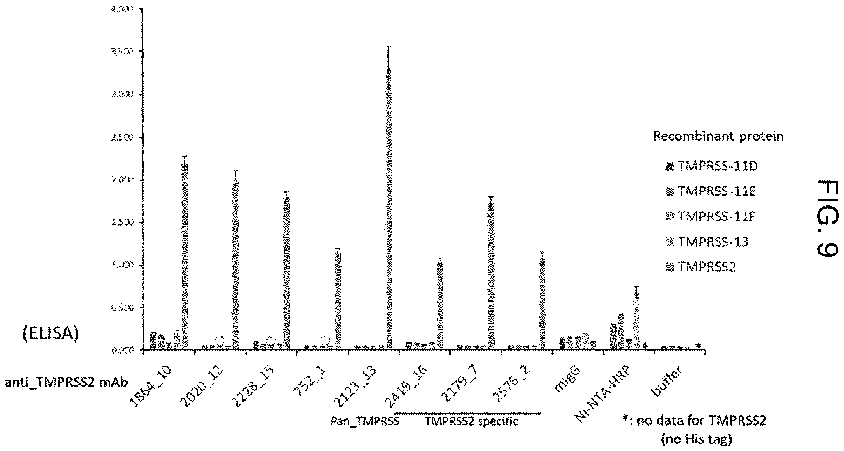

The binding properties of the 4 monoclonal antibodies (752_1 antibody, 2228_15 antibody, 1864_10 antibody, and 2020_12 antibody) to the TMPRSS family were confirmed. The results were as shown in Figure 9. As shown in Figure 9, these antibodies bound specifically only to TMPRSS2.

Cross-Reactivity

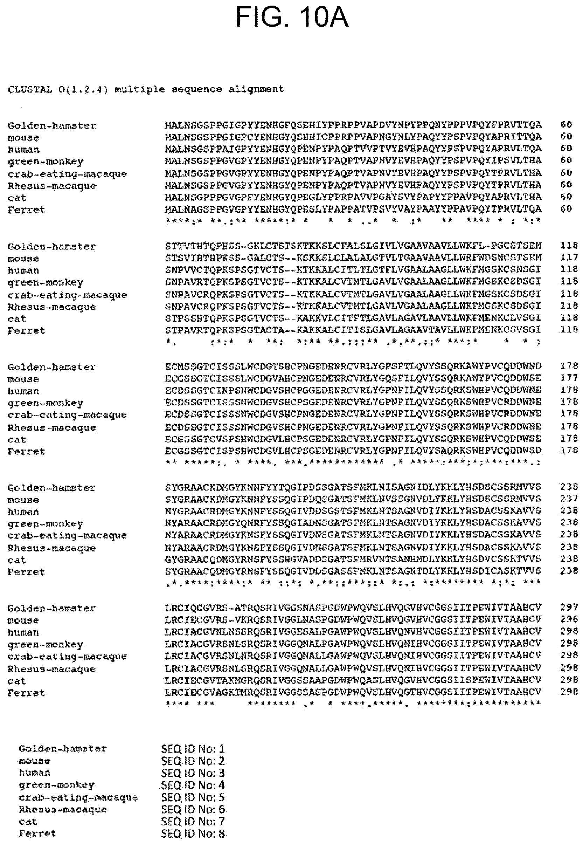

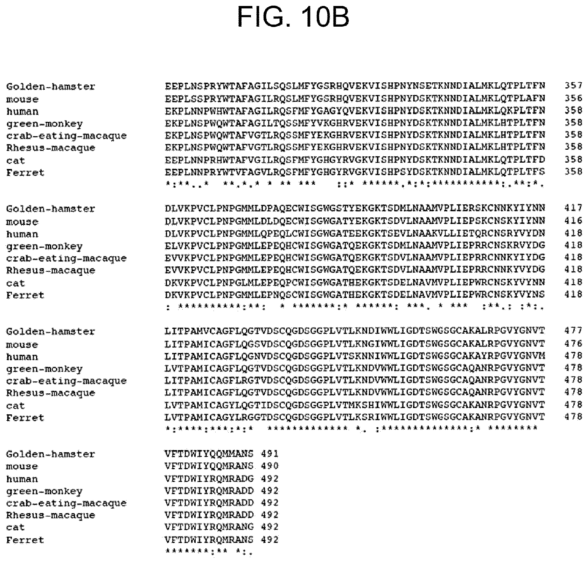

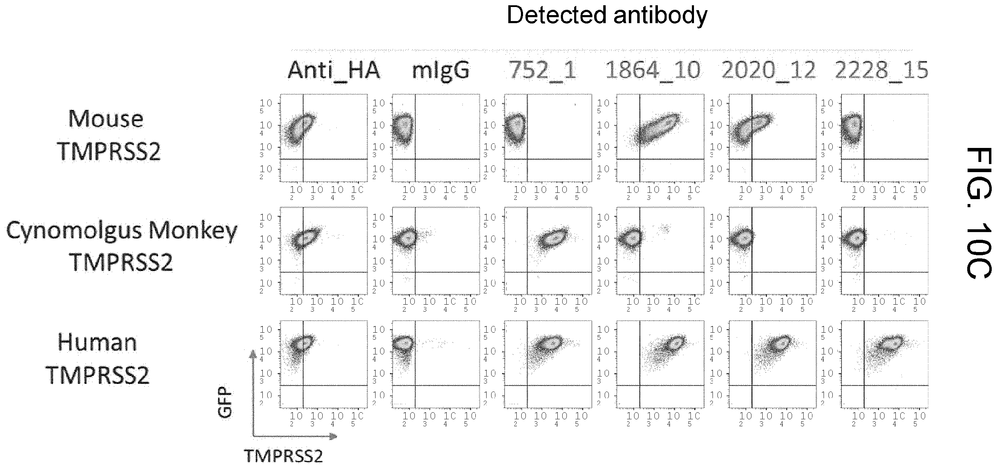

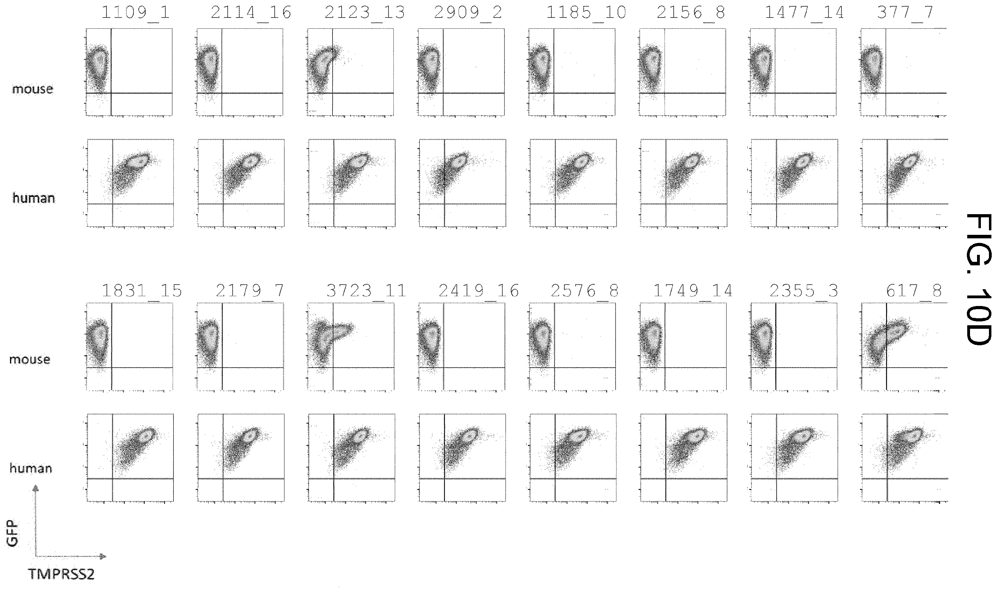

Cross-reactivity to TMPRSS2 of non-human animal species was confirmed. Specifically, the cross-reactivity of ferret (SEQ ID NO: 1), hamster (SEQ ID NO: 2), mouse (SEQ ID NO: 3), feline (SEQ ID NO: 4), human (SEQ ID NO: 5), green monkey (SEQ ID NO: 6), cynomolgus monkey (SEQ ID NO: 7), and rhesus macaque (SEQ ID NO: 8) to TMPRSS2 were confirmed. Note that the alignments of these TMPRSS2 were as shown in Figures 10A and 10B. Moreover, the results were as shown in Figures 10C and 10D.

As a result, 1864_10 and 2020_12 exhibited the cross-reactivity to mouse TMPRSS2. Also, 752_1 exhibited the cross-reactivity to cynomolgus monkey TMPRSS2.

Characteristics of the above 20 infection-inhibiting monoclonal antibodies were determined in the same manner, and the results are shown in Table 3.

[Table 3]

## Table 3: Characteristics of 20 infection-inhibiting monoclonal antibodies

| mAb | Activity | | | | Cross-reactivity | KD (M) | isotype |
|---|---|---|---|---|---|---|---|
| | Virus IC90 | Calu-3 Inhibiton | 293FT qAC50 (ug/mL.) | Binding inhibition between antibodies | | | |
| 752_1 | 0.3 | 68.4 | 0.04 | 752_1 | Cynomolgus monkey | 9.6E-09 | IgG1, κ |
| 1864_10 | 0.1 | 67.6 | 0.03 | 2228_15 | Mouse | 5.1E-09 | IgG2b, κ |
| 2020_12 | 0.2 | 66.0 | 0.04 | 2228_15 | Mouse | 2.7E-09 | IgG2c, κ |
| 2228_15 | 0.5 | 75.6 | 0.07 | 2228_15 | - | 8.4E-09 | IgG2c, κ |
| 1109_1 | 1.3 | 56.5 | 0.53 | 752_1 | Cynomolgus monkey | 1.2E-08 | IgG2c, κ |
| 2114_16 | 1.5 | 47.6 | 0.54 | 1831_15 | - | 1.0E-09 | IgG2c, λ |
| 2123_13 | 1.4 | 62.3 | 0.77 | 752_1 | Mouse·Cynomolgus monkey | 9.4E-09 | IgG2c, κ |
| 1831_15 | - | 44.8 | 0.26 | 1831_15 | Cynomolgus monkey | 4.2E-10 | IgG2b, κ |
| 617_8 | - | 52.2 | 0.46 | 2228_15 | Mouse | | IgG2c, κ |
| 2179_7 | - | 14.4 | 0.11 | 752_1 | Cynomolgus monkey | | IgG2c, κ |
| 3723_11 | - | 52.8 | 0.05 | 2228_15 | Mouse | | IgG2c, κ |
| 2909_2 | 2.4 | 61.7 | 0.13 | 2228_15 | - | | IgG3, κ |
| 1477_14 | 2.7 | 50.1 | 0.52 | 752_1 | Cynomolgus monkey | | IgG2c, κ |
| 2156_8 | 2.9 | 61.6 | 0.22 | 752_1 | Cynomolgus monkey | | IgG2c, κ |
| 1185_10 | 3.0 | 53.0 | 0.10 | 752_1 | Cynomolgus monkey | | IgG2c, κ |
| 377_7 | 4.7 | 58.9 | 0.36 | 752_1 | Cynomolgus monkey | | IgG2c, κ |
| 2419_16 | - | 5.6 | 0.26 | 752_1 | Cynomolgus monkey | | IgG2c, κ |
| 2576_2 | - | 1.1 | 0.43 | 752_1 | Cynomolgus monkey | | IgG2c, κ |
| 1749_14 | - | 43.2 | 0.53 | 752_1 | Cynomolgus monkey | | IgG2b, κ |
| 2355_3 | - | 43.1 | 0.49 | 752_1 | Cynomolgus monkey | | IgG2c, κ |

* The symbol "-" indicates not determined. The brank spaces also indicate not determined.

Antibody Sequencing

The amino acid sequences of the heavy chain and light chain of the 4 antibodies were determined, and the heavy chain CDR1 to 3 and the light chain CDR1 to 3 were presumed (see Table 4).

[Table 4]

| Table 4: Amino acid sequences of each monoclonal antibody | | | | | |
|---|---|---|---|---|---|
| Antibody | Region | SEQ ID NO. | Antibody | Region | SEQ ID NO. |
| 752_1 | Heavy chain variable region | SEQ ID NO: 9 | 2020_12 | Heavy chain variable region | SEQ ID NO: 25 |
| | Light chain variable region | SEQ ID NO: 10 | | Light chain variable region | SEQ ID NO: 26 |
| | Heavy chain CDR1 | SEQ ID NO: 11 | | Heavy chain CDR1 | SEQ ID NO: 27 |
| | Heavy chain CDR2 | SEQ ID NO: 12 | | Heavy chain CDR2 | SEQ ID NO: 28 |
| | Heavy chain CDR3 | SEQ ID NO: 13 | | Heavy chain CDR3 | SEQ ID NO: 29 |
| | Light chain CDR1 | SEQ ID NO: 14 | | Light chain CDR1 | SEQ ID NO: 30 |
| | Light chain CDR2 | SEQ ID NO: 15 | | Light chain CDR2 | SEQ ID NO: 31 |
| | Light chain CDR3 | SEQ ID NO: 16 | | Light chain CDR3 | SEQ ID NO: 32 |

(continued)

| Antibody | Region | SEQ ID NO. | Antibody | Region | SEQ ID NO. |
|---|---|---|---|---|---|
| 1864_10 | Heavy chain variable region | SEQ ID NO: 17 | 2228_15 | Heavy chain variable region | SEQ ID NO: 33 |
| | Light chain variable region | SEQ ID NO: 18 | | Light chain variable region | SEQ ID NO: 34 |
| | Heavy chain CDR1 | SEQ ID NO: 19 | | Heavy chain CDR1 | SEQ ID NO: 35 |
| | Heavy chain CDR2 | SEQ ID NO: 20 | | Heavy chain CDR2 | SEQ ID NO: 36 |
| | Heavy chain CDR3 | SEQ ID NO: 21 | | Heavy chain CDR3 | SEQ ID NO: 37 |
| | Light chain CDR1 | SEQ ID NO: 22 | | Light chain CDR1 | SEQ ID NO: 38 |
| | Light chain CDR2 | SEQ ID NO: 23 | | Light chain CDR2 | SEQ ID NO: 39 |
| | Light chain CDR3 | SEQ ID NO: 24 | | Light chain CDR3 | SEQ ID NO: 40 |
| 1831_15 | Heavy chain variable region | SEQ ID NO: 98 | 2114_16 | Heavy chain variable region | SEQ ID NO: 106 |
| | Light chain variable region | SEQ ID NO: 99 | | Light chain variable region | SEQ ID NO: 107 |
| | Heavy chain CDR1 | SEQ ID NO: 100 | | Heavy chain CDR1 | SEQ ID NO: 108 |
| | Heavy chain CDR2 | SEQ ID NO: 101 | | Heavy chain CDR2 | SEQ ID NO: 109 |
| | Heavy chain CDR3 | SEQ ID NO: 102 | | Heavy chain CDR3 | SEQ ID NO: 110 |
| | Light chain CDR1 | SEQ ID NO: 103 | | Light chain CDR1 | SEQ ID NO: 111 |
| | Light chain CDR2 | SEQ ID NO: 104 | | Light chain CDR2 | SEQ ID NO: 112 |
| | Light chain CDR3 | SEQ ID NO: 105 | | Light chain CDR3 | SEQ ID NO: 113 |

Table 4: Amino acid sequences of each monoclonal antibody

Production of Human IgG4 Chimeric Antibody

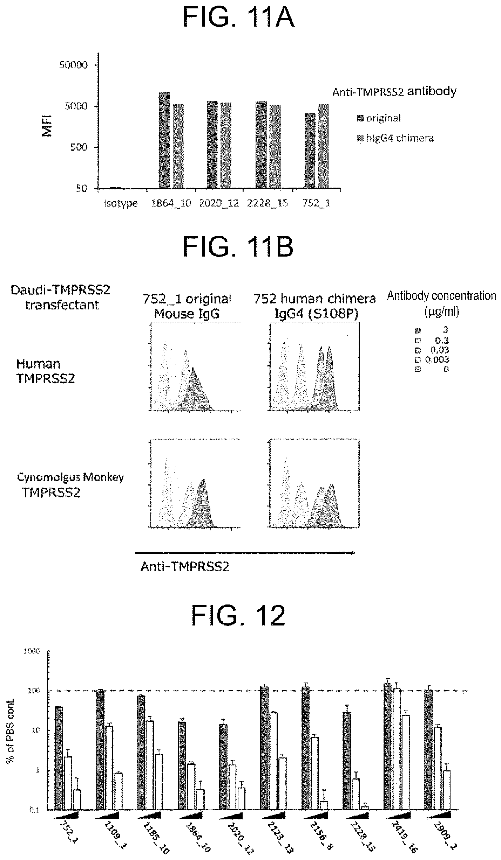

Human IgG4 chimeric antibodies were produced from the 4 monoclonal antibodies (752_1 antibody, 2228_15 antibody, 1864_10 antibody, and 2020_12 antibody) and human IgG4 antibody. The binding capacity of each of the human IgG4 chimeric antibodies to TMPRSS2 was evaluated by flow cytometry using TMPRSS2 and GFP expression Daudi cells, indicating that all of the human IgG4 chimeric antibodies maintained good binding capacity to TMPRSS2 (see Figure 11A). Further, S108P (where, 108 means the 108th amino acid as the 1st amino acid in the CH1 region) or S120P mutation was further introduced into the CH1 region of the human IgG4 chimeric antibody obtained from 752_1. From this mutation, Fab arm replacement is expected to be prevented. The binding properties between monkey TMPRSS2 and 752_1 human IgG4 chimeric antibody (S108P) were confirmed in the same manner as above. The results were as shown in Figure 11. As shown in Figure 11B, 752_1 human IgG4 chimeric antibody (S108P) exhibited better binding properties to human and monkey TMPRSS2 than to mouse antibody, which was its parent antibody.

Infection Inhibition Experiment

Next, the inhibition capacity of the obtained antibody to virus infection of human cells was tested. Calu-3 cells were used as the human cells and treated with serially diluted antibodies (0.1, 1 and 10 $\mu$g/mL). Virus (MOI = 0.1) was added 1 hour later. After 24 hours, the cells were washed with PBS to obtain cell lysates, and total RNA was extracted from the cells to synthesize cDNA. Viral genome and GAPDH as internal control were quantified by a quantitative PCR method. The amount of viral genome was estimated as the ratio of the amount of viral genome / the amount of GAPDH mRNA (S/G ratio). Also, for the above S/G ratio, the inhibitory efficiency of the antibody was estimated by determining the ratio to the negative control containing no antibody (i.e., no inhibitory effect). IC90 was determined as the antibody concentration achieving 90% inhibition. The results were as shown in Figure 12 and Table 5.

[Table 5]

Table 5: Infection inhibiting effects on raw virus by TMPRSS2 antibody

| $IC_{90}$ ($\mu$g/ml) | | | $IC_{90}$ ($\mu$g/ml) | | |
|---|---|---|---|---|---|
| Clone | First run | Second run | Clone | First run | Second run |
| 377_7 | 4.7 | n.t. | 1477_14 | 2.7 | n.t. |
| 617_8 | — | n.t. | 1749_14 | — | n.t. |
| 752_1 | 0.29 | 0.47 | 1831_15 | — | n.t. |
| 1109_1 | 1.3 | 2 | 1864_10 | 0.11 | 0.16 |
| 1185_10 | 3 | 2.5 | 2020_12 | 0.23 | 0.13 |
| $IC_{90}$ ($\mu$g/ml) | | | $IC_{90}$ ($\mu$g/ml) | | |
| Clone | First run | Second run | Clone | First run | Second run |
| 2114_16 | 1.5 | 3.4 | 2355_3 | — | n.t. |
| 2123_13 | 1.4 | 1.6 | 2419_16 | — | — |
| 2156_8 | 2.9 | n.t. | 2576_2 | — | n.t. |
| 2179_7 | — | n.t. | 2909_2 | 2.4 | 2.4 |
| 2228_15 | 0.47 | 0.24 | 3723_11 | — | n.t. |

\* The "n.t." means not tested.

As shown in Figure 12, all of the produced antibodies significantly inhibited the infection of the cells with live viruses. IC90 was as shown in Table 5.

Infection Inhibiting Effect on Viruses Having S Protein Variants

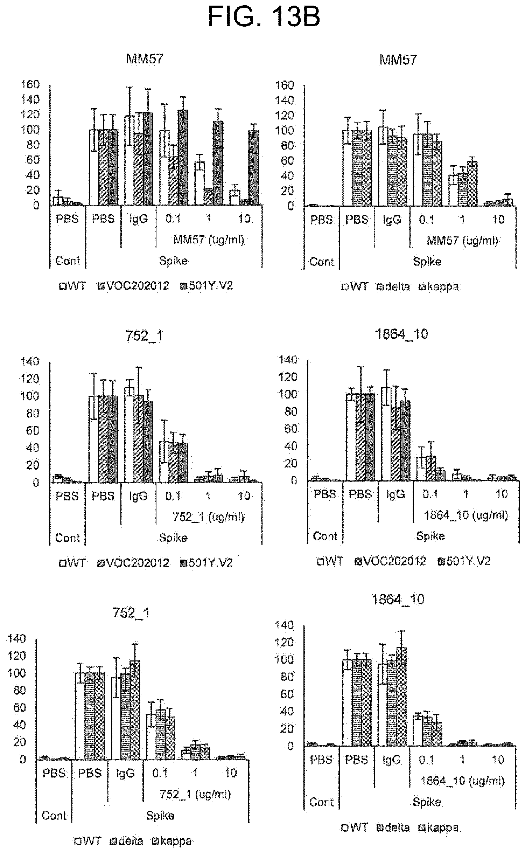

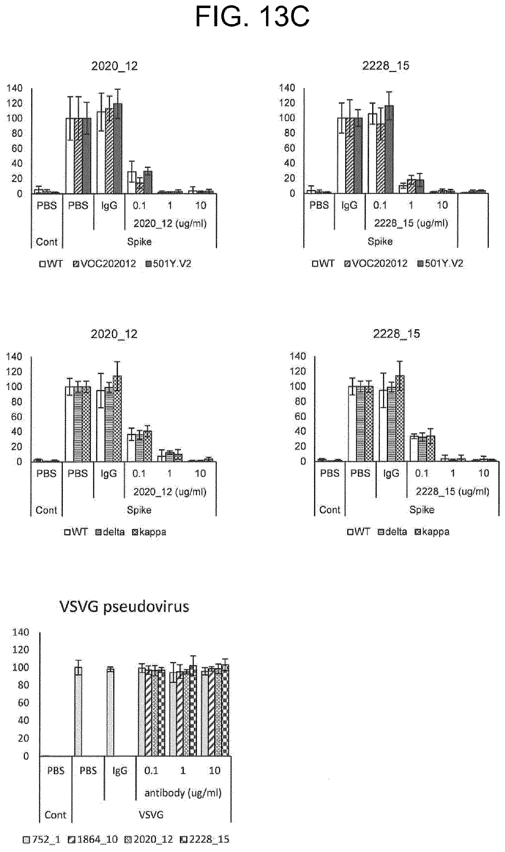

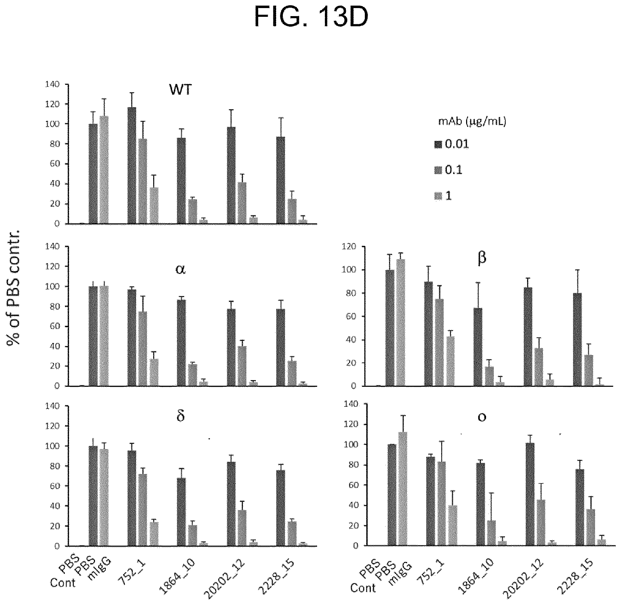

Pseudo-typed viruses having various S protein variants were prepared. Specifically, the following 3 variants were prepared: Pseudo-typed viruses having S proteins derived from SARS-CoV-2 (WT) derived from Wuhan, SARS_CoV_2 S VOC202012/01 (B.1.1.7: $\alpha$ variant), and SARS_CoV_2 S 501Y.V2 (B.1.351: $\beta$ variant). As test samples, commercially available anti-spike protein RBD antibody (Sino Biological, Inc., 40592-MM57, hereinafter, referred to as "MM57"), nafamostat, and the antibodies of the present example (752_1 antibody, 2228_15 antibody, 1864_10 antibody, and 2020_12 antibody) were used. MM57 antibody is an antibody capable of binding to the spike proteins to neutralize the S proteins of SARS-CoV-2. Nafamostat is a serine protease inhibitor known as an active ingredient for anticoagulant drugs. Recently, nafamostat has been shown to have inhibitory effects on SARS-CoV-2 infection of cells.
The results were as shown in Figure 13A. As shown in Figure 13A, MM57 exhibited strong inhibitory effects on WT-type virus and $\alpha$ variant-type virus, whereas it hardly exhibited significant inhibitory effects on $\beta$ variant-type virus. Nafamostat exhibited infection inhibiting effects on all the WT-type, $\alpha$ variant-type, and $\beta$ variant-type viruses. On the other hand, all of the antibodies of the present example exhibited infection inhibiting effects on all the WT-type, $\alpha$ variant-type, and $\beta$ variant-type viruses.
MM57 is an antibody against S proteins, which greatly reduced or eliminated the infection inhibiting effects on $\beta$ variant-type virus. On the other hand, the antibodies of the present example that bind to TMPRSS2, a protein of human cells, robustly exhibited infection inhibiting effects even on variants. Coronavirus proteins may induce mutation. Therefore, antibodies targeting the coronavirus S proteins may reduce or eliminate the binding properties to the S protein variants, and thereby reducing or eliminating the infection inhibiting effects. On the other hand, as for antibodies against human proteins, since mutation of human proteins is unlikely to occur, their effectiveness is unlikely to depend on coronavirus mutation. The results of the present example were consistent with this logic.
Furthermore, the infection inhibiting effects on pseudo-typed viruses having SARS-CoV-2 $\delta$ variant (B.1.617.2) and SARS-CoV-2 $\kappa$ variant (B.1.617.1) were also examined. As shown in Figures 13B and 13C, all of the antibodies of the present example exhibited strong infection inhibiting effects on $\delta$ variant-type, and x variant-type viruses. In addition to that, the infection inhibiting effects on pseudo-typed viruses having the S protein of the omicron variant (o variant) were examined. As shown in Figure 13D, the antibodies exhibited strong infection suppressing effects on pseudo-typed viruses having the S protein derived from the o variant, as with the α variant, β variant, and δ variant. As such, the effectiveness of the antibodies against all variants was demonstrated regardless of the virus variant from which it is derived. Also, none of the antibodies exhibited the infection inhibiting effect on VSV-G virus, which was used as a negative control. From this, it is clearly indicated that the virus infection inhibiting effects of the antibodies of the present example is exerted via the interaction between TMPRSS2 and S proteins.

The ADCC activities were tested for 1864_10 antibody (mlgG2b) and 2020_12 antibody (mlgG2c). ADE

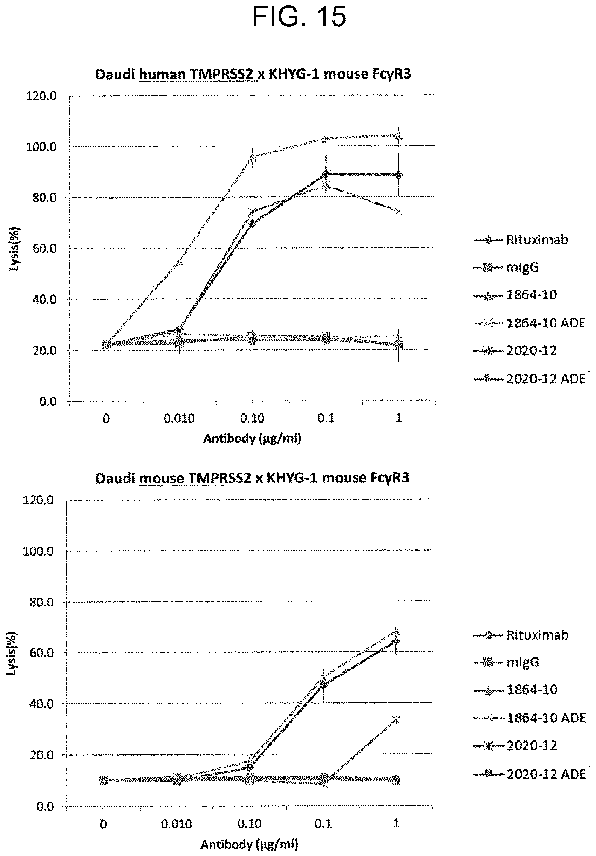

- substituted recombinant antibodies (1864_10 ADE- and 2020_12 antibody ADE-) having mutations of L244A/E245A/-P339A and L244A/E245A/P338A were each produced for these antibodies (parent antibodies). Daudi cells (Daudi; TM2-HA-k13-LD10, mouse TM2 2nd LD1, $1 \times 10^4$ cells/well) in which human TMPRSS2 or mouse TMPRSS2 was forcedly expressed were prepared. The above Daudi cells were incubated at 37°C for 1 hour in the presence of Calcein-AM. KHYG-1 mFcRy3 transfectant (hNK cell line) at $1 \times 10^5$/well was used as the effector cells. The ratio of the effector cells and Daudi cells was set to 10:1. As a positive control antibody, rituximab was used as an antibody having ADCC activity. As a negative control, a mouse IgG isotype control antibody was used. As test antibodies, 1864_10 antibody (mlgG2b) and 2020_12 antibody (mlgG2c), and ADE-substituted recombinant antibodies thereof were used. The lysis rate (%) of Daudi cells by ADCC activity was measured in the presence of various antibodies. The results were as shown in Figure 15. As shown in Figure 15, the ADE-substituted recombinant antibodies had no detectable ADCC activity.

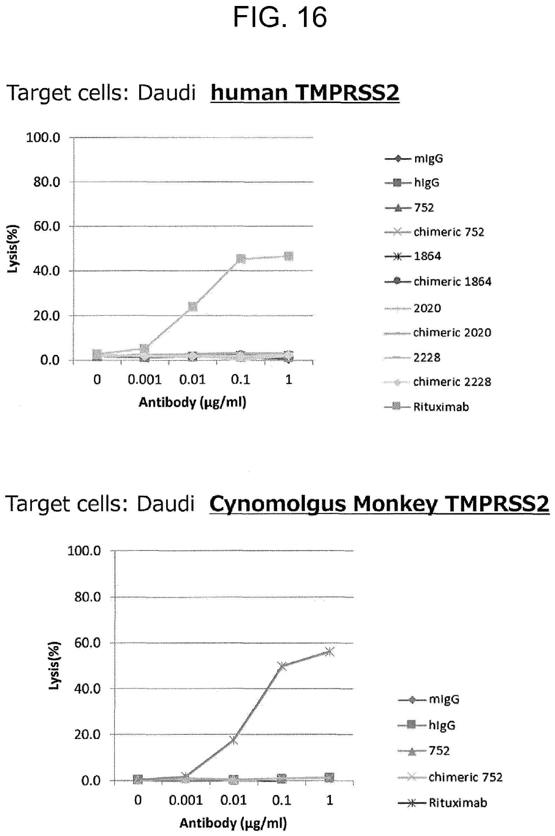

In addition to that, the presence or absence of ADCC activity of hIgG4 chimeric antibody was confirmed. In the same manner as above, Daudi cells expressing human TMPRSS2 or cynomolgus monkey TMPRSS2 were used, and KHYG-1 hFcRy3 transfectant (hNK cell line) at $1 \times 10^5$/well was used as the effector cells. The results were as shown in Figure 16. As a result, antibodies other than rituximab did not exhibit significant cell lysis (%), and no significant ADCC activity was observed.

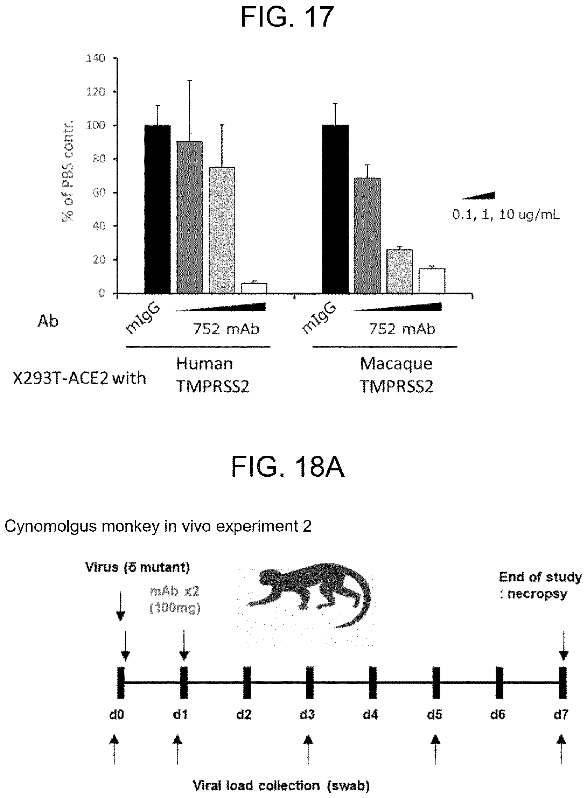

For 752hIgG4 (S120P) chimeric antibody, SARS-CoV-2 infection inhibition experiment was conducted in vitro. The cells used were X293T-ACE2 expressing human or macaque TMPRSS2. The results were as shown in Figure 17. As shown in Figure 17, the antibodies inhibited the infection of X293T cells with SARS-CoV-2 in a concentration-dependent manner.

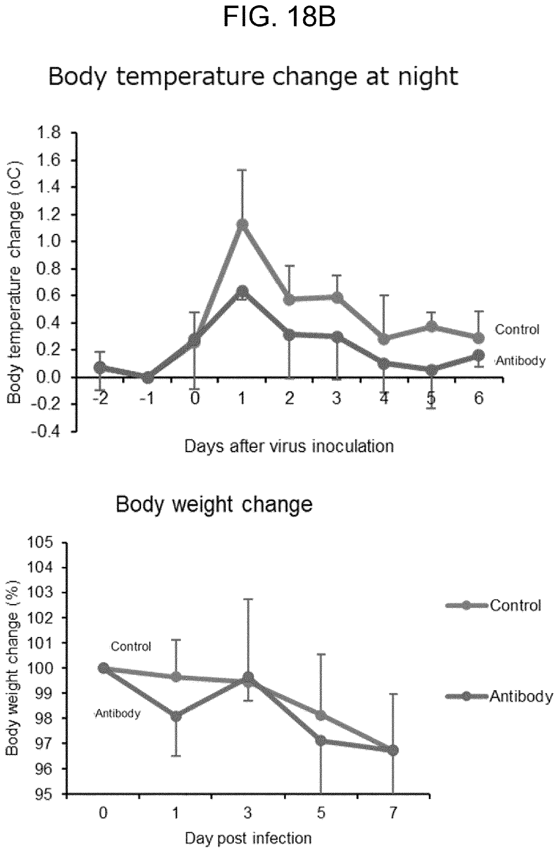

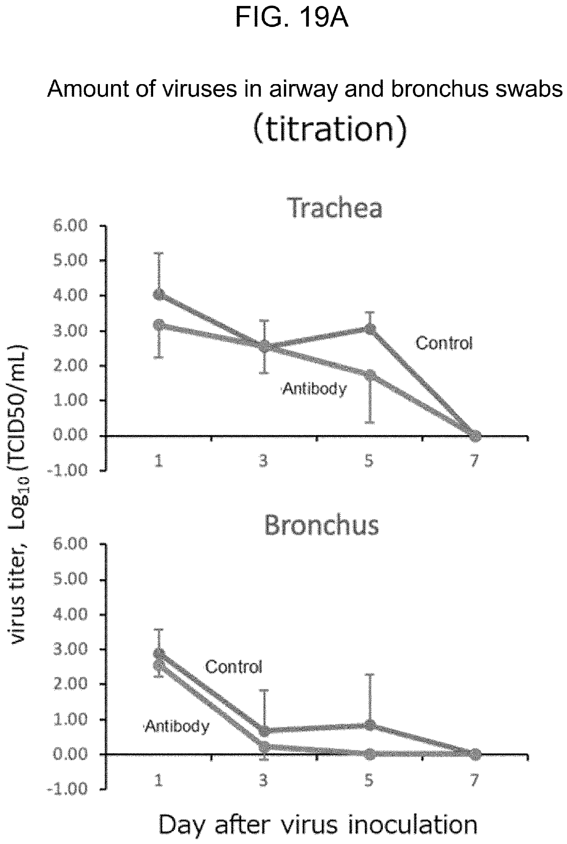

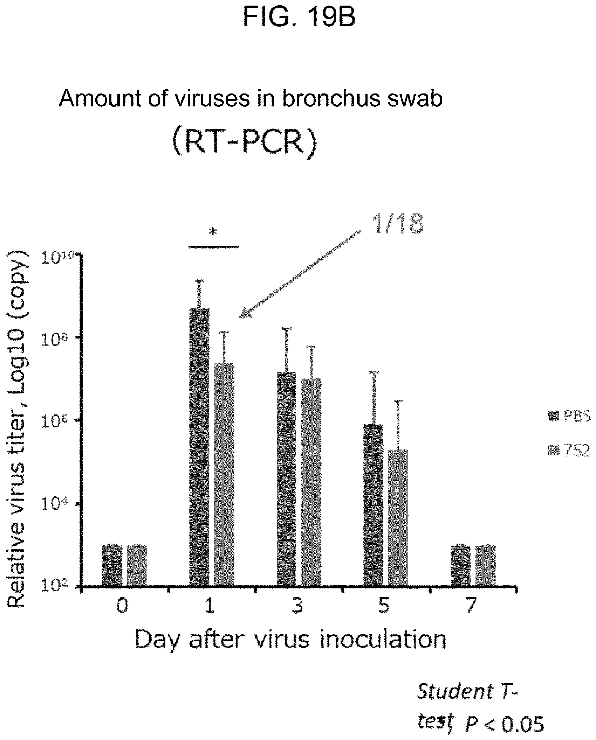

In an in vivo test using cynomolgus monkeys, an infection experiment of cynomolgus monkeys with SARS-CoV-2 was conducted. As shown in Figure 18A, the δ variant was administered on Day 0, and 752hIgG4 (S120P) chimeric antibody (100 mg) was administered twice on Day 0 and Day 1. Swabs were collected on Days 3, 5, and 7. The body temperature and weight changes of the cynomolgus monkeys were as shown in Figure 18B. As shown in Figure 18B, the body temperature of the antibody administration group was lower than the control group, indicating the therapeutic effect of the antibody administration. The number of viruses in the airway and bronchus swabs was measured by the titration method. As a result, as shown in Figure 19A, the number of viruses in the airway and bronchus swabs reduced in the antibody administration group. Also, as shown in Figure 19B, the number of viruses in the airway swabs reduced in the antibody administration group by RT-PCR. The number of viruses was approximately 1/18 of that in the control group.

The lung tissues were resected from the cynomolgus monkeys on Day 7, and the lung tissues were observed. The tissues were evaluated according to Table 6 below. The average value across all the visual fields was used as the visual lung injury score.

**[Table 6]**

| Table 6: Lung histopathologic diagnosis score | |
| --- | --- |
| Score | Tissue states |
| 0 | Healthy lung |
| 1 | Mild destruction of epithelial cells in airway and bronchus |
| 2 | Mild infiltration of inflammatory cells around periphery of bronchus |
| 3 | Moderate infiltration of inflammatory cells around alveolar walls, and resulting alveolar thickening |
| 4 | Mild alveolar injury with vascular injury of 10% or less |
| 5 | Moderate alveolar and vascular injury (11% to approximately 30%) |
| 6 | Severe alveolar injury with hyaline membrane-associated alveolar hemorrhage of 31% to approximately 50% |
| 7 | Severe alveolar injury with hyaline membrane-associated alveolar hemorrhage of 51% or more |

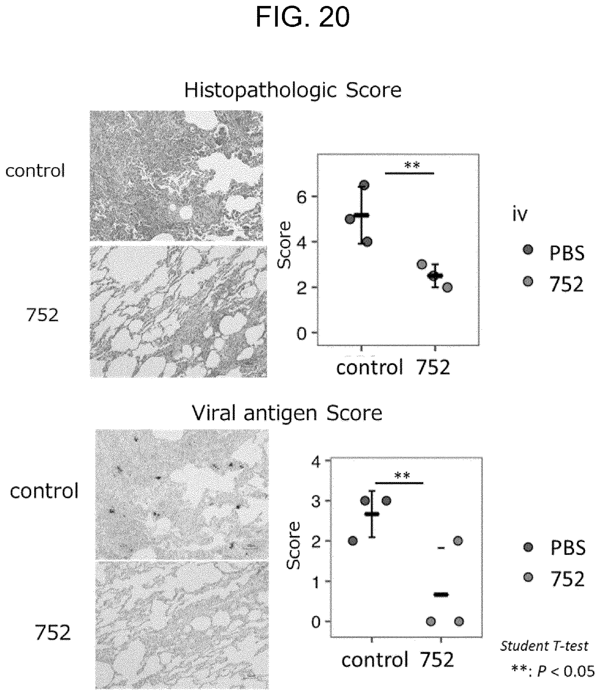

As a result, as shown in Figure 20, the lung histopathologic diagnosis score for the cynomolgus monkeys infected with SARS-CoV-2 was improved in the antibody administration group. Also, a viral antigen test was conducted. The percentage of viral antigen positive cells was evaluated using 8G8A, which is mAb against SARS nucleocapsid protein (0: none, 1: hardly observed, 2: moderately observed, 3: highly observed). As a result, as shown in Figure 20, the amount of viral antigen was significantly reduced in the antibody administration group. As such, the lung histopathologic diagnosis score for the cynomolgus monkeys infected with SARS-CoV-2 on Day 7 of infection was improved by antibody administration, and the amount of viral antigen was significantly reduced.

In the following, 752 antibody and 2228 antibody were humanized.

**[Table 7]**

Table 7: Amino acid sequences of humanized light chain variable regions (VL) and heavy chain variable regions (VH)

| subtype | FR origin | a.a. residues | amino acid sequence |
|---|---|---|---|
| 752_hVL-1 | NCBI protein DB | 106 | DIQMTQSPSSLSASVGDRVTITCKASQDINKYIAWYQQKPGKAPKLLIYYTSSLQPGVPSRFSGSGSGTDFTFTISSLQPEDIATYYCLQYYNLWTFGGGTKVEIK |
| 752_hVL-2 | 752_hVL-1 modified with back mutation | 106 | DIQMTQSPSSLSASVGDRVTITCKASQDINKYIAWYQQKPGKAPKLLIYYTSSLQPGVPSRFSGSGSGTDFTFTISSLQPEDIATYYCLQYYNLWTFGGGTKLEIK |
| 752_1 | L original | kappa | DIQMTQSPSSLSASLGGKVTITCKASQDINKYIAWYQHKPGKGPRLLIHYTSTLQPGIPSRFSGSGSGRDYSFSIYNLEPEDIATYYCLQYYNLWTFGGGTKLEIK |
| 752_hVH-1 | NCBI protein DB | 120 | QVQLVQSGAEVKKPGASVKVSCKASGYTFTSCGISWVRQAPGQGLEWMGEIYPRGGNTYYNEKFKGRVTMTTDTSTSTAYMELRSLRSDDTAVYYCARENGNYDPLFAYWGQGTLVTVSS |
| 752_hVH-2 | Germline DB | 120 | QVQLQQSGTGLARPGASVKLSCKASGYTFTSCGISWVRQRAGQGLEWIGEIYPRGGNTYYNEKFKGKATLTADKSSSTAYMELRGLTAEDSAVYFCARENGNYDPLFAYWGQGTLTVSS |
| 752_1 | H original | mIgG1 | QVQLQQSGAELARPGASVKLSCKASGYTFTSCGISWVKQRTGQGLEWIGEIYPRGGNTYYNEKFKGKATLTADKSSSTAYMELRSLTSEDSAVYFCARENGNYDPLFAYWGQGTLVTVSA |
| 2228_hVL-1 | 1st candidate Sequence of Humanized 2228-15_VL | 111 | DVVMTQSPLSLPVTLGQPASISCRASKSVSTSGYSFMHWFQQRPGQSPRLLIYLASNLESGVPDRFSGSGSGTDFTLKISRVEAEDVGVYYCQHSRELPLTFGGGTKVEIK |
| 2228_hVL-2 | Germline DB | 111 | DIVMTQSPDSLAVSLGERATINCRASKSVSTSGYSFMHWYQQKPGQPPKLLIYLASNLESGVPDRFSGSGSGTDFTLTIRPLQAEDVAVYYCQHSRELPLTFGQGTKLEIK |
| 2228_15 | L original | kappa | DVVVTQSPASLAVSLGQRATISCRASKSVSTSGYSFMHWYQQKPGQPPKLLIYLASNLESGVPARFSGSGSGTDFILNIHPVEEEDAATYYCQHSRELPLTFGAGTKLELK |
| 2228_hVH-1 | NCBI protein DB | 121 | QVQLVQSGAEVKKPGASVKVSCKASGYTFTDYIIHWVRQAPGQGLEWMGWIFPGSGSSYYNAIFKGRVTMTRDTSISTAYMELSSLRSDDTAVYYCARGDFGNFGGPFTYWGQGTLVTVSS |
| 2228_hVH-2 | Germline DB | 121 | EIQLVQSGAEVKKPGSSVKVSCKASGYTFTDYIIHWMRQAPGQGLEWIGWIFPGSGSSYYNAIFKGRATLVDTSISTAYMELSSLRSEAFYFCARGDFGNFGGPFTYWGQGTLVTVSS |
| 2228_15 | H original | mIgG2c | QVQLQQSGPELVKPGTSVKISCKASGYTFTDYIIHWVKQRPGQGLEWIGWIFPGSGSSYYNAIFKGKATLTVDTSSNTAHMSLSSLTSDDSAVYFCARGDFGNFGGPFTYWGQGTLVTVSA |

Orange: CDR    Green: a.a. similar to original a.a.    Red: a.a. dissimilar to original a.a.

When humanizing, CDRs were grafted onto 2 different frameworks, and 2 VHs and 2 VLs per 1 antibody clone were obtained (see Table 7). 4 Humanized antibody prototypes were obtained by appropriately combining the above VHs and VLs. The amino acid sequence of human IgG4 (S108P) was used as the constant region. The binding affinity of the obtained humanized antibody was measured by BIACore.

**[Table 8]**

Table 8: KD values of humanized antibodies

| | recombinant | H chain subtype | L chain subtype | ka (1/Ms) | kd (1/s) | KD (M) | Chi² (RU²) |
|---|---|---|---|---|---|---|---|
| recombinant 752-1 mIgG | **mIgG** | mIgG1 | mIg kappa | 5.42E+05 | 0.001409 | **2.60E-09** | 14.6 |
| humanized 752-1 IgG4 (S108P) | prototype #1 | 752_hVH-1 | 752_hVL-1 | 4.12E+05 | 0.009321 | 2.26E-08 | 30.2 |
| | prototype #2 | 752_hVH-1 | 752_hVL-2 | 1.13E+06 | 0.01521 | 1.34E-08 | 85.8 |
| | prototype #3 | 752_hVH-2 | 752_hVL-1 | 2.22E+06 | 0.004691 | 2.11E-09 | 6.98 |
| | prototype #4 | 752_hVH-2 | 752_hVL-2 | 2.50E+06 | 0.006114 | 2.44E-09 | 11.7 |
| | recombinant | H chain subtype | L chain subtype | ka (1/Ms) | kd (1/s) | KD (M) | Chi² (RU²) |
| recombinant 2228-15 mIgG | **mIgG** | mIgG2C | mIg kappa | 3.38E+05 | 0.001257 | **3.72E-09** | 3.63 |
| humanized 2228-15 IgG4 (S108P) | prototype #1 | 2228_hVH-1 | 2228_hVL-1 | 5.22E+08 | 0.0641 | 1.23E-10 | 87.5 |
| | prototype #2 | 2228_hVH-1 | 2228_hVL-2 | 7.31E+05 | 0.001039 | 1.42E-09 | 7.2 |
| | prototype #3 | 2228_hVH-2 | 2228_hVL-1 | 8.25E+05 | 7.74E-04 | 9.38E-10 | 3.03 |
| | prototype #4 | 2228_hVH-2 | 2228_hVL-2 | 1.44E+06 | 4.00E-04 | 2.79E-10 | 2.63 |

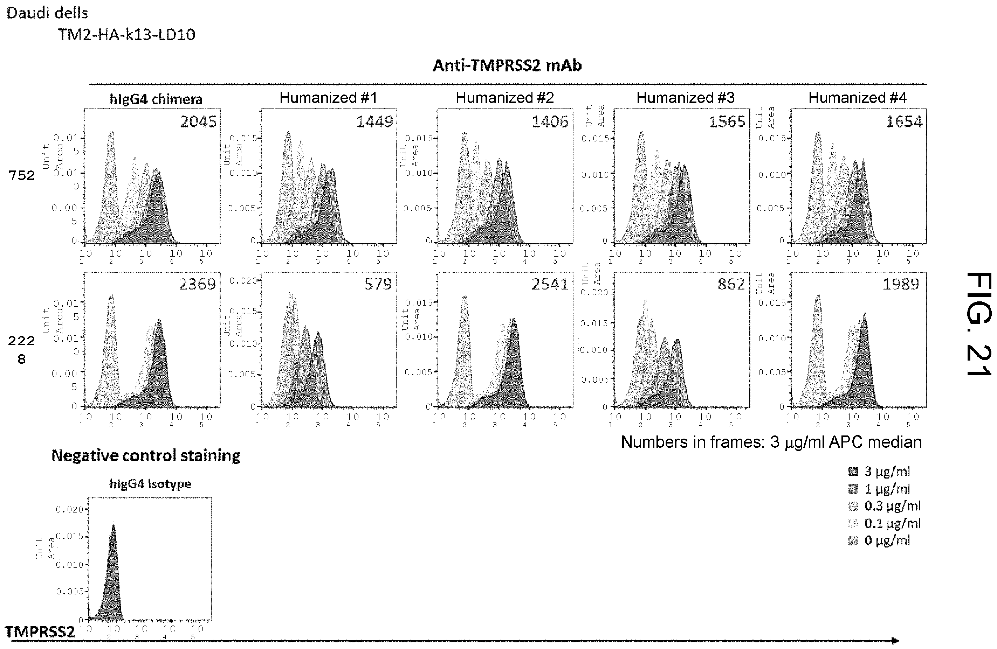

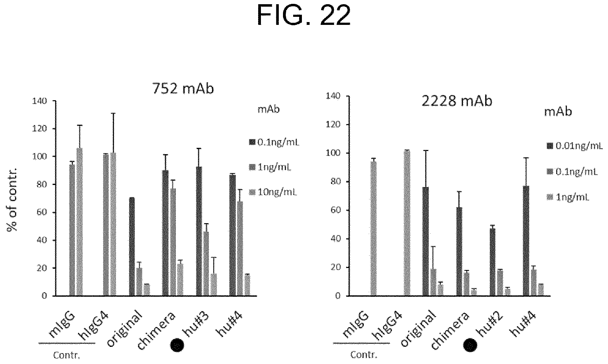

As shown in Table 8, all the humanized antibody prototypes exhibited preferable KD values. Each of these antibodies can be used as a humanized antibody. In Figure 21, concentration-dependent binding between these antibodies (each independently, also referred to as humanized #1 to #4 or hu #1 to #4) and TMPRSS2 was confirmed. Infection inhibition experiments were conducted in vitro using the obtained humanized IgG4 (S120P) antibody and pseudotyped virus. The cells were human lung epithelial adenocarcinoma (Calu3 cells). As a result, as shown in Figure 22, all the antibodies exhibited a preferable infection inhibiting effect.

The documents cited herein are incorporated herein by reference in their entirety.

Sequence Listing Contents

SEQ ID NO: 1: Example of ferret TMPRSS2

MALNSGSPPG IGPYYENHGF QSEHIYPPRP PVAPDVYNPY PPQNYPPPVP QYFPRVTTQA  60

STTVTHTQPH SSGKLCTSTS KTKKSLCFAL SLGIVLVGAA VAAVLLWKFL PGCSTSEMEC 120

MSSGTCISSS LWCDGTSHCP NGEDENRCAV RLYGPSFTLQ VYSSQRKAWY PVCQDDWNDS 180

YGRAACKDMG YKNNFYYTQG IPDSSGATSF MKLNISAGNI DLYKKLYHSD SCSSRMVVSL 240

RCIQCGVRSA TRQSRIVGGS NASPGDWPWQ VSLHVQGVHV CGGSIITPEW IVTAAHCVEE 300

PLNSPRYWTA FAGILSQSLM FYGSRHQVEK VISHPNYNSE TKNNDIALMK LQTPLTFNDL 360

VKPVCLPNPG MMLDPAQECW ISGWGSTYEK GKTSDMLNAA MVPLIERSKC NNKYIYNNLI 420

TPAMVCAGFL QGTVDSCQGD SGGPLVTLKN DIWWLIGDTS WGSGCAKALR PGVYGNVTVF 480

TDWIYQQMMA NS                    492

SEQ ID NO: 2: Example of hamster TMPRSS2

MALNSGSPPG IGPCYENHGY QSEHICPPRP PVAPNGYNLY PAQYYPSPVP QYAPRITTQA 60

STSVIHTHPK SSGALCTSKS KKSLCLALAL GTVLTGAAVA AVLLWRFWDS NCSTSEMECG 120

SSGTCISSSL WCDGVAHCPN GEDENRCVRL YGQSFILQVY SSQRKAWYPV CQDDWSESYG 180

RAACKDMGYK NNFYSSQGIP DQSGATSFMK LNVSSGNVDL YKKLYHSDSC SSRMVVSLRC 240

IECGVRSVKR QSRIVGGLNA SPGDWPWQVS LHVQGVHVCG GSIITPEWIV TAAHCVEEPL 300

SSPRYWTAFA GILRQSLMFY GSRHQVEKVI SHPNYDSKTK NNDIALMKLQ TPLAFNDLVK 360

PVCLPNPGMM LDLDQECWIS GWGATYEKGK TSDVLNAAMV PLIEPSKCNS KYIYNNLITP 420

AMICAGFLQG SVDSCQGDSG GPLVTLKNGI WWLIGDTSWG SGCAKALRPG VYGNVTVFTD 480

WIYQQMRANS 490

SEQ ID NO: 3: Example of mouse TMPRSS2

MALNSGPPPG VGPFYENHGY QPEGLYPPRP AVVPGAYSVY PAPYYPPAVP QYAPRVLTHA 60

STPSSHTQPK SPSGTVCTSK AKKVLCITFT LGAVLAGAVL AAVLLWKFME NKCLVSGIEC 120

GSSGTCVSPS HWCDGVLHCP SGEDENRCVR LYGPNFILQV YSSQRKSWHP VCQDDWSEGY 180

GRAACQDMGY RNSFYSSHGV ADDSGATSFM RVNTSANHMD LYKKLYHSDV CSSKTVVSLR 240

CIECGVTAKM GRQSRIVGGS SAAPGDWPWQ ASLHVQGVHV CGGSIISPEW IVTAAHCVEE 300

PLNNPRHWTA FVGILRQSFM FYGHGYRVGK VISHPNYDSK TKNNDIALMK LQTPLTFDDK 360

VKPVCLPNPG LMLEPEQPCW ISGWGATHEK GKTSDELNAV MVPLIEPWRC NSKYVYNNLV 420

TPAMICAGYL QGTIDSCQGD SGGPLVTMKS HIWWLIGDTS WGSGCAKANR PGVYGNVTVF 480

TDWIYRQMRA NG 492

SEQ ID NO: 4: Example of cat TMPRSS2

MALNSGSPPA IGPYYENHGY QPENPYPAQP TVVPTVYEVH PAQYYPSPVP QYAPRVLTQA 60

SNPVVCTQPK SPSGTVCTSK TKKALCITLT LGTFLVGAAL AAGLLWKFMG SKCSNSGIEC 120

DSSGTCINPS NWCDGVSHCP GGEDENRCVR LYGPNFILQV YSSQRKSWHP VCQDDWNENY 180

GRAACRDMGY KNNFYSSQGI VDDSGSTSFM KLNTSAGNVD IYKKLYHSDA CSSKAVVSLR 240

CIACGVNLNS SRQSRIVGGE SALPGAWPWQ VSLHVQNVHV CGGSIITPEW IVTAAHCVEK 300

PLNNPWHWTA FAGILRQSFM FYGAGYQVEK VISHPNYDSK TKNNDIALMK LQKPLTFNDL 360

VKPVCLPNPG MMLQPEQLCW ISGWGATEEK GKTSEVLNAA KVLLIETQRC NSRYVYDNLI 420

TPAMICAGFL QGNVDSCQGD SGGPLVTSKN NIWWLIGDTS WGSGCAKAYR PGVYGNVMVF 480

TDWIYRQMRA DG 492

SEQ ID NO: 5: Example of human TMPRSS2

MALNSGSPPG VGPYYENHGY QPENPYPAQP TVAPNVYEVH PAQYYPSPVP QYIPSVLTHA 60
SNPAVRTQPK SPSGTVCTSK TKKALCVTMT LGAVLVGAAL AAGLLWKFMG SKCSDSGIEC 120
DSSGTCISSS NWCDGVSHCP SGEDENRCVR LYGPNFILQV YSSQRKSWHP VCRDDWNENY 180
ARAACRDMGY QNRFYSSQGI ADNSGATSFM KLNTSAGNVD IYKKLYHSDA CSSKAVVSLR 240
CIACGVRSNL SRQSRIVGGQ NALPGAWPWQ VSLHVQNIHV CGGSIITPEW IVTAAHCVEK 300
PLNSPWQWTA FAGILTQSSM FYVKGHRVEK VISHPNYDSK TKNNDIALMK LHTPLTFNEL 360
VKPVCLPNPG MMLEPEQHCW ISGWGATQEK GKTSDMLNAA MVPLIEPRRC NSKRVYDGLV 420
TPAMICAGFL QGTVDSCQGD SGGPLVTLKN DVWWLIGDTS WGSGCAQANR PGVYGNVTVF 480
TDWIYRQMRA DD                     492

SEQ ID NO: 6: Example of green monkey TMPRSS2

MALNSGSPPG VGPYYENHGY QPENPYPAQP TVAPNVYEVH PAQYYPSPVP QYTPRVLTHA 60
SNPAVCRQPK SPSGTVCTSK TKKALCVTMT LGAVLVGAAL AAGLLWKFMG SKCSDSGIEC 120
DSSGTCISSS NWCDGVSHCP NGEDENRCVR LYGPNFILQV YSSQRKSWHP VCRDDWNENY 180
ARAACRDMGY KNSFYSSQGI VDNSGATSFM KLNTSAGNVD IYKKLYHSDA CSSKAVVSLR 240
CIACGVRSNL NRQSRIVGGQ NALLGAWPWQ VSLHVQNIHV CGGSIITPEW IVTAAHCVEK 300
PLNSPWQWTA FVGTLRQSSM FYEKGHRVEK VISHPNYDSK TKNNDIALMK LHTPLTFNEV 360
VKPVCLPNPG MMLEPEQHCW ISGWGATQEK GKTSDVLNAA MVPLIEPRRC NNKYIYDGLI 420
TPAMICAGFL RGTVDSCQGD SGGPLVTLKN DVWWLIGDTS WGSGCAQANR PGVYGNVTVF 480
TDWIYRQMRA DD                     492

SEQ ID NO: 7: Example of cynomolgus monkey TMPRSS2

MALNSGSPPG VGPYYENHGY QPENPYPAQP TVAPNVYEVH PAQYYPSPVP QYTPRVLTHA 60
SNPAVCRQPK SPSGTVCTSK TKKALCVTMT LGAVLVGAAL AAGLLWKFMG SKCSDSGIEC 120
DSSGTCISSS NWCDGVSHCP NGEDENRCVR LYGPNFILQV YSSQRKSWHP VCRDDWNENY 180
ARAACRDMGY KNSFYSSQGI VDNSGATSFM KLNTSAGNVD IYKKLYHSDA CSSKAVVSLR 240
CIACGVRSNL SRQSRIVGGQ NALLGAWPWQ VSLHVQNIHV CGGSIITPEW IVTAAHCVEK 300
PLNSPWQWTA FVGTLRQSSM FYEKGHRVEK VISHPNYDSK TKNNDIALMK LHTPLTFNEV 360
VKPVCLPNPG MMLEPEQHCW ISGWGATQEK GKTSDVLNAA MVPLIEPRRC NNKYVYDGLI 420
TPAMICAGFL QGTVDSCQGD SGGPLVTLKN DVWWLIGDTS WGSGCAQANR PGVYGNVTVF 480
TDWIYRQMRA DD                     492

SEQ ID NO: 8: Example of rhesus macaque TMPRSS2

MALNAGSPPG VGPYYENHGY QPESLYPAPP ATVPSVYVAY PAAYYPPAVP QYTPRVLTQA 60

STPAVRTQPK SPSGTACTAK AKKALCITIS LGAVLAGAAV TAVLLWKFME NKCSVSGIEC 120

GSSGTCISPS HWCDGVLHCP SGEDENRCVR LYGPNFILQV YSAQRKSWHP VCQDDWSDSY 180

GRAACQDMGY RNSFYSSQGI VDDSGASSFM KLNTSAGNTD LYKKLYHSDI CASKTVVSLR 240

CIECGVAGKT MRQSRIVGGS SASPGDWPWQ VSLHVQGTHV CGGSIITPEW IVTAAHCVEE 300

PLNNPRYWTV FAGVLRQSFM FYGHGYRVGK VISHPSYDSK TKNNDIALMK LQTPLTFSDK 360

VKPVCLPNPG MMLEPNQSCW ISGWGATHEK GKTSDELNAV MVPLIEPWRC NSKYVYNSLV 420

TPAMICAGYL RGGTDSCQGD SGGPLVTLKS RIWWLIGDTS WGSGCAKANR PGVYGNVTVF 480

TDWIYRQMRA NS 492

SEQ ID NO: 9: Heavy chain variable region of 752_1 antibody

QVQLQQSGAE LARPGASVKL SCKASGYTFT SCGISWVKQR TGQGLEWIGE IYPRGGNTYY 60

NEKFKGKATL TADKSSSTAY MELRSLTSED SAVYFCAREN GNYDPLFAYW GQGTLVTVSA 120

SEQ ID NO: 10: Light chain variable region of 752_1 antibody

DIQMTQSPSS LSASLGGKVT ITCKASQDIN KYIAWYQHKP GKGPRLLIHY TSTLQPGIPS 60

RFSGSGSGRD YSFSIYNLEP EDIATYYCLQ YYNLWTFGGG TKLEIK 106

SEQ ID NO: 11: Heavy chain CDR1 SCGIS of 752_1 antibody
SEQ ID NO: 12: Heavy chain CDR2 EIYPRGGNTY YNEKFKG of 752_1 antibody
SEQ ID NO: 13: Heavy chain CDR3 ENGNYDPLFA Y of 752_1 antibody
SEQ ID NO: 14: Light chain CDR1 KASQDINKYI A of 752_1 antibody
SEQ ID NO: 15: Light chain CDR2 YTSTLQP of 752_1 antibody
SEQ ID NO: 16: Light chain CDR3 LQYYNLWT of 752_1 antibody
SEQ ID NO: 17: Heavy chain variable region of 1864_10 antibody

QVQLQQSGPA LVKPGASLKI SCEASGYTFT YYSINWVKQR PGQGLEWIGW IFPGSGSTYY 60

NEKFKGKATL TIDKSSSTAY MLLSSLTSED SAVYFCARSD YGSAYGYFDV WGTGTTVTVS 120

S 121

SEQ ID NO: 18: Light chain variable region of 1864_10 antibody

DIVLTQSPAS LAVSLGQRAT ISCRASKSVS TSGYSYMHWY QQKPGQPPKL LIYLASNLES 60

GVPARFSGSG SGTDFTLNIH PVEEEDAATY YCQHSRDLPL TFGAGTKLEL K 111

SEQ ID NO: 19: Heavy chain CDR1 YYSIN of 1864_10 antibody
SEQ ID NO: 20: Heavy chain CDR2 WIFPGSGSTY YNEKFKG of 1864_10 antibody
SEQ ID NO: 21: Heavy chain CDR3 SDYGSAYGYF DV of 1864_10 antibody
SEQ ID NO: 22: Light chain CDR1 RASKSVSTSG YSYMH of 1864_10 antibody
SEQ ID NO: 23: Light chain CDR2 LASNLES of 1864_10 antibody
SEQ ID NO: 24: Light chain CDR3 QHSRDLPLT of 1864_10 antibody
SEQ ID NO: 25: Heavy chain variable region of 2020_12 antibody

QVQLQQPGAE LAKPGASVKL SCKASGYTFT SYWMHWVKQR PGQGLEWIGL IPPNSGSSNY 60

NEKFKSKATL TVDKSSRTAY MQLSSLTSED SAVYYCARWD YGSSYGYFDV WGTGTTVTVS 120

S                       121

SEQ ID NO: 26: Light chain variable region of 2020_12 antibody

DIVLTQSPAS LAVSLGQRAT ISCRASESVS TSGYSYMHWY QQKPGQPPKL LIYLASNLES 60

GVPVRFSGSG SGTDFTLNIH PVEEEDAATY YCQHSRELPP TFGAGTKLEL K      111

SEQ ID NO: 27: Heavy chain CDR1 SYWMH of 2020_12 antibody
SEQ ID NO: 28: Heavy chain CDR2 LIPPNSGSSN YNEKFKS of 2020_12 antibody
SEQ ID NO: 29: Heavy chain CDR3 WDYGSSYGYF DV of 2020_12 antibody
SEQ ID NO: 30: Light chain CDR1 RASESVSTSG YSYMH of 2020_12 antibody
SEQ ID NO: 31: Light chain CDR2 LASNLES of 2020_12 antibody
SEQ ID NO: 32: Light chain CDR3 QHSRELPPT of 2020_12 antibody
SEQ ID NO: 33: Heavy chain variable region of 2228_15 antibody

QVQLQQSGPE LVKPGTSVKI SCKASGYTFT DYYINWVKQR PGQGLEWIGW IFPGSGSSYY 60

NAIFKGKATL TVDTSSNTAH MSLSSLTSDD SAVYFCARGD FGNFGGFFTY WGQGTLVTVS 120

A                       121

SEQ ID NO: 34: Light chain variable region of 2228_15 antibody

DVVVTQSPAS LAVSLGQRAT ISCRASKSVS TSGYSFMHWY QQKPGQPPKL LIYLASNLES 60

GVPARFSGSG SGTDFILNIH PVEEEDAATY YCQHSRELPL TFGAGTKLEL K      111

SEQ ID NO: 35: Heavy chain CDR1 DYYIN of 2228_15 antibody
SEQ ID NO: 36: Heavy chain CDR2 WIFPGSGSSY YNAIFKG of 2228_15 antibody
SEQ ID NO: 37: Heavy chain CDR3 GDFGNFGGFF TY of 2228_15 antibody
SEQ ID NO: 38: Light chain CDR1 RASKSVSTSG YSFMH of 2228_15 antibody
SEQ ID NO: 39: Light chain CDR2 LASNLES of 2228_15 antibody
SEQ ID NO: 40: Light chain CDR3 QHSRELPLT of 2228_15 antibody
SEQ ID NO: 41: TMPRSS2 portion peptide AKAYRPGVYG NVMVFTDWIY
SEQ ID NO: 42: TMPRSS2 portion peptide AYRPGVYGNV MVFTDWIYRQ
SEQ ID NO: 43: TMPRSS2 portion peptide RPGVYGNVMV FTDWIYRQMR
SEQ ID NO: 44: TMPRSS2 portion peptide GVYGNVMVFT DWIYRQMRAD
SEQ ID NO: 45: TMPRSS2 portion peptide VYGNVMVFTD WIYRQMRADG
SEQ ID NO: 46: TMPRSS2 portion peptide DSGGPLVTSK NNIWWLIGDT
SEQ ID NO: 47: TMPRSS2 portion peptide GGPLVTSKNN IWWLIGDTSW
SEQ ID NO: 48: TMPRSS2 portion peptide PLVTSKNNIW WLIGDTSWGS
SEQ ID NO: 49: TMPRSS2 portion peptide VTSKNNIWWL IGDTSWGSGC
SEQ ID NO: 50: TMPRSS2 portion peptide SKNNIWWLIG DTSWGSGCAK
SEQ ID NO: 51: TMPRSS2 portion peptide NNIWWLIGDT SWGSGCAKAY
SEQ ID NO: 52: TMPRSS2 portion peptide VLNAAKVLLI ETQRCNSRYV
SEQ ID NO: 53: TMPRSS2 portion peptide NAAKVLLIET QRCNSRYVYD
SEQ ID NO: 54: TMPRSS2 portion peptide AKVLLIETQR CNSRYVYDNL
SEQ ID NO: 55: TMPRSS2 portion peptide VLLIETQRCN SRYVYDNLIT
SEQ ID NO: 56: TMPRSS2 portion peptide WTAFAGILRQ SFMFYGAGYQ
SEQ ID NO: 57: TMPRSS2 portion peptide AFAGILRQSF MFYGAGYQVE
SEQ ID NO: 58: TMPRSS2 portion peptide AGILRQSFMF YGAGYQVEKV
SEQ ID NO: 59: TMPRSS2 portion peptide ILRQSFMFYG AGYQVEKVIS
SEQ ID NO: 60: TMPRSS2 portion peptide RQSFMFYGAG YQVEKVISHP
SEQ ID NO: 61: TMPRSS2 portion peptide SFMFYGAGYQ VEKVISHPNY

SEQ ID NO: 62: TMPRSS2 portion peptide MFYGAGYQVE KVISHPNYDS
SEQ ID NO: 63: TMPRSS2 portion peptide YGAGYQVEKV ISHPNYDSKT
SEQ ID NO: 64: TMPRSS2 portion peptide WIVTAAHCVE KPLNNPWHWT
SEQ ID NO: 65: TMPRSS2 portion peptide VTAAHCVEKP LNNPWHWTAF
SEQ ID NO: 66: TMPRSS2 portion peptide AAHCVEKPLN NPWHWTAFAG
SEQ ID NO: 67: TMPRSS2 portion peptide HCVEKPLNNP WHWTAFAGIL
SEQ ID NO: 68: TMPRSS2 portion peptide VEKPLNNPWH WTAFAGILRQ
SEQ ID NO: 69: TMPRSS2 portion peptide KPLNNPWHWT AFAGILRQSF
SEQ ID NO: 70: TMPRSS2 portion peptide KAVVSLRCIA CGVNLNSSRQ
SEQ ID NO: 71: TMPRSS2 portion peptide VVSLRCIACG VNLNSSRQSR
SEQ ID NO: 72: TMPRSS2 portion peptide SLRCIACGVN LNSSRQSRIV
SEQ ID NO: 73: TMPRSS2 portion peptide RCIACGVNLN SSRQSRIVGG
SEQ ID NO: 74: TMPRSS2 portion peptide IACGVNLNSS RQSRIVGGES
SEQ ID NO: 75: TMPRSS2 portion peptide WNENYGRAAC RDMGYKNNFY
SEQ ID NO: 76: TMPRSS2 portion peptide RLYGPNFILQ VYSSQRKSWH
SEQ ID NO: 77: TMPRSS2 portion peptide YGPNFILQVY SSQRKSWHPV
SEQ ID NO: 78: TMPRSS2 portion peptide PNFILQVYSS QRKSWHPVCQ
SEQ ID NO: 79: TMPRSS2 portion peptide FILQVYSSQR KSWHPVCQDD
SEQ ID NO: 80: TMPRSS2 portion peptide LQVYSSQRKS WHPVCQDDWN
SEQ ID NO: 81: TMPRSS2 portion peptide VYSSQRKSWH PVCQDDWNEN
SEQ ID NO: 82: TMPRSS2 portion peptide SSQRKSWHPV CQDDWNENYG
SEQ ID NO: 83: TMPRSS2 portion peptide QRKSWHPVCQ DDWNENYGRA
SEQ ID NO: 84: TMPRSS2 portion peptide KSWHPVCQDD WNENYGRAAC
SEQ ID NO: 85: TMPRSS2 portion peptide GVYGNVMVFT DWIY
SEQ ID NO: 86: TMPRSS2 portion peptide PLVTSKNNIW WLIGDTSW
SEQ ID NO: 87: TMPRSS2 portion peptide VLLIETQRCN SRYV
SEQ ID NO: 88: TMPRSS2 portion peptide MFYGAGYQVE KV
SEQ ID NO: 89: TMPRSS2 portion peptide VTAAHCVEKP LNNPWHWT
SEQ ID NO: 90: TMPRSS2 portion peptide KPLNNPWHWT AFAGILRQSF
SEQ ID NO: 91: TMPRSS2 portion peptide IACGVNLNSS RQSR
SEQ ID NO: 92: TMPRSS2 portion peptide WNENYGRAAC RDMGYKNNFY
SEQ ID NO: 93: TMPRSS2 portion peptide PNFILQVYSS QRKSWHPVCQ DD
SEQ ID NO: 94: TMPRSS2 portion peptide WSEGYGRAAC QDMGYRNSFY
SEQ ID NO: 95: TMPRSS2 portion peptide VTAAHCVEEP LNNPRHWT
SEQ ID NO: 96: TMPRSS2 portion peptide RQSFMF
SEQ ID NO: 97: TMPRSS2 portion peptide SQSLMF
SEQ ID NO: 98: Heavy chain variable region of 1831_15 antibody

QVQLQQPGAE LVKPGASVKL SCKASGYSFT TYWMHWVRQR PGQGLEWIGI IHPNSGTTNY

NEKFKSKASL TVDKSSSTAY MQLSSLTSED SAVYYCARLL WDYFDYWGQG TTLTVSS

SEQ ID NO: 99: Light chain variable region of 1831_15 antibody

DIVMSQSPSS LAVSAGEKVT MSCKSSQSLL NSRTRKNFLA WYQQKPGQSP KLLIYWASTR

ESGVPDRFTG SGSGTDFTLT ISSVQAEDLA VYYCKQSYNL YTFGGGTKLE IK

SEQ ID NO: 100: Heavy chain CDR1 GYSFTTYW of 1831_15 antibody
SEQ ID NO: 101: Heavy chain CDR2 IHPNSGTT of 1831_15 antibody
SEQ ID NO: 102: Heavy chain CDR3 ARLLWDYFDY of 1831_15 antibody
SEQ ID NO: 103: Light chain CDR1 QSLLNSRTRK NF of 1831_15 antibody
SEQ ID NO: 104: Light chain CDR2 WAS of 1831_15 antibody
SEQ ID NO: 105: Light chain CDR3 KQSYNLYT of 1831_15 antibody
SEQ ID NO: 106: Heavy chain variable region of 2113_16 antibody

EVQLQQSGPE LVKPGASVKI SCKASGYTFT DYYMNWVKRR HGKSLEWIGN INPNNGGTSY0

NQKFKGKATL TVDKSSSTAY MELRSLTSED SVVYYCAREG DLFPFAYWGQ GTLVTVSA

SEQ ID NO: 107: Light chain variable region of 2113_16 antibody

QAVVTQESAL TTSPGETVTL TCRSSIGTVT TSNFANWVQE KPDHLFTGLI GASNNRAPGV

PARFSGSLIG DKAALTITGA QTEDEAIYFC ALWFSNHWVF GGGTKLTVL

SEQ ID NO: 108: Heavy chain CDR1 GYTFTDYY of 2113_16 antibody
SEQ ID NO: 109: Heavy chain CDR2 INPNNGGT of 2113_16 antibody
SEQ ID NO: 110: Heavy chain CDR3 AREGDLFPFA Y of 2113_16 antibody
SEQ ID NO: 111: Light chain CDR1 IGTVTTSNF of 2113_16 antibody
SEQ ID NO: 112: Light chain CDR2 ASN of 2113_16 antibody
SEQ ID NO: 113: Light chain CDR3 ALWFSNHWV of 2113_16 antibody
SEQ ID NO: 114: 752_hVL-1

DIQMTQSPSS LSASVGDRVT ITCKASQDIN KYIAWYQQKP GKAPKLLIYY TSTLQPGVPS

RFSGSGSGTD FTFTISSLQP EDIATYYCLQ YYNLWTFGGG TKVEIK

SEQ ID NO: 115: 752_hVL-1 FR1 DIQMTQSPSS LSASVGDRVT ITC
SEQ ID NO: 116: 752_hVL-1 FR2 WYQQKPGKAP KLLIY
SEQ ID NO: 117: 752_hVL-1 FR3 GVPSRFSGSG SGTDFTFTIS SLQPEDIATY YC SEQ ID NO: 118: 752_hVL-1 FR4 FGGGTKVEIK
SEQ ID NO: 119: 752_hVL-2

DIQMTQSPSS LSASVGDRVT ITCKASQDIN KYIAWYQQKP GKAPKLLIHY TSTLQPGVPS

RFSGSGSGTD FTFTISSLQP EDIATYYCLQ YYNLWTFGGG TKVEIK

SEQ ID NO: 120: 752_hVL-2 FR1 DIQMTQSPSS LSASVGDRVT ITC
SEQ ID NO: 121: 752_hVL-2 FR2 WYQQKPGKAP KLLIH
SEQ ID NO: 122: 752_hVL-2 FR3 GVPSRFSGSG SGTDFTFTIS SLQPEDIATY YC
SEQ ID NO: 123: 752_hVL-2 FR4 FGGGTKVEIK
SEQ ID NO: 124: 752_hVH-1

QVQLVQSGAE VKKPGASVKV SCKASGYTFT SCGISWVRQA PGQGLEWMGE IYPRGGNTYY

NEKFKGRVTM TTDTSTSTAY MELRSLRSDD TAVYYCAREN GNYDPLFAYW GQGTLVTVSS

SEQ ID NO: 125: 752_hVH-1 FR1 QVQLVQSGAE VKKPGASVKV SCKASGYTFT
SEQ ID NO: 126: 752_hVH-1 FR2 WVRQAPGQGL EWMG
SEQ ID NO: 127: 752_hVH-1 FR3 RVTMTTDTST STAYMELRSL RSDDTAVYYC AR
SEQ ID NO: 128: 752_hVH-1 FR4 WGQGTLVTVS S
SEQ ID NO: 129: 752_hVH-2

QVQLQQSGTG LARPGASVKL SCKASGYTFT SCGISWVTQR AGQGLEWIGE IYPRGGNTYY

NEKFKGKATL TADKSSSSAY MELRGLTAED SAVYFCAREN GNYDPLFAYW GQGTTLTVSS

SEQ ID NO: 130: 752_hVH-2 FR1 QVQLQQSGTG LARPGASVKL SCKASGYTFT
SEQ ID NO: 131: 752_hVH-2 FR2 WVTQRAGQGL EWIG
SEQ ID NO: 132: 752_hVH-2 FR3 KATLTADKSS SSAYMELRGL TAEDSAVYFC AR
SEQ ID NO: 133: 752_hVH-2 FR4 WGQGTTLTVS S
SEQ ID NO: 134: 2228_hVL-1

DVVMTQSPLS LPVTLGQPAS ISCRASKSVS TSGYSFMHWF QQRPGQSPRR LIYLASNLES

GVPDRFSGSG SGTDFTLKIS RVEAEDVGVY YCQHSRELPL TFGGGTKVEI K

SEQ ID NO: 135: 2228_hVL-1 FR1 DVVMTQSPLS LPVTLGQPAS ISC
SEQ ID NO: 136: 2228_hVL-1 FR2 WFQQRPGQSP RRLIY

SEQ ID NO: 137: 2228_hVL-1 FR3 GVPDRFSGSG SGTDFTLKIS RVEAEDVGVY YC
SEQ ID NO: 138: 2228_hVL-1 FR4 FGGGTKVEIK
SEQ ID NO: 139: 2228_hVL-2

DIVMTQSPDS LAVSLGERAT INCRASKSVS TSGYSFMHWY QQKPGQPPKL LIYLASNLES

GVPDRFSGSG SGTDFTLTIR PLQAEDVAVY YCQHSRELPL TFGQGTKLEI K

SEQ ID NO: 140: 2228_hVL-2 FR1 DIVMTQSPDS LAVSLGERAT INC
SEQ ID NO: 141: 2228_hVL-2 FR2 WYQQKPGQPP KLLIY
SEQ ID NO: 142: 2228_hVL-2 FR3 GVPDRFSGSG SGTDFTLTIR PLQAEDVAVY YC
SEQ ID NO: 143: 2228_hVL-2 FR4 FGQGTKLEIK
SEQ ID NO: 144: 2228_hVH-1

QVQLVQSGAE VKKPGASVKV SCKASGYTFT DYYINWVRQA PGQGLEWMGW IFPGSGSSYY

NAIFKGRVTM TRDTSISTAY MELSRLRSDD TAVYYCARGD FGNFGGFFTY WGQGTLVTVS

S

SEQ ID NO: 145: 2228_hVH-1 FR1 QVQLVQSGAE VKKPGASVKV SCKASGYTFT
SEQ ID NO: 146: 2228_hVH-1 FR2 WVRQAPGQGL EWMG
SEQ ID NO: 147: 2228_hVH-1 FR3 RVTMTRDTSI STAYMELSRL RSDDTAVYYC AR
SEQ ID NO: 148: 2228_hVH-1 FR4 WGQGTLVTVS S
SEQ ID NO: 149: 2228_hVH-2

EIQLVQSGAE VKKPGSSVKV SCKASGYTFT DYYINWMRQA PGQGLEWIGW IFPGSGSSYY

NAIFKGRATL TVDTSTNTAY MELSSLRSED TAFYFCARGD FGNFGGFFTY WGQGTLVTVS

S

SEQ ID NO: 150: 2228_hVH-2 FR1 EIQLVQSGAE VKKPGSSVKV SCKASGYTFT
SEQ ID NO: 151: 2228_hVH-2 FR2 WMRQAPGQGL EWIG
SEQ ID NO: 152: 2228_hVH-2 FR3 RATLTVDTST NTAYMELSSL RSEDTAFYFC AR
SEQ ID NO: 153: 2228_hVH-2 FR4 WGQGTLVTVS S

**Claims**

1. An antibody or an antigen-binding fragment thereof that binds to an extracellular domain of TMPRSS2, capable of inhibiting coronavirus infection of cells.

2. The antibody or an antigen-binding fragment thereof according to claim 1, capable of binding to (i) one or more extracellular domains of TMPRSS2 selected from the group consisting of mice, green monkeys, cats, hamsters, and macaque monkeys, and (ii) an extracellular domain of human TMPRSS2.

3. The antibody or an antigen-binding fragment thereof according to claim 1 or 2, wherein a binding affinity (KD) to human TMPRSS2 is 10-8 M or less.

4. The antibody or an antigen-binding fragment thereof according to any one of claims 1 to 3, wherein IC90 for inhibition of the coronavirus infection of human cells is 10 $\mu$g/mL or less.

5. The antibody or an antigen-binding fragment thereof according to any one of claims 1 to 4, wherein the antibody binds to a peptide having the amino acid sequence consisting of the amino acid sequence selected from the group consisting of the amino acid sequence set forth in SEQ ID NO: 75 (WNENYGRAACRDMGYKNNFY), and the amino acid sequence set forth in SEQ ID NO: 89 (VTAAHCVEKPLNNPWHWT).

6. The antibody or an antigen-binding fragment thereof according to any one of claims 1 to 5, wherein the antibody binds to human TMPRSS2 having the amino acid sequence set forth in SEQ ID NO: 5, and wherein:

(α) the antibody does not significantly bind to chimeric TMPRSS2 having the amino acid sequence wherein the amino acid sequence set forth in SEQ ID NO: 75 (WNENYGRAACRDMGYKNNFY) of the human TMPRSS2 is substituted for a corresponding sequence of a cat (i.e., the amino acid sequence set forth in SEQ ID NO: 94 WSEGYGRAACQDMGYRNSFY).

(β) the antibody does not significantly bind to chimeric TMPRSS2 having the amino acid sequence wherein the amino acid sequence set forth in SEQ ID NO: 89 (VTAAHCVEKPLNNPWHWT) of the human TMPRSS2 is substituted for a corresponding sequence of a cat (i.e., the amino acid sequence set forth in SEQ ID NO: 95: VTAAHCVEEPLNNPRHWT); or

(γ) the antibody does not significantly bind to chimeric TMPRSS2 having the amino acid sequence wherein the amino acid sequence set forth in SEQ ID NO: 96 (RQSFMF) of the human TMPRSS2 is substituted for a corresponding sequence of a hamster (i.e., the amino acid sequence set forth in SEQ ID NO: 97: SQSLMF).

7. The antibody or an antigen-binding fragment thereof according to any one of claims 1 to 6, wherein the antibody binds to human TMPRSS2 having the amino acid sequence set forth in SEQ ID NO: 5, and wherein:

(A) the antibody does not significantly bind to a human TMPRSS2 variant having a point mutation at N179A; or
(B) the antibody does not significantly bind to a human TMPRSS2 variant having a point mutation at W306A.

8. The antibody or an antigen-binding fragment thereof according to any one of claims 1 to 5, being:

(1) an antibody or an antigen-binding fragment thereof, comprising a heavy chain variable region that has HCDR1 having the amino acid sequence set forth in SEQ ID NO: 11,

HCDR2 having the amino acid sequence set forth in SEQ ID NO: 12, and
HCDR3 having the amino acid sequence set forth in SEQ ID NO: 13,
a light chain variable region that has LCDR1 having the amino acid sequence set forth in SEQ ID NO: 14,
LCDR2 having the amino acid sequence set forth in SEQ ID NO: 15, and
LCDR3 having the amino acid sequence set forth in SEQ ID NO: 16.

(2) an antibody or an antigen-binding fragment thereof, comprising a heavy chain variable region that has HCDR1 having the amino acid sequence set forth in SEQ ID NO: 19,

HCDR2 having the amino acid sequence set forth in SEQ ID NO: 20, and
HCDR3 having the amino acid sequence set forth in SEQ ID NO: 21,
a light chain variable region that has LCDR1 having the amino acid sequence set forth in SEQ ID NO: 22,
LCDR2 having the amino acid sequence set forth in SEQ ID NO: 23, and
LCDR3 having the amino acid sequence set forth in SEQ ID NO: 24.

(3) an antibody or an antigen-binding fragment thereof, comprising a heavy chain variable region that has HCDR1 having the amino acid sequence set forth in SEQ ID NO: 27,

HCDR2 having the amino acid sequence set forth in SEQ ID NO: 28, and
HCDR3 having the amino acid sequence set forth in SEQ ID NO: 29,
a light chain variable region that has LCDR1 having the amino acid sequence set forth in SEQ ID NO: 30,
LCDR2 having the amino acid sequence set forth in SEQ ID NO: 31, and
LCDR3 having the amino acid sequence set forth in SEQ ID NO: 32.

(4) an antibody or an antigen-binding fragment thereof, comprising a heavy chain variable region that has HCDR1 having the amino acid sequence set forth in SEQ ID NO: 35,

HCDR2 having the amino acid sequence set forth in SEQ ID NO: 36, and
HCDR3 having the amino acid sequence set forth in SEQ ID NO: 37,
a light chain variable region that has LCDR1 having the amino acid sequence set forth in SEQ ID NO: 38,
LCDR2 having the amino acid sequence set forth in SEQ ID NO: 39, and
LCDR3 having the amino acid sequence set forth in SEQ ID NO: 40.

(5) an antibody or an antigen-binding fragment thereof, comprising a heavy chain variable region that has HCDR1 having the amino acid sequence set forth in SEQ ID NO: 100,

HCDR2 having the amino acid sequence set forth in SEQ ID NO: 101, and
HCDR3 having the amino acid sequence set forth in SEQ ID NO: 102,
a light chain variable region that has LCDR1 having the amino acid sequence set forth in SEQ ID NO: 103,
LCDR2 having the amino acid sequence set forth in SEQ ID NO: 104, and
LCDR3 having the amino acid sequence set forth in SEQ ID NO: 105.

(6) an antibody or an antigen-binding fragment thereof, comprising a heavy chain variable region that has HCDR1 having the amino acid sequence set forth in SEQ ID NO: 108,

HCDR2 having the amino acid sequence set forth in SEQ ID NO: 109, and
HCDR3 having the amino acid sequence set forth in SEQ ID NO: 110,
a light chain variable region that has LCDR1 having the amino acid sequence set forth in SEQ ID NO: 111,
LCDR2 having the amino acid sequence set forth in SEQ ID NO: 112, and
LCDR3 having the amino acid sequence set forth in SEQ ID NO: 113.

(7) an antibody or an antigen-binding fragment thereof, competing with the antibody according to any of (1) to (6) above for binding with TMPRSS2; or
(8) an antibody or an antigen-binding fragment thereof, comprising the amino acid sequence having one or several amino acid mutations in the CDR sequence of the antibody according to any of (1) to (6) above; or
(9) a human chimeric antibody or a humanized antibody of the antibody according to any of (1) to (8) above.

9. A composition comprising the antibody or an antigen-binding fragment thereof according to any one of claims 1 to 8.

10. The composition according to claim 9, for use in preventing a subject in need thereof from being infected with coronavirus.

11. A pharmaceutical composition according to claim 9, for use in administering to a subject with coronavirus.

12. The composition according to claim 9, for use in treating coronavirus infection in a subject infected with a beta coronavirus.

# FIG. 1

Novel coronavirus
SARS-CoV-2

S protein

Genome RNA

Anti-TMPRSS2
monoclonal antibody

ACE2
receptor

Airway cell

Infection

TMPRSS2
proteolytic activation of S protein

# FIG. 2

# FIG. 3A

Competitive antibody concentration (µg/ml)

# FIG. 3B

Competitive antibody concentration (µg/ml)

# FIG. 3C

# FIG. 3D

# FIG. 4

# FIG. 5A

SPD

### 1: Futhan binding site

| Name | Sequence | 752_1 | 2228_15 | 1864_10 | 2020_12 | | |
|------|----------|-------|---------|---------|---------|--|--|
| Peptide_181 | AKAYRPGVYGNVMVFTDWIY | O | | | O | Futhan | SEQ ID NO:41 |
| Peptide_182 | AYRPGVYGNVMVFTDWIYRQ | O | | | O | Futhan | SEQ ID NO:42 |
| Peptide_183 | RPGVYGNVMVFTDWIYRQMR | O | O | | O | Futhan | SEQ ID NO:43 |
| Peptide_184 | GVYGNVMVFTDWIYRQMRAD | O | | | O | | SEQ ID NO:44 |
| Peptide_185 | VYGNVMVFTDWIYRQMRADG | O | | | | | SEQ ID NO:45 |

SEQ ID NO: 85 : **GVYGNVMVFTDWIY**

### 2 : Active center of Futhan binding site

| Name | Sequence | 752_1 | 2228_15 | 1864_10 | 2020_12 | | |
|------|----------|-------|---------|---------|---------|--|--|
| Peptide_168 | DSGGPLVTSKNNIWWLIGDT | O | | | O | Active center/Futhan | SEQ ID NO: 46 |
| Peptide_169 | GGPLVTSKNNIWWLIGDTSW | O | | | | | SEQ ID NO: 47 |
| Peptide_170 | PLVTSKNNIWWLIGDTSWGS | O | | | | | SEQ ID NO: 48 |
| Peptide_171 | VTSKNNIWWLIGDTSWGSGC | O | | | | Futhan | SEQ ID NO: 49 |
| Peptide_172 | SKNNIWWLIGDTSWGSGCAK | O | | | | Futhan | SEQ ID NO: 50 |
| Peptide_173 | NNIWWLIGDTSWGSGCAKAY | O | | | | Futhan | SEQ ID NO: 51 |

SEQ ID NO: 86 : **PLVTSKNNIWWLIGDTSW**

### 3

| Name | Sequence | 752_1 | 2228_15 | 1864_10 | 2020_12 | | |
|------|----------|-------|---------|---------|---------|--|--|
| Peptide_146 | VLNAAKVLLIETQRCNSRYV | O | O | | O | | SEQ ID NO: 52 |
| Peptide_147 | NAAKVLLIETQRCNSRYVYD | | O | | O | | SEQ ID NO: 53 |
| Peptide_148 | AKVLLIETQRCNSRYVYDNL | O | O | | O | | SEQ ID NO: 54 |
| Peptide_149 | VLLIETQRCNSRYVYDNLIT | O | O | | O | 1258 chimera | SEQ ID NO: 55 |

SEQ ID NO: 87 : **VLLIETQRCNSRYV**

### 4

SPD

| Name | Sequence | 752_1 | 2228_15 | 1864_10 | 2020_12 | | |
|------|----------|-------|---------|---------|---------|--|--|
| Peptide_102 | WTAFAGILRQSFMFYGAGYQ | | | O | | Chimera (1831) | SEQ ID NO:56 |
| Peptide_103 | AFAGILRQSFMFYGAGYQVE | | | O | | Chimera (1831) | SEQ ID NO:57 |
| Peptide_104 | AGILRQSFMFYGAGYQVEKV | | | O | | Chimera (1831) | SEQ ID NO:58 |
| Peptide_105 | ILRQSFMFYGAGYQVEKVIS | O | O | O | O | | SEQ ID NO:59 |
| Peptide_106 | RQSFMFYGAGYQVEKVISHP | O | O | | O | | SEQ ID NO:60 |
| Peptide_107 | SFMFYGAGYQVEKVISHPNY | O | O | | O | | SEQ ID NO:61 |
| Peptide_108 | MFYGAGYQVEKVISHPNYDS | | | | O | | SEQ ID NO:62 |
| Peptide_109 | YGAGYQVEKVISHPNYDSKT | | | | O | | SEQ ID NO:63 |

SEQ ID NO: 88 : **MFYGAGYQVEKV**

### 5, 6

| Name | Sequence | 752_1 | 2228_15 | 1864_10 | 2020_12 | | |
|------|----------|-------|---------|---------|---------|--|--|
| Peptide_093 | WIVTAAHCVEKPLNNPWHWT | O | | O | | Active center/Chimera (1831) | SEQ ID NO:64 |
| Peptide_094 | VTAAHCVEKPLNNPWHWTAF | O | | O | O | Active center/Chimera (1831) | SEQ ID NO:65 |
| Peptide_095 | AAHCVEKPLNNPWHWTAFAG | O | | | | Active center/Chimera (1831) | SEQ ID NO:66 |
| Peptide_096 | HCVEKPLNNPWHWTAFAGIL | | | | | Active center/Chimera (1831) | SEQ ID NO:67 |
| Peptide_097 | VEKPLNNPWHWTAFAGILRQ | | | | | Chimera (1831) | SEQ ID NO:68 |
| Peptide_098 | KPLNNPWHWTAFAGILRQSF | | O | O | O | Chimera (1831) | SEQ ID NO:69 |

SEQ ID NO: 89 : **VTAAHCVEKPLNNPWHWT**

SEQ ID NO: 90 : **KPLNNPWHWTAFAGILRQSF**

# FIG. 5B

7: Self-cleavage site

| Name | Sequence | 752_1 | 2228_15 | 1864_10 | 2020_12 | | |
|---|---|---|---|---|---|---|---|
| Peptide_065 | KAVVSLRCIACGVNLNSSRQ | ○ | | | | Self-cleavage site | SEQ ID NO: 70 |
| Peptide_066 | VVSLRCIACGVNLNSSRQSR | ○ | | | ○ | Self-cleavage site | SEQ ID NO: 71 |
| Peptide_067 | SLRCIACGVNLNSSRQSRIV | ○ | | | ○ | Self-cleavage site | SEQ ID NO: 72 |
| Peptide_068 | RCIACGVNLNSSRQSRIVGG | ○ | | | ○ | Self-cleavage site | SEQ ID NO: 73 |
| Peptide_069 | IACGVNLNSSRQSRIVGGES | ○ | | | | Self-cleavage site | SEQ ID NO: 74 |

SEQ ID NO: 91 : **IACGVNLNSSRQSR**

8: Common to all TMPRSS2

| Name | Sequence | 752_1 | 2228_15 | 1864_10 | 2020_12 | |
|---|---|---|---|---|---|---|
| Peptide_036 | WNENYGRAACRDMGYKNNFY | ○ | | | ○ | SEQ ID NO: 75 |

SEQ ID NO: 92 : **WNENYGRAACRDMGYKNNFY**

9

| Name | Sequence | 752_1 | 2228_15 | 1864_10 | 2020_12 | |
|---|---|---|---|---|---|---|
| Peptide_023 | RLYGPNFILQVYSSQRKSWH | ○ | ○ | | ○ | SEQ ID NO: 76 |
| Peptide_024 | YGPNFILQVYSSQRKSWHPV | ○ | | | ○ | SEQ ID NO: 77 |
| Peptide_025 | PNFILQVYSSQRKSWHPVCQ | ○ | | | ○ | SEQ ID NO: 78 |
| Peptide_026 | FILQVYSSQRKSWHPVCQDD | ○ | | | ○ | SEQ ID NO: 79 |
| Peptide_027 | LQVYSSQRKSWHPVCQDDWN | ○ | | | | SEQ ID NO: 80 |
| Peptide_028 | VYSSQRKSWHPVCQDDWNEN | ○ | | | | SEQ ID NO: 81 |
| Peptide_029 | SSQRKSWHPVCQDDWNENYG | ○ | | | | SEQ ID NO: 82 |
| Peptide_030 | QRKSWHPVCQDDWNENYGRA | ○ | | | | SEQ ID NO: 83 |
| Peptide_031 | KSWHPVCQDDWNENYGRAAC | ○ | | | | SEQ ID NO: 84 |

SEQ ID NO: 93 : PNF**ILQVYSSQRKSWH**PVCQDD

# FIG. 6

Side view    752_1     2228_15     2020_12    Nafamostat

Epitope-predicted amino acid sequence

| 1. NNIWWLIGDT |
| 2. AAHCVEKPLNNPWHWT |
| 3. IACGVNLNSSRQSR |
| 4. VYSSQRKSWHPVCQ |

| 5. GVYGNVMVFTDWIYRQ |

| 6. GVYGNVMVFTDWIY |
| 7. VLLIETQRCNSRYV |
| 8. MFYGAGYQVEKV |

Dye antibody    mIgG     1831_15     2020_12     1864_10     2228_15     752_1

**TMPRSS2-wild type**

    176 to 195 a.a.

      WNENYGRAACRDMGYKNNFY

    292-309 a.a.

      VTAAHCVEKPLNNPWHWT

**TMPRSS2 chimera**
**human/cat**

   176 to 195 a.a.

(2) cat WSEGYGRAACQDMGYRNSFY

   292-309 a.a.

(4) cat VTAAHCVEEPLNNPRHWT

GFP / APC

# FIG. 7A

176 to 195 a.a.

Dye antibody

TMPRSS2- wild type

WNENYGRAACRDMGYKNNFY

Alanine substitution

N179A

WNEAYGRAACRDMGYKNNFY

TMPRSS2 chimera
human/cat
(2)- cat-chimera

WSEGYGRAACQDMGYRNSFY

GFP
APC

mIgG    1831_15    2020_12    1864_10    2228_15    752_1

FIG. 7B

EP 4 509 529 A1

51

# FIG. 7C

# FIG. 7D

# FIG. 8

Fab752                                            Fab2228

Amino acid residues    N177, N179, R183, R186, I221                R316, F319, F321

# FIG. 9

EP 4 509 529 A1

# FIG. 10A

CLUSTAL O(1.2.4) multiple sequence alignment

```
Golden-hamster       MALNSGSPPGIGPYYENHGFQSEHIYPPRPPVAPDVYNPYPPQNYPPPVPQYFPRVTTQA    60
mouse                MALNSGSPPGIGPCYENHGYQSEHICPPRPPVAPNGYNLYPAQYYPSPVPQYAPRITTQA    60
human                MALNSGSPPAIGPYYENHGYQPENPYPAQPTVVPTVYEVHPAQYYPSPVPQYAPRVLTQA    60
green-monkey         MALNSGSPPGVGPYYENHGYQPENPYPAQPTVAPNVYEVHPAQYYPSPVPQYIPSVLTHA    60
crab-eating-macaque  MALNSGSPPGVGPYYENHGYQPENPYPAQPTVAPNVYEVHPAQYYPSPVPQYTPRVLTHA    60
Rhesus-macaque       MALNSGSPPGVGPYYENHGYQPENPYPAQPTVAPNVYEVHPAQYYPSPVPQYTPRVLTHA    60
cat                  MALNSGPPPGVGPFYENHGYQPEGLYPPRPAVVPGAYSVYPAPYYPPAVPQYAPRVLTHA    60
Ferret               MALNAGSPPGVGPYYENHGYQPESLYPAPPATVPSVYVAYPAAYYPPAVPQYTPRVLTQA    60
                     ****:* **.:** *****:* *   *  *  ..* *  :*   ** **** * : *:*

Golden-hamster       STTVTHTQPHSS-GKLCTSTSKTKKSLCFALSLGIVLVGAAVAAVLLWKFL-PGCSTSEM   118
mouse                STSVIHTHPKSS-GALCTS--KSKKSLCLALALGTVLTGAAVAAVLLWRFWDSNCSTSEM   117
human                SNPVVCTQPKSPSGTVCTS--KTKKALCITLTLGTFLVGAALAAGLLWKFMGSKCSNSGI   118
green-monkey         SNPAVRTQPKSPSGTVCTS--KTKKALCVTMTLGAVLVGAALAAGLLWKFMGSKCSDSGI   118
crab-eating-macaque  SNPAVCRQPKSPSGTVCTS--KTKKALCVTMTLGAVLVGAALAAGLLWKFMGSKCSDSGI   118
Rhesus-macaque       SNPAVCRQPKSPSGTVCTS--KTKKALCVTMTLGAVLVGAALAAGLLWKFMGSKCSDSGI   118
cat                  STPSSHTQPKSPSGTVCTS--KAKKVLCITFTLGAVLAGAVLAAVLLWKFMENKCLVSGI   118
Ferret               STPAVRTQPKSPSGTACTA--KAKKALCITISLGAVLAGAAVTAVLLWKFMENKCSVSGI   118
                     *.   :*:*  * **:  *:** **.::::** .*.**.::* ***:*   *  * :

Golden-hamster       ECMSSGTCISSSLWCDGTSHCPNGEDENRCVRLYGPSFTLQVYSSQRKAWYPVCQDDWND   178
mouse                ECGSSGTCISSSLWCDGVAHCPNGEDENRCVRLYGQSFILQVYSSQRKAWYPVCQDDWSE   177
human                ECDSSGTCINPSNWCDGVSHCPGGEDENRCVRLYGPNFILQVYSSQRKSWHPVCQDDWNE   178
green-monkey         ECDSSGTCISSSNWCDGVSHCPSGEDENRCVRLYGPNFILQVYSSQRKSWHPVCRDDWNE   178
crab-eating-macaque  ECDSSGTCISSSNWCDGVSHCPNGEDENRCVRLYGPNFILQVYSSQRKSWHPVCRDDWNE   178
Rhesus-macaque       ECDSSGTCISSSNWCDGVSHCPNGEDENRCVRLYGPNFILQVYSSQRKSWHPVCRDDWNE   178
cat                  ECGSSGTCVSPSHWCDGVLHCPSGEDENRCVRLYGPNFILQVYSSQRKSWHPVCQDDWSE   178
Ferret               ECGSSGTCISPSHWCDGVLHCPSGEDENRCVRLYGPNFILQVYSAQRKSWHPVCQDDWSD   178
                     ** *****:. * ****. ***.************ .* *****:***:*:***:***.:

Golden-hamster       SYGRAACKDMGYKNNFYYTQGIPDSSGATSFMKLNISAGNIDLYKKLYHSDSCSSRMVVS   238
mouse                SYGRAACKDMGYKNNFYSSQGIPDQSGATSFMKLNVSSGNVDLYKKLYHSDSCSSRMVVS   237
human                NYGRAACRDMGYKNNFYSSQGIVDDSGSTSFMKLNTSAGNVDIYKKLYHSDACSSKAVVS   238
green-monkey         NYARAACRDMGYQNRFYSSQGIADNSGATSFMKLNTSAGNVDIYKKLYHSDACSSKAVVS   238
crab-eating-macaque  NYARAACRDMGYKNSFYSSQGIVDNSGATSFMKLNTSAGNVDIYKKLYHSDACSSKAVVS   238
Rhesus-macaque       NYARAACRDMGYKNSFYSSQGIVDNSGATSFMKLNTSAGNVDIYKKLYHSDACSSKAVVS   238
cat                  GYGRAACQDMGYRNVNTSANHMDLYKKLYHSDVCSSKTVVS   238
Ferret               SYGRAACQDMGYRNSFYSSQGIVDDSGASSFMKLNTSAGNTDLYKKLYHSDICASKTVVS   238
                     .*.****:****:* ** ::*: *.**::***::* *:.: *:********* *:*: ***

Golden-hamster       LRCIQCGVRS-ATRQSRIVGGSNASPGDWPWQVSLHVQGVHVCGGSIITPEWIVTAAHCV   297
mouse                LRCIECGVRS-VKRQSRIVGGLNASPGDWPWQVSLHVQGVHVCGGSIITPEWIVTAAHCV   296
human                LRCIACGVNLNSSRQSRIVGGESALPGAWPWQVSLHVQNVHVCGGSIITPEWIVTAAHCV   298
green-monkey         LRCIACGVRSNLSRQSRIVGGQNALPGAWPWQVSLHVQNIHVCGGSIITPEWIVTAAHCV   298
crab-eating-macaque  LRCIACGVRSNLNRQSRIVGGQNALLGAWPWQVSLHVQNIHVCGGSIITPEWIVTAAHCV   298
Rhesus-macaque       LRCIACGVRSNLSRQSRIVGGQNALLGAWPWQVSLHVQNIHVCGGSIITPEWIVTAAHCV   298
cat                  LRCIECGVTAKMGRQSRIVGGSAAPGDWPWQASLHVQGVHVCGGSIISPEWIVTAAHCV   298
Ferret               LRCIECGVAGKTMRQSRIVGGSSASPGDWPWQVSLHVQGTHVCGGSIITPEWIVTAAHCV   298
                     **** ***      ********  .*   * ****.*****. ********:**********
```

| | |
|---|---|
| Golden-hamster | SEQ ID No: 1 |
| mouse | SEQ ID No: 2 |
| human | SEQ ID No: 3 |
| green-monkey | SEQ ID No: 4 |
| crab-eating-macaque | SEQ ID No: 5 |
| Rhesus-macaque | SEQ ID No: 6 |
| cat | SEQ ID No: 7 |
| Ferret | SEQ ID No: 8 |

56

# FIG. 10B

```
Golden-hamster       EEPLNSPRYWTAFAGILSQSLMFYGSRHQVEKVISHPNYNSETKNNDIALMKLQTPLTFN   357
mouse                EEPLSSPRYWTAFAGILRQSLMFYGSRHQVEKVISHPNYDSKTKNNDIALMKLQTPLAFN   356
human                EKPLNNPWHWTAFAGILRQSFMFYGAGYQVEKVISHPNYDSKTKNNDIALMKLQKPLTFN   358
green-monkey         EKPLNSPWQWTAFAGILTQSSMFYVKGHRVEKVISHPNYDSKTKNNDIALMKLHTPLTFN   358
crab-eating-macaque  EKPLNSPWQWTAFVGTLRQSSMFYEKGHRVEKVISHPNYDSKTKNNDIALMKLHTPLTFN   358
Rhesus-macaque       EKPLNSPWQWTAFVGTLRQSSMFYEKGHRVEKVISHPNYDSKTKNNDIALMKLHTPLTFN   358
cat                  EEPLNNPRHWTAFVGILRQSFMFYGHGYRVGKVISHPNYDSKTKNNDIALMKLQTPLTFD   358
Ferret               EEPLNNPRYWTVFAGVLRQSFMFYGHGYRVGKVISHPSYDSKTKNNDIALMKLQTPLTFS   358
                     *:**..*   **.*.* * ** ***   ::* ******.*:*:***********:.**:*.

Golden-hamster       DLVKPVCLPNPGMMLDPAQECWISGWGSTYEKGKTSDMLNAAMVPLIERSKCNNKYIYNN   417
mouse                DLVKPVCLPNPGMMLDLDQECWISGWGATYEKGKTSDVLNAAMVPLIEPSKCNSKYIYNN   416
human                DLVKPVCLPNPGMMLQPEQLCWISGWGATEEKGKTSEVLNAAKVLLIETQRCNSRYVYDN   418
green-monkey         ELVKPVCLPNPGMMLEPEQHCWISGWGATQEKGKTSDMLNAAMVPLIEPRRCNSKRVYDG   418
crab-eating-macaque  EVVKPVCLPNPGMMLEPEQHCWISGWGATQEKGKTSDVLNAAMVPLIEPRRCNNKYIYDG   418
Rhesus-macaque       EVVKPVCLPNPGMMLEPEQHCWISGWGATQEKGKTSDVLNAAMVPLIEPRRCNNKYVYDG   418
cat                  DKVKPVCLPNPGLMLEPEQPCWISGWGATHEKGKTSDELNAVMVPLIEPWRCNSKYVYNN   418
Ferret               DKVKPVCLPNPGMMLEPNQSCWISGWGATHEKGKTSDELNAVMVPLIEPWRCNSKYVYNS   418
                     : **********:**:  * *******:* ******: ***. * ***  :**.: :*:.

Golden-hamster       LITPAMVCAGFLQGTVDSCQGDSGGPLVTLKNDIWWLIGDTSWGSGCAKALRPGVYGNVT   477
mouse                LITPAMICAGFLQGSVDSCQGDSGGPLVTLKNGIWWLIGDTSWGSGCAKAYRPGVYGNVM   476
human                LITPAMICAGFLQGNVDSCQGDSGGPLVTSKNNIWWLIGDTSWGSGCAKAYRPGVYGNVM   478
green-monkey         LVTPAMICAGFLQGTVDSCQGDSGGPLVTLKNDVWWLIGDTSWGSGCAQANRPGVYGNVT   478
crab-eating-macaque  LITPAMICAGFLRGTVDSCQGDSGGPLVTLKNDVWWLIGDTSWGSGCAQANRPGVYGNVT   478
Rhesus-macaque       LITPAMICAGFLQGTVDSCQGDSGGPLVTLKNDVWWLIGDTSWGSGCAQANRPGVYGNVT   478
cat                  LVTPAMICAGYLQGTIDSCQGDSGGPLVTMKSHIWWLIGDTSWGSGCAKANRPGVYGNVT   478
Ferret               LVTPAMICAGYLRGGTDSCQGDSGGPLVTLKSRIWWLIGDTSWGSGCAKANRPGVYGNVT   478
                     *:****:***:*:*  ************ *. ;***************:* *******

Golden-hamster       VFTDWIYQQMMANS   491
mouse                VFTDWIYQQMRANS   490
human                VFTDWIYRQMRADG   492
green-monkey         VFTDWIYRQMRADD   492
crab-eating-macaque  VFTDWIYRQMRADD   492
Rhesus-macaque       VFTDWIYRQMRADD   492
cat                  VFTDWIYRQMRANG   492
Ferret               VFTDWIYRQMRANS   492
                     *******:** *:.
```

# FIG. 10C

# FIG. 10D

# FIG. 11A

# FIG. 11B

# FIG. 12

# FIG. 13A

# FIG. 13B

MM57

MM57

752_1

1864_10

752_1

1864_10

# FIG. 13C

2020_12

□WT ▨VOC202012 ▦501Y.V2

2228_15

□WT ▨VOC202012 ▦501Y.V2

2020_12

□WT ▦delta ▨kappa

2228_15

□WT ▦delta ▨kappa

VSVG pseudovirus

□752_1 ▨1864_10 ▦2020_12 ▧2228_15

# FIG. 13D

# FIG. 14

Cell-fusion inhibitory activity (%)

Log concentration of Ab (μg/mL)

# FIG. 15

**Daudi human TMPRSS2 x KHYG-1 mouse FcγR3**

Legend:
- Rituximab
- mIgG
- 1864-10
- 1864-10 ADE⁻
- 2020-12
- 2020-12 ADE⁻

**Daudi mouse TMPRSS2 x KHYG-1 mouse FcγR3**

Legend:
- Rituximab
- mIgG
- 1864-10
- 1864-10 ADE⁻
- 2020-12
- 2020-12 ADE⁻

# FIG. 16

Target cells: Daudi **human TMPRSS2**

Target cells: Daudi **Cynomolgus Monkey TMPRSS2**

# FIG. 17

# FIG. 18A

Cynomolgus monkey in vivo experiment 2

# FIG. 18B

Body temperature change at night

Body weight change

# FIG. 19A

Amount of viruses in airway and bronchus swabs

## (titration)

Day after virus inoculation

# FIG. 19B

## Amount of viruses in bronchus swab
## (RT-PCR)

# FIG. 20

Histopathologic Score

Viral antigen Score

# FIG. 21

# FIG. 22

## INTERNATIONAL SEARCH REPORT

International application No.

**PCT/JP2023/014855**

### A. CLASSIFICATION OF SUBJECT MATTER

***C07K 16/40***(2006.01)i; ***A61K 39/395***(2006.01)i; ***A61P 31/14***(2006.01)i; ***C12N 15/13***(2006.01)i; ***C12N 15/57***(2006.01)i; ***C12P 21/08***(2006.01)i

FI: C07K16/40 ZNA; A61K39/395 D; A61K39/395 P; A61P31/14; C12N15/13; C12N15/57; C12P21/08

According to International Patent Classification (IPC) or to both national classification and IPC

### B. FIELDS SEARCHED

Minimum documentation searched (classification system followed by classification symbols)

C07K16/40; A61K39/395; A61P31/14; C12N15/13; C12N15/57; C12P21/08

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN); UniProt/GeneSeq

### C. DOCUMENTS CONSIDERED TO BE RELEVANT

| Category* | Citation of document, with indication, where appropriate, of the relevant passages | Relevant to claim No. |
|---|---|---|
| X | WO 2021/163076 A1 (REGENERON PHARMACEUTICALS, INC.) 19 August 2021 (2021-08-19) claims, examples | 1-4, 6, 7, 9-12 |
| Y | claims, examples | 1-12 |
| Y | WO 2021/211406 A1 (MADDON ADVISORS LLC) 21 October 2021 (2021-10-21) claims, pages 19, 20 | 1-12 |
| P, X | HARADA, M. et al. Establishment of anti-TMPRSS2 mAbs that potently inhibit infection of any variants of SARS-CoV-2. 日本免疫学会総会・学術集会記録, 07 December 2022, vol. 51, WS28-09-P, non-official translation (Records of Annual Meeting of the Japanese Society for Immunology.) entire text | 1-12 |
| A | WO 2019/147831 A1 (REGENERON PHARMACEUTICALS, INC.) 01 August 2019 (2019-08-01) claims, examples | 1-12 |

☑ Further documents are listed in the continuation of Box C.      ☑ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **19 June 2023** | **27 June 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**

| International application No. |
| --- |
| **PCT/JP2023/014855** |

**C. DOCUMENTS CONSIDERED TO BE RELEVANT**

| Category* | Citation of document, with indication, where appropriate, of the relevant passages | Relevant to claim No. |
| --- | --- | --- |
| A | WO 2021/211416 A1 (MADDON ADVISORS LLC) 21 October 2021 (2021-10-21)<br>    examples | 1-12 |
| A | TOMITA, Y. et al. The Physiological TMPRSS2 Inhibitor HAI-2 Alleviates SARS-CoV-2 Infection. J. Virol. 2021, vol. 95, issue 12, e00434-21<br>    fig. 1 | 1-12 |
| T | CN 116003611 A (CENTRAL SOUTH UNIV. XIANGYA HOSPITAL) 25 April 2023 (2023-04-25)<br>    examples 3-6 | 1-12 |

Form PCT/ISA/210 (second sheet) (January 2015)

**EP 4 509 529 A1**

**INTERNATIONAL SEARCH REPORT**

| International application No. |
|---|
| **PCT/JP2023/014855** |

**Box No. I        Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

    a.  ☑ forming part of the international application as filed:

        ☑ in the form of an Annex C/ST.25 text file.

        ☐ on paper or in the form of an image file.

    b.  ☐ furnished together with the international application under PCT Rule 13*ter*.1(a) for the purposes of international search only in the form of an Annex C/ST.25 text file.

    c.  ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2.  ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

    "The form of Annex C/ST.25 text file" above shall read as "the form of ST.26.".

Form PCT/ISA/210 (continuation of first sheet) (January 2015)

**INTERNATIONAL SEARCH REPORT**
Information on patent family members

| International application No. |
| --- |
| **PCT/JP2023/014855** |

| Patent document cited in search report | | | Publication date (day/month/year) | Patent family member(s) | | | Publication date (day/month/year) |
| --- | --- | --- | --- | --- | --- | --- | --- |
| WO | 2021/163076 | A1 | 19 August 2021 | US | 2021/0246226 | A1 | |
| | | | | claims, examples | | | |
| | | | | EP | 4103619 | A1 | |
| | | | | claims, examples | | | |
| | | | | JP | 2023-513124 | A | |
| | | | | claims, examples | | | |
| | | | | KR | 10-2022-0140503 | A | |
| | | | | claims, examples | | | |
| | | | | CN | 115427457 | A | |
| | | | | claims, examples | | | |
| WO | 2021/211406 | A1 | 21 October 2021 | US | 2022/0056153 | A1 | |
| | | | | claims | | | |
| | | | | US | 2022/0056154 | A1 | |
| | | | | claims | | | |
| | | | | US | 2022/0098283 | A1 | |
| | | | | claims | | | |
| | | | | WO | 2021/211414 | A1 | |
| | | | | claims | | | |
| | | | | WO | 2021/211416 | A1 | |
| | | | | claims | | | |
| | | | | WO | 2021/211402 | A2 | |
| | | | | claims | | | |
| | | | | EP | 4136254 | A2 | |
| | | | | claims | | | |
| | | | | CN | 115398006 | A | |
| | | | | claims | | | |
| | | | | KR | 10-2023-0002612 | A | |
| | | | | claims | | | |
| WO | 2019/147831 | A1 | 01 August 2019 | JP | 2021-511058 | A | |
| | | | | claims, examples | | | |
| | | | | US | 2019/0300625 | A1 | |
| | | | | claims, examples | | | |
| | | | | EP | 3638698 | A1 | |
| | | | | claims, examples | | | |
| | | | | CN | 111936517 | A | |
| | | | | claims, examples | | | |
| WO | 2021/211416 | A1 | 21 October 2021 | US | 2022/0098283 | A1 | |
| | | | | examples | | | |
| | | | | US | 2022/0056153 | A1 | |
| | | | | examples | | | |
| | | | | US | 2022/0056154 | A1 | |
| | | | | examples | | | |
| | | | | WO | 2021/211406 | A1 | |
| | | | | examples | | | |
| | | | | WO | 2021/211414 | A1 | |
| | | | | examples | | | |
| | | | | WO | 2021/211402 | A2 | |
| | | | | examples | | | |
| | | | | EP | 4136254 | A2 | |
| | | | | examples | | | |
| | | | | CN | 115398006 | A | |
| | | | | examples | | | |
| | | | | KR | 10-2023-0002612 | A | |
| | | | | examples | | | |
| CN | 116003611 | A | 25 April 2023 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007024708 A
- WO 2009127060 A
- WO 2019147831 A

- WO 2021163076 A
- WO 2021211406 A
- WO 2021211416 A

**Non-patent literature cited in the description**

- **ANDERSEN et al.** *Nature Medicine*, 2020, vol. 26, 450-452
- **KIM TS. et al.** *Mol. Cell Bio.*, 2006, vol. 26, 965-975
- **SAKAI K. et al.** *J. Viol.*, 2014, vol. 88, 5608-5616

- **SILVA et al.** *J Biol Chem*, 2015, vol. 290, 5462-5469
- **TOMITA et al.** *Journal of Virology*, 2021, vol. 95 (12)
- **YAMAMOTO et al.** *Viruses*, 2020